(19) Europäisches Patentamt
European Patent Office
Office européen des brevets

(11) Numéro de publication: **0 412 899 B1**

# (12) FASCICULE DE BREVET EUROPEEN

(45) Date de publication de fascicule du brevet: **19.04.95**

(51) Int. Cl.[6]: **C07D 498/04**, C07D 213/74, A61K 31/435, //(C07D498/04, 263:00,221:00)

(21) Numéro de dépôt: **90402255.5**

(22) Date de dépôt: **07.08.90**

(54) **Dérivés d'oxazolo pyridines, leur procédé de préparation et les compositions pharmaceutiques qui les contiennent.**

(30) Priorité: **07.08.89 FR 8910597**

(43) Date de publication de la demande:
**13.02.91 Bulletin 91/07**

(45) Mention de la délivrance du brevet:
**19.04.95 Bulletin 95/16**

(84) Etats contractants désignés:
**AT BE CH DE DK ES FR GB GR IT LI LU NL SE**

(56) Documents cités:

**EUROPEAN JOURNAL OF MEDICINAL CHEMISTRY, vol. 17, no. 1, 1982, pages 85-88,Chateway-Malabry, FR; M. DEBAERT et al.: "Pharmacomodulation du modèle benzoxazolinone par la structure aryl-pipérazinique"**

(73) Titulaire: **SCIENCE ET ORGANISATION**
**31 rue du Pont**
**F-92200 Neuilly sur Seine (FR)**

(72) Inventeur: **Guillaumet, Gérald**
**2 rue Auguste Renoir**
**F-45100 Orléans (FR)**
Inventeur: **Flouzat, Christine**
**14 rue des Tanneurs**
**F-45000 Orléans (FR)**
Inventeur: **Bonnet, Jacqueline**
**19 rue Charcot**
**F-75013 Paris (FR)**

**EP 0 412 899 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux dérivés des oxazolo - [4,5b] pyridines, leur procédé de préparation et les compositions pharmaceutiques qui les contiennent.

On connaît déjà les propriétés à la fois analgésiques et anti-inflammatoires des phényl - 2 oxazolo - [5,4] ou [4,5] pyridines (brevets US 4 038 396, Fr 2 328 471, Fr 2 319 354, GB 1 421 619).

Toutefois, ces produits possèdent un profil essentiellement anti-inflammatoire comme le confirment les indications thérapeutiques mentionnées dans les brevets cités ci-dessus ou bien présentent l'inconvénient de ne pas dissocier les deux types d'activités : analgésique d'une part, anti-pyrétique, anti-inflammatoire d'autre part. Certaines (aryl-4 piperazinyl-1) alkyl-3 benzoxazolinones ont déjà été décrites dans EUR J.MED. CHEM. 1982, 17, pp.85-88 comme ayant des activités analgésiques.

La demanderesse a maintenant découvert de nouveaux composés, dont le niveau d'activité analgésique est au moins comparable voire supérieur à celui des phényl - 2 [3H] oxazolo [4,5b] pyridines déjà connues, mais possédant la caractéristique particulièrement avantageuse d'être totalement dépourvus d'activité anti-inflammatoire : les composés de la présente invention sont en effet doués d'une activité analgésique pure de haut niveau. Or, la plupart des substances analgésiques non morphiniques connues à ce jour possèdent également une activité anti-inflammatoire (exemples : salicylés, pyrazolés...) et ils interviennent par conséquent sur les processus intervenant dans l'inflammation. Ceux-ci impliquent de très nombreux médiateurs chimiques (prostaglandines, thomboxane A2...) ; il s'ensuit donc de multiples effets secondaires dont les plus connus sont : attaque de la muqueuse gastrique avec possibilité d'ulcères, inhibition de l'agrégation plaquettaire avec troubles de la coagulation. Outre les dérangements qu'ils occasionnent, ces effets parallèles interdisent l'usage de ces produits chez de nombreux sujets qui y sont particulièrement sensibles. En étant dépourvus de toute activité anti-inflammatoire, les composés de la présente invention n'interviennent donc pas sur les médiateurs de l'inflammation et sont donc dépourvus des effets secondaires mentionnés précédemment. Cette caractéristique, jointe à leur absence totale de toxicité et à leur haut niveau d'activité rend les composés de la présente invention utilisables en tant qu'analgésique d'une façon beaucoup plus sûre et sans les restrictions d'usage habituellement connues pour la grande majorité de ces produits.

Plus spécifiquement l'invention concerne des dérivés de formule générale (I) :

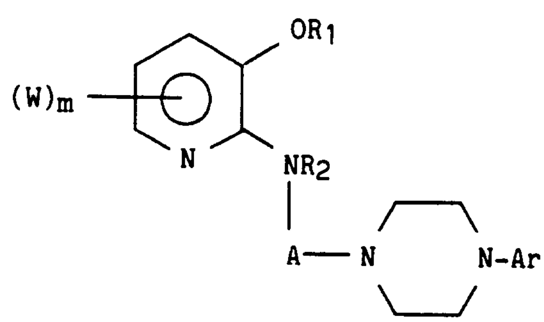

(I)

dans laquelle :

- $R_1$ et $R_2$ représentent chacun un atome d'hydrogène ou forment avec l'oxygène et l'azote qui les portent un chaînon-O-CO-N,
- W représente un atome d'halogène ou un groupement alkyle ou alkoxy inférieur éventuellement substitué par un ou plusieurs atomes d'halogène tel que trifluorométhyle, m étant compris entre 0 et 3,
- A représente un goupement alcoyle inférieur linéaire ou ramifié,
- Ar représente un groupement aryle ou hétéroaryle éventuellement substitué par un ou plusieurs atomes d'halogène ou par un ou plusieurs groupements alkyle inférieur, hydroxy, hydroxysulfonyloxy ou alkoxy inférieur, ou aryloxy éventuellement substitué par un ou plusieurs atomes d'halogène tel que le groupement trifluorométhyle,

étant entendu que par radical alkyle inférieur ou alkyloxy inférieur, on entend un groupement alkyle linéaire ou ramifié comprenant de 1 à 6 atomes de carbone et que par groupements aryle ou hétéroaryle, on entend des groupements mono ou bicyclique insaturés comprenant de 5 à 12 atomes intégrant ou non dans leur squelette carboné un, deux ou trois hétéroatomes choisis parmi azote, oxygène ou soufre,

leurs isomères,
ainsi que leurs sels d'addition à un acide pharmaceutiquement acceptable et lorsque R1 et R2 représentent chacun un atome d'hydrogène, leurs sels d'addition à une base pharmaceutiquement acceptable.

Parmi les acides que l'on peut ajouter aux composés de formule (I) pour former un sel d'addition, on peut citer à titre d'exemple les acides chlorhydrique, sulfurique, phosphorique, tartrique, malique, maléique, fumarique, oxalique, méthane sulfonique, éthane sulfonique, camphorique, citrique...

Parmi les bases que l'on peut ajouter aux composés de formule (I) pour lesquels $R_1$ et $R_2$ représentent chacun un atome d'hydrogène, on peut citer à titre d'exemple les hydroxydes et carbonates de sodium, potassium, calcium.

Parmi les dérivés de l'invention, on préfère actuellement ceux pour lesquels :

- Ar représente un groupement phényle éventuellement substitué par un atome d'halogène ou un groupement alkyle inférieur, hydroxy, hydroxysulfonyloxy ou alkoxy inférieur éventuellement substitué par un ou plusieurs atomes d'halogène tel que trifluorométhyle.

L'invention s'étend également au procédé d'obtention de composés de formule (I), caractérisé en ce que l'on fait réagir un dérivé de formule (II) :

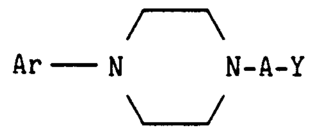

(II)

dans laquelle A et Ar ont la même signification que dans la formule (I), et Y représente un atome d'halogène,
que l'on soumet à réaction après dissolution dans un solvant organique avec un dérivé de formule (III) :

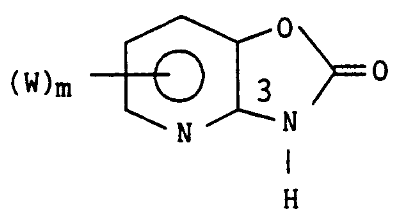

(III)

dans laquelle W et m ont la même signification que dans la formule (I),
ou de son dérivé d'addition en 3 à un métal alcalin, obtenu préférentiellement par dissolution du dérivé de formule (III) dans une solution alcoolique d'un éthylate de métal alcalin,
pour conduire après chauffage, préférentiellement à reflux du milieu réactionnel, refroidissement, filtration, évaporation du milieu réactionnel, reprise par l'eau, extraction par un solvant organique choisi préférentiellement parmi chloroforme, chlorure de méthylène ou éther éthylique et purification par chromatographie sur colonne de silice,
à un dérivé de formule (I/A) cas particulier des dérivés de formule (I) :

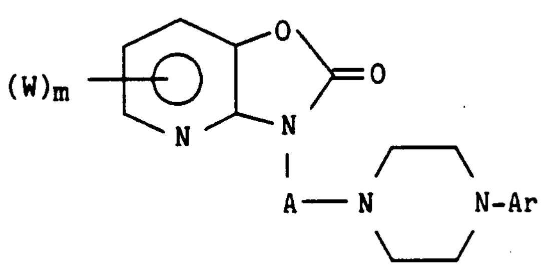

(I/A)

dans lesquels W, m et A ont la même signification que précédemment et $R_1$ et $R_2$ forment avec l'azote et l'oxygène qui les portent un chaînon -O-CO-N que l'on peut, si nécessaire, séparer en ses isomères et, si on le désire, salifier par un acide pharmaceutiquement acceptable,
dérivé de formule (I/A) que l'on peut traiter, si on le désire, par un agent alcalin en solution aqueuse, à une

température comprise entre la température ambiante et la température d'ébullition du milieu réactionnel pour conduire à un dérivé de formule (I/B) :

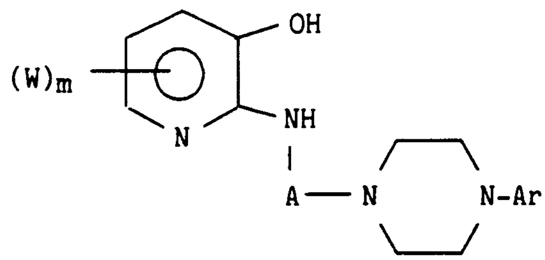

(I/B)

cas particulier des dérivés de formule (I) pour lesquels $R_1$ et $R_2$ représentent chacun un atome d'hydrogène,
que l'on purifie si nécessaire par une technique choisie parmi cristallisation ou chromatographie et que l'on salifie, si on le désire, par un acide ou une base pharmaceutiquement acceptable.

Les composés de formule (I/A) peuvent également être obtenus en faisant réagir un dérivé de formule (III) :

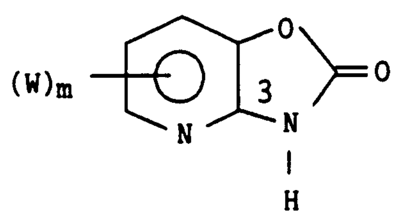

(III)

dans laquelle W et m ont la même signification que précédemment,
avec un hydroxyde de métal alcalin en milieu aqueux ou un alcoolate de métal alcalin en milieu organique, pour conduire à un dérivé de formule (IV) :

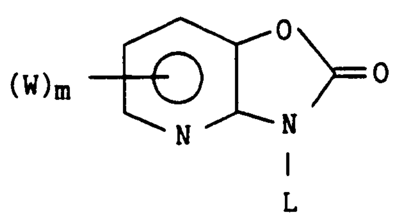

(IV)

dans laquelle W et m ont la même signification que précédemment, et L représente un métal alcalin,
que l'on condense avec un dérivé de formule (V) :

**X-A-X'     (V)**

dans laquelle A a la même signification que précédemment, X et X' identiques ou différents représentent un atome d'halogène,
préférentiellement sous atmosphère inerte, en milieu organique, préférentiellement à température du reflux du solvant choisi pour conduire après éventuelle extraction et purification par chromatographie à un dérivé de formule (VI) :

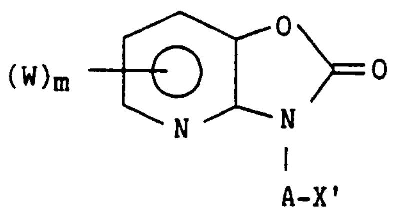

(VI)

dans laquelle W, X', m et A ont la même signification que précédemment,
que l'on condense, préférentiellement sous atmosphère inerte avec un dérivé de formule (VII), préférentiellement en excès :

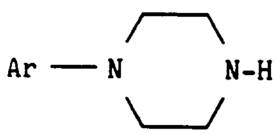

(VII)

dans laquelle Ar a la même définition que précédemment,
en milieu organique en présence d'un excès d'une amine tertiaire et à température du reflux du solvant choisi pour conduire après refroidissement, extraction et éventuelle purification par cristallisation à un dérivé de formule (I) pour lequel A représente un chaînon alkyle inférieur,
que l'on peut, si nécessaire, séparer en ses isomères et si on le désire, salifier par un acide pharmaceutiquement acceptable.

Ce dernier procédé pourra avantageusement utiliser, lorsque A est un chaînon alkyle linéaire, un dérivé de formule (VIII) :

**X-A-X (VIII)**

cas particulier des dérivés de formule (V) pour lequel X et X' sont identiques

Un cas particulier des dérivés de la présente invention est constitué par les dérivés de formule (I/A) pour lesquels A représente un groupement $CH_2$.

Les dérivés pour lesquels A représente un groupement $CH_2$ et $R_1$ et $R_2$ forment avec l'azote et l'oxygène qui les portent un chaînon O-CO-N seront avantageusement obtenus en une seule étape par dissolution, en milieu d'alcool aliphatique inférieur, d'un dérivé de formule (III) tel que ci dessus défini, d'une aryl pipérazine de formule (VII) telle que ci dessus définie en léger excès, et d'un excès de formol, chauffage de la solution ainsi obtenue à une température comprise entre la température ambiante et la température d'ébullition de la solution,
pour conduire, après éventuel refroidissement, repos d'une à deux heures et filtration, et éventuelle chromatographie sur colonne de silice, à un dérivé de formule (I/A1) :

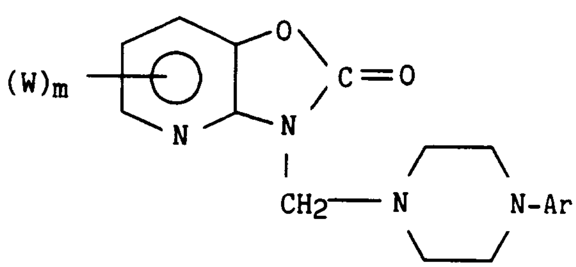

(I/A1)

cas particulier des dérivés de formule (I/A) pour lesquels A représente un chaînon $CH_2$,
dans laquelle W, m et Ar ont la même signification que précédemment,
que l'on peut salifier, si on le désire par un acide pharmaceutiquement acceptable.

Les composés de formule (I) possèdent des propriétés pharmacologiques intéressantes.

En particulier, ces dérivés ont révélé une activité analgésique intéressante.

L'étude pharmacologique des dérivés de l'invention a montré qu'ils étaient peu toxiques, doués d'une activité analgésique pure de haut niveau, dépourvue de composante anti-inflammatoire et donc dépourvue d'inconvénients inhérents à la plupart des composés présentant cette activité (action ulcérigène sur les muqueuses, perturbation de la coagulation...). Ce spectre d'activité rend donc les composés de la présente invention particulièrement intéressants dans un certain nombre d'indications telles que algies rhumatismales, telles que névralgies artérites, lombo-sciatique, algies traumatiques telles que entorses, fractures, luxations ,douleurs post-traumatiques, douleurs post-opératoires, douleurs dentaires, douleurs neurologiques telles que névralgies faciales, douleurs viscérales telles que coliques néphrétiques, dysménorrhées, chirurgie proctologique, pancréatite, algies diverses, céphalées, migraine, douleurs des cancéreux...

La présente invention a également pour objet les compositions pharmaceutiques contenant les produits de formule (I) ou un de leurs sels d'addition à un acide pharmaceutiquement acceptable, seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, rectale, perlinguale, oculaire ou respiratoire, et notamment les préparations injectables, les aérosols, les gouttes oculaires ou nasales, les comprimés simples ou dragéifiés, les comprimés sublinguaux, les sachets, les paquets, les gélules, les glossettes, les tablettes, les suppositoires, les crèmes, pommades, gels dermiques, etc...

La posologie utile varie selon l'âge et le poids du patient, la voie d'administration, la nature de l'indication thérapeutique et des traitements éventuellement associés et s'échelonne entre 1 milligramme et 1 gramme par 24 heures.

Les exemples suivants illustrent l'invention et ne la limitent en aucune façon.

Les spectres de résonance magnétique nucléaire du $^{1}H$ ont été enregistrés en utilisant le TMS comme référence interne. Les spectres infrarouges ont été enregistrés à partir d'une pastille de KBr renfermant 1% environ du produit à analyser.

Les produits obtenus selon les protocoles opératoires décrits dans la rubrique "Préparations" ne font pas partie de l'invention, ils constituent néanmoins des intermédiaires de synthèse utiles pour la préparation des composés de l'invention.

## PREPARATIONS :

### PREPARATION 1 : ARYL - 4 (CHLORO - 2 ETHYL) - 1 PIPERAZINES

Dissoudre 0,04 mmole d'aryl pipérazine dans 40 ml de diméthylformamide. Ajouter sous argon 6,63 g (0,048 mole) de carbonate de potassium sec puis 6,88 g (0,048 mmol) de bromo -1 chloro - 2 éthane. Agiter sous argon à température ambiante pendant 22 heures. Filtrer pour éliminer l'insoluble minéral. Acidifier le filtrat par de l'éthanol saturé en acide chlorhydrique sec jusqu'à l'obtention d'un PH voisin de 1. Ajouter 400 ml d'éther éthylique anhydre. Il apparaît un précipité de chlorhydrate de (chloro - 2 éthyl) - 1 aryl - 4 pipérazine. Evaporer et placer le précipité obtenu dans une solution de $Na_2CO_3$ à 10 %. Extraire au dichlorométhane. Sécher la phase organique sur sulfate de magnésium. Filtrer et évaporer à sec la phase organique au bain-marie sous vide. Le produit ainsi obtenu est directement utilisable dans les réactions ultérieures.

### PREPARATION 2 : ARYL - 4 (CHLORO - 3 PROPYL) - 1 PIPERAZINES

En remplaçant dans la préparation 1, le bromo - 1 chloro - 2 éthane par le bromo - 1 chloro - 3 propane, on obtient les produits attendus.

### PREPARATION 3 : ARYL - 4 (METHYL - 1 CHLORO - 1 ETHYL) - 1 PIPERAZINES

En remplaçant dans la préparation 1 le bromo - 1 chloro - 2 éthane par le bromo - 1 chloro - 2 propane, on obtient les produits attendus.

**PREPARATION 4 : [3H] OXAZOLO [4,5b] PYRIDINONE - 2 ***

Dans un tricol, verser 5,5 g (0,05 M) d'amino - 2 hydroxy - 3 pyridine et mettre le système sous argon. Additionner 100 ml de Tétrahydrofuranne anhydre (THF). Verser ensuite 12,15 g (0,075 M) de 1,1 - carbonyldiimidazole. Chauffer à reflux pendant 5 heures (sous argon). Evaporer ensuite le THF. Reprendre le résidu par du dichlorométhane. Effectuer des lavages de la phases organique avec une solution de NaOH (5 %) (6 x 150 ml), le produit cyclisé passe dans la phase aqueuse et précipite à pH voisin de 5 (par addition d'une solution 2N d'acide chlorhydrique). Filtrer et conserver au dessicateur.

Rendement : 77 %

Point de fusion : 212 - 214 °C.

**PREPARATION 5 : METHYL - 5 [3H] OXAZOLO - [4,5b] PYRIDINONE - 2**

**STADE A : NITRO - 2 HYDROXY - 3 METHYL - 6 PYRIDINE**

Ajouter 5,45 g (50 mM) d'hydroxy - 5 méthyl - 2 pyridine dans 20 ml d'acide sulfurique concentré, en refroidissant dans un bain de glace. Maintenir la température à +6°C et ajouter 2,35 ml d'acide nitrique fumant, sous agitation. Laisser une nuit à température ambiante. Ajouter 100 g de glace sous agitation. Filtrer et rincer à l'eau, sécher.

**STADE B : AMINO - 2 HYDROXY - 3 METHYL - 6 PYRIDINE**

Placer sous pression d'hydrogène 3,5 g de nitro - 2 hydroxy - 3 méthyl - 6 pyridine dans 50 ml de méthanol en présence d'un gramme de charbon palladié. Agiter, filtrer. Evaporer le méthanol.

**STADE C : METHYL - 5 [3H] OXAZOLO - [4,5b] PYRIDINONE - 2**

Dans un ballon tricol verser 1,24 g (10 mM) d'amino - 2 hydroxy - 3 méthyl - 6 pyridine. Placer sous argon. Additionner 20 ml de tétrahydrofuranne anhydre puis 2,43 g (15 mM) de 1,1 - carbonyl diimidazole. Chauffer 6 heures à reflux. Evaporer le milieu réactionnel. Laver les cristaux obtenus à l'eau, filtrer et redissoudre dans du méthanol chaud. Filtrer et réévaporer.

Rendement : 75 %

Point de fusion : 243 °C

Caractéristiques spectrales :

RMN $^1H$ Solvant $CDCl_3$ : δ ppm

δ : 12,3 1H, massif, NH

δ : 7,5 1H ; doublet ; $H_7$ ; J = 8 Hz

δ : 6,9 1H ; doublet ; $H_6$ ; J = 8 Hz

δ : 2,4 3H ; singulet ; $CH_3$

Infrarouge : 1750 $cm^{-1}$, ν(C=O)

1610 $cm^{-1}$, ν (C=C)

**PREPARATION 6 : (BROMO - 2 ETHYL) - 3 [3H] OXAZOLO - [4,5b] PYRIDINONE - 2**

**STADE A : DERIVE SODE DE L'OXAZOLO [4,5b] PYRIDINONE - 2**

Dissoudre 6 g (44,11 mM) de [3H] d'oxazolo [4,5b] pyridinone - 2 dans une quantité suffisante de tétrahydrofuranne puis additionner cette solution à une solution éthanolique d'éthylate de sodium obtenue à partir d'un gramme (44,11 mM) de sodium dans environ 150 ml d'éthanol. Evaporer sous vide et reprendre le résidu par une quantité suffisante de diméthylformamide pour le dissoudre.

**STADE B : (BROMO - 2 ETHYL - 3 [3H] OXAZOLO [4,5b] PYRIDINONE - 2**

Dans un ballon sous argon, surmonté d'un réfrigérant, mettre 7,6 ml (88,22 mM) de dibromo - 1,2 éthane en solution dans environ 50 ml de diméthylformamide puis additionner lentement, sous agitation, la solution obtenue à l'étape précédente . Porter à 100 °C pendant 2 heures.

* Voir nomenclature en annexe

Après refroidissement, évaporer sous vide le diméthylformamide puis reprendre le résidu par de l'eau et extraire au chlorure de méthylène. Après séchage sur $MgSO_4$, évaporer le chlorure de méthylène et purifier le résidu sur colonne de silice flash (230-240 Mesh) dans le chlorure de méthylène. On obtient après évaporation 5,2 g d'une poudre blanche.

Rendement : 50 %

Point de fusion : 84 °C

Caractérisitiques spectrales :

$^1H$ RMN - $CDCl_3$ - $\delta$ : ppm

$\delta$ : 3,78 2H ; triplet; $CH_2$ - $\underline{CH_2}$ Br ; J = 6,3 Hz

$\delta$ : 4,36 2H ; triplet; $\underline{CH_2}$ - $CH_2$ Br ; J = 6,3 Hz

$\delta$ : 7,10 1H ; doublet dédoublé; $H_6$ ; $JH_6H_7$ = 8,2 Hz et $JH_6H_7$ = 5,6Hz

$\delta$ : 7,43 1H ; doublet dédoublé; $H_7$ ; $JH_7H_6$ = 8.2 Hz et $JH_7H_5$ = 0,5Hz

$\delta$ : 8,13 1H ; doublet dédoublé; $H_5$ ; $JH_5H_6$ = 5,6 Hz et $JH_5H_7$ = 0,5Hz

Infrarouge : 1760 $cm^{-1}$ : $\nu$CO

En procédant de la même façon, mais en utilisant le dichloro - 1,2 éthane, on obtient la (chloro - 2 éthyl) - 3 [3H] oxazolo [4,5b] pyridinone - 2.

En procédant de la même façon, mais en remplaçant le dibromo - 1,2 éthane par des dérivés de formule générale :

**X - $(CH_2)_{n+1}$ - X**

on obtient plus généralement des (halogéno alkyl) - 3 [3H] oxazolo [4,5b] pyridinones - 2.

**PREPARATION 7 : BROMO-6[3H] OXAZOLO [4,5b] PYRIDINONE - 2**

Dissoudre 0,01 mole d'oxazolo [4,5b] pyridinone - 2 dans 100 ml de diméthylformamide. Ajouter par l'intermédiaire d'une ampoule à brome 0,011 mole de brome. Maintenir l'agitation 1 heure 30 à température ambiante, ajouter un mélange glace-eau. Filtrer. Laver à l'eau. Sécher.

Rendement : 90%

Point de fusion : 234 °C

**EXEMPLE 1 : (PHENYL - 4 PIPERAZINYL - 1) METHYL - 3 [3H] OXAZOLO [4,5b] PYRIDINONE - 2**

Dissoudre 4,1 g (0,03 mole) de [3H] oxazolo [4,5b] pyridinone - 2 dans 100 ml d'alcool à 95 °C.

Ajouter 5,35 g (0,033 mole) de phényl - 1 pipérazine puis 3 ml d'une solution aqueuse de formol à 30 %. Agiter au bain-marie, à une température voisine de 50 °C, pendant 1H30, en maintenant l'agitation. Laisser reposer 1 heure à température ambiante.

Essorer les cristaux et filtrer sur une colonne de silice (60 Ä ; 60 - 220 microns) en éluant par du dichlorométhane.

Rendement : 81 %

Point de fusion : 151 - 152 °C

Les caractéristiques physico-chimiques de ce produit sont visualisées dans le tableau 1.

**EXEMPLE 2 : [(TRIFLUOROMETHYL - 3 PHENYL) - 4 PIPERAZINYL - 1] METHYL - 3 [3H] OXAZOLO [4,5b] PYRIDINONE - 2**

En remplaçant dans l'exemple 1 la phényl - 1 pipérazine par la (trifluorométhyl - 3 phényl) - 1 pipérazine, on obtient le produit attendu.

Rendement : 71 %

Point de fusion : 112 - 118 °C

Les caractéristiques physico-chimiques de ce produit sont visualisées dans le tableau 1.

**EXEMPLES 3 A 8 : [(ARYL - 4 PIPERAZINYL - 1) METHYL] - 3 [3H] OXAZOLO [4,5b] PYRIDINONES - 2**

En procédant comme dans l'exemple 1, mais en remplaçant la phényl - 1 pipérazine par :

**EXEMPLE 3 :**

- la (chloro - 2 phényl) - 1 pipérazine, on obtient la :
  **[(CHLORO - 2 PHENYL) - 4 PIPERAZINYL - 1] METHYL] - 3 [3H] OXAZOLO [4,5b] PYRIDINONE - 2**

**EXEMPLE 4 :**

- la (fluoro - 4 phényl) - 1 pipérazine, on obtient la :
  **[(FLUORO - 4 PHENYL) - 4 PIPERAZINYL - 1 METHYL] - 3 [3H] OXAZOLO [4,5b] PYRIDINONE - 2**

**EXEMPLE 5 :**

- la (méthoxy - 2 phényl) - 1 pipérazine, on obtient la :
  **[(METHOXY - 2 PHENYL) - 4 PIPERAZINYL - 1 METHYL] - 3 [3H] OXAZOLO [4,5b] PYRIDINONE - 2**

**EXEMPLE 6 :**

- la (méthyl - 2 phényl) - 1 pipérazine, on obtient la :
  **[(METHYL - 2 PHENYL) - 4 PIPERAZINYL - 1 METHYL] - 3 [3H] OXAZOLO [4,5b] PYRIDINONE - 2**

**EXEMPLE 7 :**

- la (trifluorométhyl - 3 chloro - 4 phényl) - 1 pipérazine, on obtient la :
  **[(TRIFLUOROMETHYL - 3 CHLORO - 4 PHENYL) - 4 PIPERAZINYL - 1 METHYL] - 3 [3H] OXAZOLO [4,5b] PYRIDINONE - 2**

**EXEMPLE 8 :**

- la (pyrimidinyl - 2) - 1 pipérazine, on obtient la :
  **[(PYRIMIDINYL 2) - 4 PIPERAZINYL - 1 METHYL] - 3 [3H] OXAZOLO [4,5b] PYRIDINONE - 2**

**EXEMPLE 9 :**

- la (naphtyl - 1) - 1 pipérazine, on obtient la :
  **[(NAPHTYL - 1) - 4 PIPERAZINYL - 1 METHYL] - 3 [3H] OXAZOLO [4,5b] PYRIDINONE - 2**

**EXEMPLE 10 :**

- la (pyridyl - 2) - 1 pipérazine, on obtient la :
  **[(PYRIDYL - 2) - 4 PIPERAZINYL - 1 METHYL] - 3 [3H] OXAZOLO [4,5b] PYRIDINONE - 2**

**EXEMPLE 11 :**

- l'(isoquinolyl - 1) - 1 pipérazine, on obtient la :
  **[(ISOOUINOLYL - 1) - 4 PIPERAZINYL - 1 METHYL] - 3 [3H] OXAZOLO [4,5b] PYRIDINONE - 2**

**EXEMPLE 12 :**

- la (quinolyl - 2) - 1 pipérazine, on obtient la :
  **[(OUINOLYL - 2) - 4 PIPERAZINYL - 1 METHYL] - 3 [3H] OXAZOLO [4,5b] PYRIDINONE - 2**

**EXEMPLE 13 :**

- la (thiazolyl - 2) - 1 pipérazine, on obtient le :
  **[(THIAZOLYL - 2) - 4 PIPERAZINYL - 1 METHYL] - 3 [3H] OXAZOLO [4,5b] PYRIDINONE - 2**

**EXEMPLE 14 : [(PHENYL - 4 PIPERAZINYL - 1) - 2 ETHYL] - 3 [3H] OXAZOLO [4,5b] PYRIDINONE - 2**

**MODE OPERATOIRE 1**

Dissoudre 1,13 g (0,049 mole) de sodium dans 600 ml d'éthanol. Additionner 6,6 g (0,049 mole) de [3H] oxazolo [4,5 - b] pyridinone - 2 à la solution précédemment obtenue. Agiter vigoureusement à température ambiante puis évaporer l'éthanol.

Dissoudre par ailleurs 11,0 g (0,049 mole) de phényl - 4 (chloro - 2 éthyl) - 1 pipérazine obtenue selon le protocole de la préparation 1 dans 50 ml de diméthylformamide et ajouter lentement et sous agitation le dérivé sodé de la [3H] oxazolo [4,5-b] pyridinone - 2 précédemment préparé. Chauffer à reflux pendant 1H30. Après refroidissement, essorer l'insoluble minéral et évaporer le filtrat au bain-marie sous vide.

Reprendre le résidu par l'eau et purifier par extraction au dichlorométhane. Filtrer le résidu obtenu sur colonne de silice en éluant directement au dichlorométhane.

Rendement : 59 %

**MODE OPERATOIRE 2**

Dans un ballon placé sous argon et surmonté d'un réfrigérant, additionner à 2,43 g (0,01 mole) de (bromo - 2 éthyl) - 3 [3H] oxazolo [4,5b] pyridinone - 2 obtenue dans la préparation 6, 1,5 équivalent de phényl - 1 pipérazine puis 1,5 équivalent de diisopropyléthylamine. Porter à 80 °C pendant 12 heures. Après refroidissement, évaporer l'acétonitrile sous vide et reprendre le résidu par l'eau. Vérifier l'alcalinité du milieu et extraire au dichlorométhane. Sécher sur sulfate de magnésium, évaporer et recristalliser.

Rendement : 95 %

Point de fusion : 105 - 110 °C

Les caractéristiques physico-chimiques de ce composé sont visualisées dans le tableau 1.

**EXEMPLE 15 : {[(TRIFLUOROMETHYL - 3 PHENYL) - 4 PIPERAZINYL - 1] - 2 ETHYL} - 3 [3H] OXAZOLO [4,5b] PYRIDINONE - 2**

En procédant comme dans l'exemple 14, mode opératoire 1, mais en remplaçant la phényl - 4 (chloro - 2 éthyl) - 1 pipérazine par la (trifluorométhyl - 3 phényl) - 4 (chloro - 2 éthyl) - 1 pipérazine, on obtient le produit attendu.

Rendement : 40 %

Point de fusion : 92 - 93 °C.

Les caractéristiques physico-chimiques de ce composé figurent dans le tableau 1.

**EXEMPLE 16 : [(PHENYL - 4 PIPERAZINYL - 1) - 3 PROPYL)] - 3 [3H] OXAZOLO [4,5b] PYRIDINONE - 2**

En procédant comme dans l'exemple 14, mode opératoire 1, mais en remplaçant la phényl - 4 (chloro - 2 éthyl) - 1 pipérazine par la phényl - 4 (chloro - 3 propyl) - 1 pipérazine, on obtient le produit attendu.

Rendement : 47 %

Point de fusion : 141 - 142 °C

Les caractéristiques physico-chimiques de ce composé figurent dans le tableau 1.

**EXEMPLE 17 : {[(TRIFLUOROMETHYL - 3 PHENYL) - 4 PIPERAZINYL - 1] - 3 PROPYL} - 3 [3H] OXAZOLO [4,5b] PYRIDINONE - 2**

En procédant comme dans l'exemple 14, mode opératoire 1, mais en remplaçant la phényl - 4 (chloro - 2 éthyl) - 1 pipérazine par la (trifluorométhyl - 3 phényl) - 4 (chloro - 3 propyl) - 1 pipérazine, on obtient le produit attendu.

Rendement : 63 %

Point de fusion : 62 - 63 °C - Voir tableau 1

**EXEMPLES 18 A 21 :**

En remplaçant dans l'exemple 14, mode opératoire 1, la phényl - 4 (chloro - 2 éthyl) - 1 pipérazine par :

**EXEMPLE 18 :**

- la (méthoxy - 2 phényl) - 4 (chloro - 2 éthyl) - 1 pipérazine, on obtient la :
  **{[(METHOXY - 2 PHENYL) - 4 PIPERAZINYL - 1] - 2 ETHYL} - 3 [3H] OXAZOLO [4,5b] PYRIDINONE - 2**

Rendement : 53 %
Point de fusion : 95 - 96 °C (voir tableau 1)

**EXEMPLE 19 :**

- la (chloro - 2 phényl) - 4 (chloro - 2 éthyl) - 1 pipérazine on obtient la :
  **{[(CHLORO - 2 PHENYL) - 4 PIPERAZINYL - 1] - 2 ETHYL} - 3 [3H] OXAZOLO [4,5b] PYRIDINONE - 2**

**EXEMPLE 20 :**

- la (méthyl - 2 phényl) - 4 (chloro - 2 éthyl) - 1 pipérazine, on obtient la :
  **{[(METHYL - 2 PHENYL) - 4 PIPERAZINYL - 1] - 2 ETHYL} - 3 [3H] OXAZOLO [4,5b] PYRIDINONE - 2**

**EXEMPLE 21 :**

- la (trifluorométhyl - 3 chloro - 4 phényl) - 4 (chloro - 2 éthyl) - 1 pipérazine, on obtient la :
  **{[(TRIFLUOROMETHYL - 3 CHLORO - 4 PHENYL) - 4 PIPERAZINYL - 1] - 2 ETHYL} - 3 [3H] OXAZOLO [4,5b] PYRIDINONE - 2**

**EXEMPLES 22 A 25 :**

En remplaçant dans l'exemple 14, mode opératoire 2 la phényl-1 pipérazine par :

**EXEMPLE 22 :**

- la (pyrimidinyl - 2) - 1 pipérazine, on obtient la :
  **{[(PYRIMIDINYL - 2) - 4 PIPERAZINYL - 1] - 2 ETHYL} - 3 [3H] OXAZOLO [4,5b] PYRIDINONE - 2**

Point de fusion : 150 °C

**EXEMPLE 23 :**

- la (fluoro - 4 phényl) - 1 pipérazine, on obtient la :
  **{[(FLUORO - 4 PHENYL) - 4 PIPERAZINYL - 1] - 2 ETHYL} - 3 [3H] OXAZOLO [4,5b] PYRIDINONE - 2**

Rendement : 95 %
Point de fusion : 94 °C - Voir tableau 1

**EXEMPLE 24 :**

- la (phénoxy - 4 phényl) - 1 pipérazine, on obtient la :
  **{[(PHENOXY - 4 PHENYL) - 4 PIPERAZINYL - 1] - 2 ETHYL} - 3 [3H] OXAZOLO [4,5b] PYRIDINONE - 2**

Rendement : 92 %
Point de fusion : 148 °C
Caractéristiques spectrales :
Infrarouge : 1760 $\nu$ CO
RMN $^1H$ ($COCl_3$) : $\delta$ = 6,8-6,9 et 7,2-7,3, 9H massif, protons aromatiques.

**EXEMPLE 25 :**

- la (chloro - 4 phényl) -1 pipérazine, on obtient la :
  **{[(CHLORO - 4 PHENYL) - 4 PIPERAZINYL - 1] - 2 ETHYL} - 3 [3H] OXAZOLO [4,5b] PYRIDINONE - 2**

Point de fusion : 110 °C
Caractéristiques spectrales :
Infrarouge : 1760 ν OCON

**EXEMPLE 26 :**

- la (pyridyl - 2) -1 pipérazine, on obtient la :
  **{[(PYRIDYL - 2) - 4 PIPERAZINYL - 1] - 2 ETHYL} - 3 [3H] OXAZOLO [4,5b] PYRIDINONE - 2**

Point de fusion : 148 °C
Caractéristiques spectrales :
Infrarouge : 1760 ν CO

**EXEMPLES 27 A 32 :**

En remplaçant dans les exemples 18 à 22, les aryl - 4 (chloro - 2 éthyl) - 1 pipérazines par les aryl - 4 (chloro - 3 propyl) - 1 pipérazines correspondantes, on obtient les :
**[(ARYL - 4 PIPERAZINYL - 1) - 3 PROPYL] - 3 [3H] OXAZOLO [4,5b] PYRIDINONES - 2**

**EXEMPLE 33 : [(PHENYL - 4 PIPERAZINYL - 1) - 2 ETHYL] - 3 METHYL - 5 [3H] OXAZOLO [4,5b] PYRIDINONE - 2**

En remplaçant dans l'exemple 14, la [3H] oxazolo [4,5b] pyridinone - 2 par la méthyl - 5 [3H] oxazolo [4,5b] pyridinone - 2 obtenue dans la préparation 5, on obtient le produit du titre.
Rendement : 50 %
Point de fusion : 100-102 °C

**EXEMPLE 34 : [(PHENYL - 4 PIPERAZINYL - 1) - 2 ETHYL] - 3 BROMO - 6 [3H] OXAZOLO [4,5b] PYRIDINONE - 2**

En remplaçant dans l'exemple 14, la [3H] oxazolo [4,5b] pyridinone - 2 par la bromo - 6 [3H] oxazolo [4,5b] pyridinone - 2 obtenue dans la préparation 7, on obtient le produit du titre.
Point de fusion : 110 °C

**EXEMPLE 35 : [((METHOXY - 4 PHENYL) - 4 PIPERAZINYL - 1) - 2 ETHYL] - 3 [3H] OXAZOLO [4,5b] PYRIDINONE - 2**

En remplaçant dans l'exemple 14, mode opératoire 1, la phényl - 4 (chloro - 2 éthyl) - 1 pipérazine par la (méthoxy - 4 phényl) - 4 (chloro - 2 éthyl) - 1 pipérazine, on obtient la [((méthoxy - 4 phényl) - 4 pipérazinyl - 1) - 2 éthyl] - 3 [3H] oxazolo [4,5b] pyridinone - 2.
Rendement : 48 %
Point de fusion : 125 °C

**EXEMPLE 36 : [((HYDROXY - 4 PHENYL) - 4 PIPERAZINYL - 1) - 2 ETHYL] - 3 [3H] OXAZOLO [4,5b] PYRIDINONE - 2**

A une solution de 5 grammes (14,1 mmol) de [((méthoxy - 4 phényl) - 4 pipérazinyl - 1) - 2 éthyl] - 3 [3H] oxazolo [4,5b] pyridinone - 2, dans 250 ml de dichlorométhane, ajouter 140 ml (140 mmole) d'une solution 1M de tribromure de bore dans le dichlorométhane, sous courant d'azote et à une température de -70 °C. Après addition, l'agitation est maintenue pendant 24 heures à température ambiante. Le milieu réactionnel est neutralisé par une solution molaire de carbonate acide de sodium, extrait par le chlorure de méthylène.
La phase organique est séchée, évaporée sous pression réduite. Le résidu est chromatographié sur colonne de silice (éluant cyclohexane, acétate d'éthyle 6/4).

Rendement : 30%

Point de fusion : 159 ° C

En procédant comme dans les exemples 14, mode opératoire 1, 15 à 21, mais en remplaçant les aryl - 4 (chloro - 2 éthyl) - 1 pipérazines et les aryl - 4 (chloro - 3 propyl) - 1 pipérazines par les :

- aryl - 4 (chloro - 4 n butyl) - 1 pipérazines, on obtient les [(aryl - 4 pipérazinyl - 1) - 4 butyl] - 3 [3H] oxazolo [4,5b] pyridinones - 2.
- aryl - 4 (chloro - 5 n pentyl) - 1 pipérazines, on obtient les [(aryl - 4 pipérazinyl - 1) - 5 pentyl] - 3 [3H] oxazolo [4,5b] pyridinones - 2.
- aryl - 4 (chloro - 6 n hexyl) - 1 pipérazines, on obtient les [(aryl - 4 pipérazinyl - 1) - 6 hexyl] - 3 [3H] oxazolo [4,5b] pyridinones - 2.
- aryl - 4 (méthyl - 1 chloro - 1 éthyl) - 1 pipérazines, on obtient les [(aryl - 4 pipérazinyl - 1) - 2 méthyl - 1 éthyl] - 3 [3H] oxazolo [4,5b] pyridinones - 2.

En procédant comme dans les exemples 14, mode opératoire 2, 22 à 25, mais en remplaçant la (bromo - 2 éthyl) - 3 [3H] oxazolo [4,5b] pyridinone - 2 par la :

- (bromo - 3 propyl) - 3 [3H] oxazolo [4,5b] pyridinone - 2, on obtient les [(aryl - 4 pipérazinyl - 1) - 3 propyl] - 3 [3H] oxazolo [4,5b] pyridinones - 2
- (bromo - 4 n butyl) - 3 [3H] oxazolo [4,5b] pyridinone - 2, on obtient les [(aryl - 4 pipérazinyl - 1) - 4 n butyl] - 3 [3H] oxazolo [4,5b] pyridinones - 2
- (bromo - 5 n pentyl) - 3 [3H] oxazolo [4,5b] pyridinone - 2, on obtient les [(aryl - 4 pipérazinyl - 1) - 5 n pentyl] - 3 [3H] oxazolo [4,5b] pyridinones - 2
- (bromo 6 n hexyl) - 3 [3H] oxazolo [4,5b] pyridinone - 2, on obtient les [(aryl - 4 pipérazinyl - 1) - 6 n hexyl] - 3 [3H] oxazolo [4,5b] pyridinones - 2

Les synthèses des exemples précédents sont également applicables aux dérivés des [3H] oxazolo [4,5b] pyridinones - 2 substituées sur le noyau aromatique par un ou plusieurs atomes d'halogène ou groupement alcoyle ou alkoxy inférieur, éventuellement substitué par un ou plusieurs atomes d'halogène.

**EXEMPLE 1A : [(PHENYL - 4 PIPERAZINYL - 1) - 2 ETHYLAMINO] - 2 PYRIDINOL - 3**

Placer 0,01 mole de [(phényl - 4 pipérazinyl - 1) - 2 éthyl] - 3 [3H] oxazolo [4,5b] pyridinone - 2 obtenue dans l'exemple 14 dans 50 ml de solution d'hydroxyde de sodium à 10%. Chauffer à reflux quatre heures sous agitation magnétique. Après refroidissement, la solution est acidifiée par de l'acide chlorhydrique à 30%. Ajouter, tout en refroidissant, une solution aqueuse saturée de bicarbonate de sodium jusqu'à PH = 7. Le précipité est filtré et lavé trois fois à l'eau, séché sous vide dans un dessicateur puis relavé à nouveau par du dichlorométhane.

Point de fusion : 191 ° C

En opérant comme dans l'exemple 1A, mais en utilisant comme matière première les composés obtenus dans les exemples 1 à 36 on obtient les :

- (aryl (substitués ou non)-4 pipérazinyl) alkyl amino - 2 pyridinol - 3 éventuellement substitués.

**ETUDE PHARMACOLOGIOUE DES DERIVES DE L'INVENTION**

**EXEMPLE A : Etude de la toxicité aiguë**

La toxicité aiguë a été appréciée après administration orale à des lots de 8 souris (26±2 grammes). Les animaux ont été observés à intervalles réguliers au cours de la première journée et quotidiennement pendant les 2 semaines suivant le traitement. La $DL_{50}$, dose entraînant la mort de 50 % des animaux, a été évaluée.

La $DL_{50}$ des produits selon l'invention qui ont été testés est supérieure à 1000 mg/kg à l'exception de celle des exemples 1 et 2 pour lesquels elle est voisine de 500 mg/kg, ce qui indique la faible toxicité des composés de l'invention.

**EXEMPLE B : Recherche de l'activité analgésique**

L'activité sur la douleur a été recherchée chez la souris (23-25 g) selon un protocole dérivé de la technique décrite par SIEGMUND (SIEGMUND E.A., R.A. CADMUS, & GOLU, J. Pharm. Exp. Ther. 119, 184, 1957). Les souris, réparties par randomisation en lots de 12 animaux, ont reçu le traitement par voie orale (excipient pour les témoins) 1 heure avant l'injection intra-péritonéale d'une solution hydroalcoolique de phényl-p-benzoquinone à 0,02 %. Les étirements sont dénombrés entre la 5ème et 10ème minute après

l'injection.

Le pourcentage d'activité obtenu a été évalué pour chaque dose (% de diminution du nombre d'étirements chez les traités par rapport aux témoins). Une $ED_{50}$, dose entraînant une activité de 50 %, a été déterminée pour chaque produit testé.

Il est apparu que les composés de l'invention possédent une activité analgésique très intéressante.

Ainsi l'$ED_{50}$ du composé des exemples 14 et 15 est voisine du $mg.kg^{-1}$.

A titre de comparaison l'$ED_{50}$ du produit de l'exemple 6 (Fluoro - 2 phényl) - 2 oxazolo [4,5b] pyridine) du brevet US 4 038 396 possède dans le même test une dose efficace 50 voisine de 12 $mg.kg^{-1}$.

**EXEMPLE C : Etude de l'activité anti-inflammatoire**

Le potentiel anti-inflammatoire des composés a été recherché sur un modèle d'inflammation aiguë provoquée par injection sous-cutanée d'une solution de carragénine au niveau de la patte postérieure de rat, selon une technique inspirée de la méthode de WINTERC.H,E.A. RISLEY, G.N. NUSS - Proc. Soc. Exp.Med.111, 554, 1962). Les rats (100-120 g), randomisés en lot de 8, ont été traités (y compris les témoins qui reçoivent l'excipient) 1 heure avant l'injection locale d'une suspension à 0,5 % de carragénine (type IV, Sigma ; 0,1 ml par rat). L'oedème est déterminé 3 heures après l'injection, par mesure pléthysmométrique (pléthysmomètre à eau UGO BASILE) du volume de chacune des pattes postérieures (oedème = volume patte enflammée - volume patte non enflammée).

Le pourcentage d'activité correspond au pourcentage de diminution de l'oedème moyen du lot comparativement à la moyenne du lot témoin correspondant. Une $ED_{30}$, dose entraînant une activité de 30 %, a été déterminée.

Cette $ED_{30}$ est égale à 50 $mg.kg^{-1}$ pour le composé de l'exemple 6 du brevet US 4 038 396. Elle est très nettement supérieure à cette valeur pour tous les composés de l'invention.

L'étude pharmacologique des produits de l'invention montre donc que ces produits sont peu toxiques, doués d'une activité analgésique plus intense que celle des composés de structure voisine de l'art antérieur et dépourvue d'activité anti-inflammatoire contrairement à ces mêmes composés de l'art antérieur.

**EXEMPLE D : Composition pharmaceutique : COMPRIME**

Comprimés dosés à 25 mg de (phényl - 4 pipérazinyl - 1) - 2 éthyl - 3 [3H] oxazolo [4,5b] pyridinone - 2.

| Formule de préparation pour 1000 comprimés. | |
|---|---|
| (Phényl - 4 pipérazinyl - 1) - 2 éthyl - 3 [3H] oxazolo [4,5b] pyridinone - 2 | 25 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

TABLEAU I : CARACTERISTIQUES SPECTRALES DE QUELQUES COMPOSES DE L'INVENTION

(formules : voir annexe)

| EX | p* | R | Infrarouge | Résonance Magnétique Nucléaire (δ : ppm) |
|---|---|---|---|---|
| 1 | 1 | H | 3100 - 2785 :ν CH<br>1750 : ν CO<br>1590 : ν C = C conjugé | δ:2,91-2,96;4H;m;A-A' δ:3,18-3,22;4H;m;B-B'<br>δ:5,00;2H;s;$N-CH_2-N$; δ:6,80-6,92 ppm;3H;m;Ha,a',c<br>δ:7,03;1H;dd,$H_6$,$JH_6H_7$=8,3Hz et $JH_6H_5$=5,4Hz<br>δ:7,2-7,3 (2H,m):Hb,b'<br>δ:7,37;1H;dd;$H_7$;$JH_6H_7$=8,3Hz et $JH_5H_7$=1Hz<br>δ:8,10;1H;dd;$H_5$;$JH_5H_6$=5,4Hz et $JH_5H_7$=1Hz |
| 2 | 1 | $mCF_3$ | 3100 - 2785 :ν CH<br>1750 : ν CO<br>1590 : ν C = C conjugé | δ:2,85-3,05;4H;m;AA' δ:3,15-3,35 ppm;4H;BB'<br>δ:5,00;2H;s;$N-CH_2-N$ δ:6,9-7,15 ppm;4H;m;Ha,a',c et $H_6$<br>δ:7,20-7,45;2H;m;$H_7$ et Hb<br>δ:8,10;1H;dd;$H_5$;$JH_5H_6$=4,8Hz et $JH_5H_7$=1Hz |
| 14 | 2 | H | 3000 - 2700 :ν CH<br>1750 : ν CO<br>1590 : νC=C conjugé | δ:2,66-2,72;4H;m;AA' δ:2,85;2H;t;$CH_2$-<u>$CH_2$</u>-pipe;J=6,6hz<br>δ:3,07-3,13;4H;m;BB' δ:4,10;2H;t;<u>$CH_2$</u>-$CH_2$-pipe;J=6,6Hz<br>δ:6,79-6,91;3H;m;Ha,a';c δ:7,19-7,27;2H;m;Hb,b'<br>δ:7,03;1H;dd;$H_6$;$JH_6H_7$=8Hz et $JH_5H_6$=5,4Hz<br>δ:7,38;1H;dd;H7;$JH_5H_7$=0,3Hz et$JH_6H_7$=8Hz<br>δ:8,10;1H;dd;$H_5$;$JH_5H_6$:5,4Hz et $JH_5H_7$=0,3Hz |

m : massif ; s : singulet ; dd : doublet dédoublé ; pipé : pipérazine ; t : triplet ; *P = n + 1 lorsque B=H T = H

**TABLEAU I : CARACTERISTIQUES SPECTRALES DE QUELQUES COMPOSES DE L'INVENTION**

**(formule : voir annexe)**

| EX | p* | R | Infrarouge | Résonance Magnétique Nucléaire (δ : ppm) |
|---|---|---|---|---|
| 15 | 2 | $mCF_3$ | 3000-2700 : ν CH<br>1750 : ν CO<br>1590 : ν C=C conjugé | 2,65-2,75 ppm:4H;m;AA' 2,85;2H;t;$CH_2$-$\underline{CH_2}$-pipé;J=6,6Hz<br>3,0-3,2 ppm:4H;m;BB' 4,10;2H;t;$\underline{CH_2}$-$CH_2$;pipé;J=6,6Hz<br>6,90-7,15;4H;m;$H_6$ et Ha,a',c 7,25-7,45;2H;m;$H_7$ et Hb<br>8,10;1H; dd;$H_5$;$JH_5H_7$=0,3Hz et $JH_5H_6$=4,8Hz |
| 16 | 3 | H | 3000-2700 :ν CH<br>1750 : ν CO<br>1590 : ν C=C conjugé | 2,0-2,1;2H;m;$CH_2$-$CH_2$-$\underline{CH_2}$-pipé 2,46-2,56;6H;m;$CH_2\underline{CH_2}CH_2$ pipé,AA'<br>3,0-3,1;4H:m:BB' 4,07;2H;t;$\underline{CH_2}$-$CH_2$-$CH_2$-pipé;J=6,6Hz<br>7,03;1H;dd;$H_6$;J=$H_6H_7$=8Hz et 7,19-7,28;2H;m;Hbb'<br>7,37;1H;dd;JH6H7=8Hz et JH5H7=0,3Hz:H7<br>6,80-6,92;3H;m;Haa'c et $JH_6H_7$=8Hz<br>8,10;1H;dd;$H_5$;$JH_5H_7$=0,3Hz et $JH_5H_6$=5,4Hz |
| 17 | 3 | $mCF_3$ | 3000-2700 :ν CH<br>1750 : ν CO<br>1590 : ν C=C conjugé | 2,0-2,1;2H;m;$CH_2CH_2\underline{CH_2}$pipé 2,45-2,55;6H;m;$CH_2\underline{CH_2}CH_2$ pipé,AA'<br>3,0-3,1;4H:m:BB' 4,07;2H;t;$\underline{CH2}$-$CH\underline{2}$-$CH\underline{2}$-pipé;J=6,6Hz<br>6,9-7,15;4H;m;$H\underline{6}$ et Haa'b 7,25-7,45;2H;m;$H\underline{7}$ et Hb<br>8,10;1H;dd;$H\underline{5}$;$JH\underline{5}H\underline{7}$=0,3Hz et $JH5H\underline{6}$=5,4,Hz |

m : massif ; t : triplet ; dd : doublet dédoublé ; pipé : pipérazine ; t : triplé ; p = n + 1 lorsque B = H
T = H

**TABLEAU I : CARACTERISTIQUES SPECTRALES DE QUELQUES COMPOSES DE L'INVENTION**

**(formule : voir annexe)**

| EX | p | R | T | Infrarouge | Résonance Magnétique Nucléaire (δ : ppm) |
|---|---|---|---|---|---|
| 18 | 2 | $OCH_3$ | H | 3100-2630 : ν CH<br>1780 : ν C=O<br>1590 : ν C=C conjugé | 2,69-2,78; 4H;m;AA' 2,86;2H;t;$CH_2$-$\underline{CH_2}$-pipé;J=6,7Hz<br>2,92-3,03;4H;m;BB' 3,84;3H;s;-O$\underline{CH_3}$<br>4,10;2H;t;$\underline{CH_2}$-$CH_2$ pipé J=6,7Hz 6,82-6,90;2H;m;$H_a$'Hz<br>6,92-7,02;2H;m;Hb,Hb' 7,03;1H;dd;H6; $JH_6$-$H7$=7,6Hz<br>7,38;1H;dd;$H_7$;$JH_7$-$H_6$=7,6Hz;$JH_7h_5$=Hz<br>8,09;1H;dd;$H_5$;$JH_5$-$H_6$=5,0Hz;$JH_5H_7$;1Hz |
| 23 | 2 | pF | H | 3100-2600 : ν CH<br>1720 : ν CO<br>1590 : ν C=C conjugé | 2,69-2,78;4H;m;AA' 2,88;2H;t;$CH_2\underline{CH_2}$ pipé;J=6,3Hz<br>3,01-3,10;4H:m:BB' 4,12;2H;t;$\underline{CH_2}CH_2$ pipé;J=6,3Hz<br>6,79-6,87;2H;m;HaHa' 6,88-6,97;2H;m;HbHb'<br>7,04;1H;dd;$H_6$;$JH_6H_7$=7,3Hz ; $JH_6H_5$=5,3Hz<br>7,38;1H;dd;Hz; $JH_7H_6$=7,7Hz ;JH7H5=1Hz<br>8,10;1H;dd;$H_5$;$JH_5H_6$=5,3Hz et $JH_5H_7$=1Hz |
| 31 | 2 | H | 5-$CH_3$ | 3000-2610 : ν CH<br>1770 : ν CO<br>1590 : ν C=C conjugé | 2,51;3H;s;-$CH_3$ 2,66-2,74;4H;m;AA'<br>2,82;2H;t;$CH_2$-$\underline{CH_2}$-pipé;J=6,4Hz 3,05-3,14;4H;m;BB'<br>4,08;2H;t;$\underline{CH_2}$-$CH_2$ pipé;J=6,4Hz 6,80-6,90;4H;m;$H_6$ Ha,Ha',$H_6$<br>7,2-7,3;3H;m;$H_7$,Hb,Hb' |

m : massif ; t : triplet ; dd : doublet dédoublé ; pipé : pipérazine ; t : triplé ; p = n + 1 lorsque B = H
T = H

## ANNEXE

### FORMULE TABLEAU

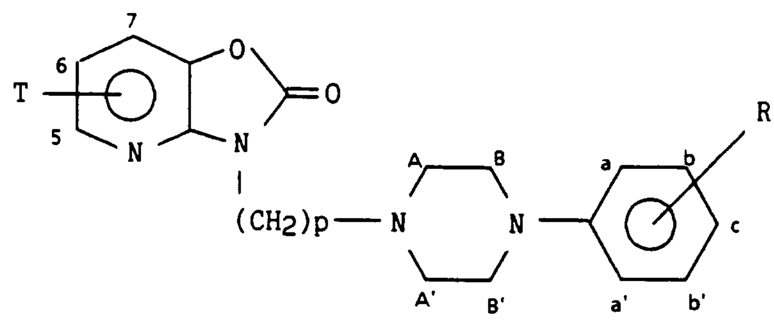

### NOMENCLATURE

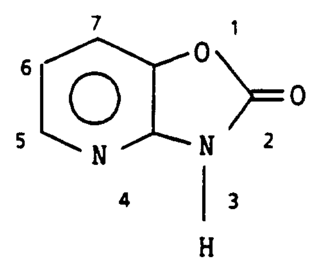

[3H] oxazolo 4,5b pyridinone - 2

## Revendications

### Revendications pour les Etats contractants suivants : AT, BE, CH, DE, DK, FR, GB, IT, LI, LU, NL, SE

**1.** Composé de formule générale (I) :

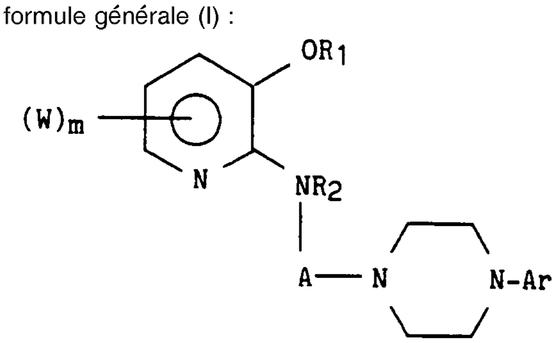

(I)

dans laquelle :

| | |
|---|---|
| $R_1$ et $R_2$ | représentent chacun un atome d'hydrogène ou forment avec l'oxygène et l'azote qui les portent un chaînon-O-CO-N, |
| W | représente un atome d'halogène ou un groupement alkyle ou alkoxy inférieur éventuel- |

| | |
|---|---|
| | lement substitué par un ou plusieurs atomes d'halogène tel que trifluorométhyle, et m étant compris entre 0 et 3, |
| A | représente un goupement alcoyle inférieur linéaire ou ramifié, |
| Ar | représente un groupement aryle ou hétéroaryle éventuellement substitué par un ou plusieurs atomes d'halogène ou par un ou plusieurs groupements alkyle inférieur, hydroxy, hydroxysulfonyloxy, ou alkoxy inférieur ou aryloxy éventuellement substitués par un ou plusieurs atomes d'halogène tel que le groupement trifluorométhyle, |

étant entendu que par radical alkyle inférieur ou alkyloxy inférieur, on entend un groupement alkyle linéaire ou ramifié comprenant de 1 à 6 atomes de carbone,
et que par groupements aryle ou hétéroaryle, on entend des groupements mono ou bicycliques insaturés comprenant de 5 à 12 atomes intégrant ou non dans leur squelette carbone, un, deux ou trois hétéroatomes choisis parmi azote, oxygène ou soufre,
leurs isomères,
ainsi que leurs sels d'addition à un acide pharmaceutiquement acceptable et lorsque $R_1$ et $R_2$ représentent un atome d'hydrogène, leurs sels d'addition à une base pharmaceutiquement acceptable.

**2.** Composés selon la revendication 1 pour lesquels $R_1$ et $R_2$ forment ensemble un groupement CO, de formule (I/A) :

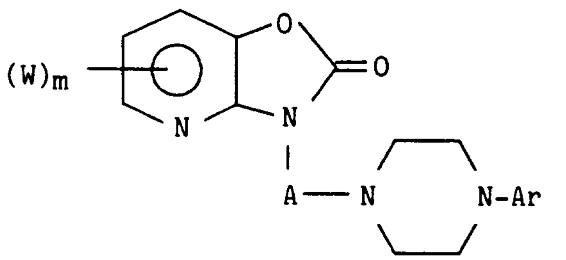

(I/A)

leurs isomères ainsi que leurs sels d'addition à un acide pharmaceutiquement acceptable.

**3.** Composés selon la revendication 1 pour lesquels $R_1$ et $R_2$ représentent chacun un atome d'hydrogène de formule (I/B) :

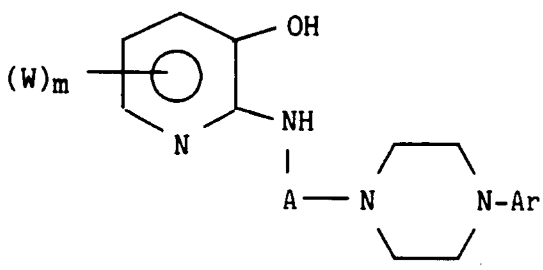

(I/B)

leurs isomères ainsi que leurs sels d'addition à un acide pharmaceutiquement acceptable ou à une base pharmaceutiquement acceptable.

**4.** Composés selon la revendication 1, pour lesquels Ar représente un groupement phényle éventuellement substitué par un atome d'halogène ou par un groupement alkyle inférieur, hydroxy, hydroxysulfonyloxy, ou alkoxy inférieur, eux-mêmes éventuellement substitués par un ou plusieurs atomes d'halogène ainsi que leurs sels d'addition à un acide pharmaceutiquement acceptable ou lorsque $R_1$ et $R_2$ représentent un atome d'hydrogène, leurs isomères et leurs sels d'addition à une base pharmaceutiquement acceptable.

**5.** Composé selon l'une des revendications 1 et 2 qui est la [(Trifluorométhyl - 3 phényl) - 4 pipérazinyl - 1] méthyl - 3 [3H] oxazolo [4,5b] pyridinone - 2 ainsi que ses sels d'addition à un acide pharmaceutiquement acceptable.

**6.** Composé selon l'une des revendications 1 et 2 qui est la (Phényl - 4 pipérazinyl - 1) méthyl - 3 [3H] oxazolo (4,5b] pyridinone - 2 ainsi que ses sels d'addition à un acide pharmaceutiquement acceptable.

**7.** Composé selon l'une des revendications 1 et 2 qui est la {[(Trifluorométhyl - 3 phényl) - 4 pipérazinyl - 1] - 2 éthyl} - 3 [3H] oxazolo [4,5b] pyridinone - 2 ainsi que ses sels d'addition à un acide pharmaceutiquement acceptable.

**8.** Composé selon l'une des revendications 1 et 2 qui est la {[(Trifluorométhyl - 3 phényl) - 4 pipérazinyl - 1] - 3 propyl} - 3 [3H] oxazolo [4,5b] pyridinone - 2 ainsi que ses sels d'addition à un acide pharmaceutiquement acceptable.

**9.** Composé selon l'une des revendications 1 et 2 qui est la [(Phényl - 4 pipérazinyl - 1) - 2 éthyl] - 3 [3H] oxazolo [4,5b] pyridinone - 2 ainsi que ses sels d'addition à un acide pharmaceutiquement acceptable.

**10.** Composé selon l'une des revendications 1 et 2 qui est la {[(Pyridyl - 2) - 4 pipérazinyl - 1} - 2 éthyl] - 3 [3H] oxazolo [4,5b] pyridinone - 2 ainsi que ses sels d'addition à un acide pharmaceutiquement acceptable.

**11.** Composé selon l'une des revendications 1 et 2 qui est la {[(Pyrimidinyl - 2) - 4 pipérazinyl - 1} - 2 éthyl] - 3 [3H] oxazolo [4,5b] pyridinone - 2 ainsi que ses sels d'addition à un acide pharmaceutiquement acceptable.

**12.** Composé selon l'une des revendications 1 et 3 qui est le [(Phényl - 4 pipérazinyl - 1) - 2 éthylamino] - 2 pyridinol - 3 ainsi que ses sels d'addition à un acide ou une base pharmaceutiquement acceptable.

**13.** Procédé de préparation des composés de formule (I) caractérisé en ce que l'on fait réagir un dérivé de formule (II) :

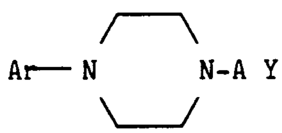

(II)

dans laquelle A et Ar ont la même signification que dans la formule (I), et Y représente un atome d'halogène,
après dissolution dans un solvant organique avec un dérivé de formule (III) :

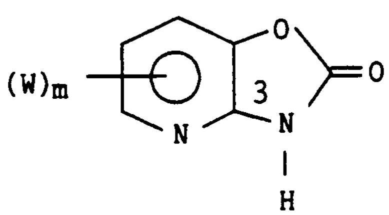

(III)

dans laquelle W et m ont la même signification que dans la formule (I),
préférentiellement sous forme de son dérivé sodé en 3 d'addition en 3 à un métal alcalin
pour conduire après chauffage du milieu réactionnel, refroidissement, filtration, évaporation du milieu réactionnel, reprise par l'eau, extraction par un solvant organique et éventuelle purification par chromatographie sur colonne de silice,
à un dérivé de formule (I/A) :

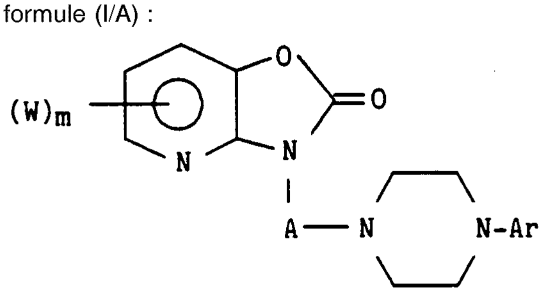

(I/A)

cas particulier des dérivés de formule (I) pour lesquels $R_1$ et $R_2$ forment avec l'azote et l'oxygène qui les portent un chaînon O-CO-A-,
que l'on peut si nécessaire, séparer en ses isomères et, si on le désire, salifier par un acide pharmaceutiquement acceptable,
dérivé de formule (I/A) que l'on peut traiter, si on le désire, par une solution aqueuse d'un agent alcalin, à une température comprise entre la température ambiante et la température d'ébullition du milieu réactionnel pour conduire, après neutralisation ou acidification du milieu réactionnel et éventuelle purification, à un dérivé de formule (I/B) :

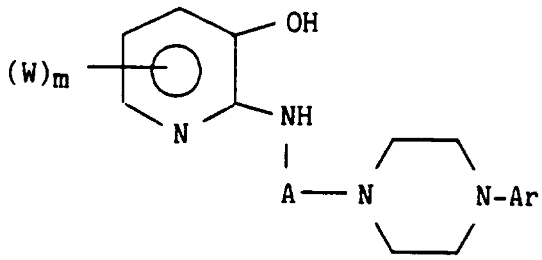

(I/B)

cas particulier des dérivés de formule (I) pour lesquels $R_1$ et $R_2$ représentent chacun un atome d'hydrogène,
que l'on peut si nécessaire séparer en ses isomères et salifier, si on le désire, par un acide ou une base pharmaceutiquement acceptable.

**14.** Procédé de préparation de composés de formule (I/A) selon la revendication 2 dans laquelle A représente un groupement $CH_2$, caractérisé en ce que l'on dissout en milieu d'alcool aliphatique inférieur,
d'une part un dérivé de formule (III) :

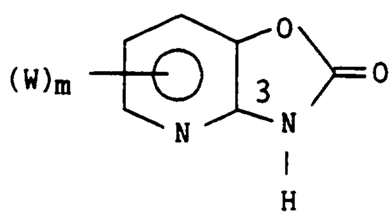

(III)

dans laquelle W et m ont la même signification que dans la formule (I),
d'autre part un léger excès d'une aryl pipérazine de formule (VII) :

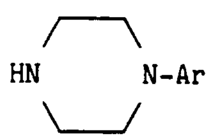

(VII)

dans laquelle Ar a la même signification que dans la formule (I),
et d'autre part un excès de formol,
pour conduire, après chauffage de la solution obtenue à une température comprise entre la température ambiante et la température d'ébullition, éventuel refroidissement, repos d'une à deux heures, filtration et éventuelle chromatographie sur colonne de silice à un dérivé de formule (I/A1) :

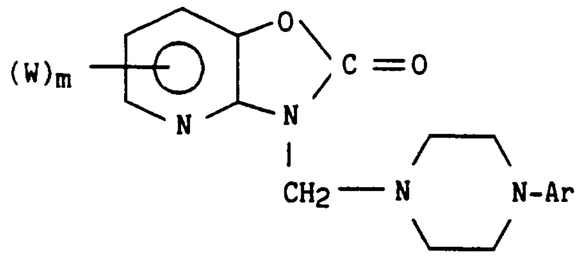

(I/A1)

dans laquelle W, m et Ar ont la même signification que dans la formule (I), que l'on peut salifier si on le désire par un acide pharmaceutiquement acceptable.

**15.** Procédé de préparation des composés de formule (I/A) selon la revendication 2 caractérisé en ce que l'on soumet à réaction un dérivé de formule (III) :

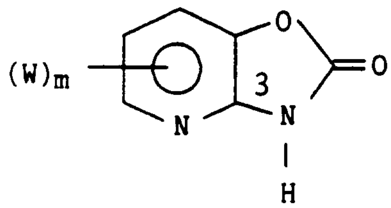

(III)

dans laquelle W et m ont la même signification que dans la formule (I), avec l'hydroxyde de métal alcalin en milieu aqueux ou un alcoolate de métal alcalin en milieu organique, pour conduire à un dérivé de formule (IV) :

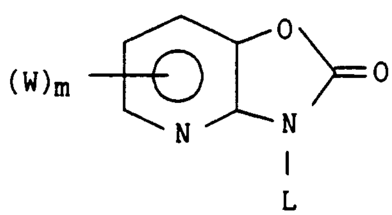

(IV)

dans laquelle W et m ont la même signification que précédemment, et L représente un métal alcalin que l'on condense avec un dérivé de formule (V) :

**X - A - X'    (V)**

dans laquelle A a la même signification que dans la formule (I), X et X' identiques ou différents représentent un atome d'halogène, préférentiellement sous atmosphère inerte, en milieu organique, préférentiellement à température du reflux du solvant choisi pour conduire, après éventuelle extraction et purification par chromatographie à un dérivé de formule (VI) :

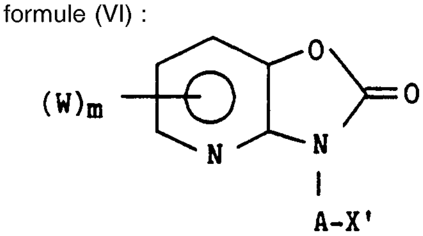

(VI)

dans laquelle W, X', m et A ont la même signification que précédemment, que l'on condense, préférentiellement sous atmosphère inerte avec un dérivé de formule (VII), préférentiellement en excès :

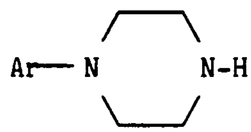

(VII)

dans laquelle Ar a la même définition que dans la formule (I), en milieu organique en présence d'un excès d'une amine tertiaire et à température du reflux du solvant choisi pour conduire après refroidissement, extraction et éventuelle purification par cristallisation à un dérivé de formule (I/A), que l'on peut, si nécessaire, séparer en ses isomères et si on le désire, salifier par un acide pharmaceutiquement acceptable.

**16.** Procédé selon la revendication 15 de préparation de composés de formule (I/A) dans laquelle A représente un chaînon alkyle inférieur linéaire caractérisé en ce que l'on soumet à réaction un dérivé de formule (III) :

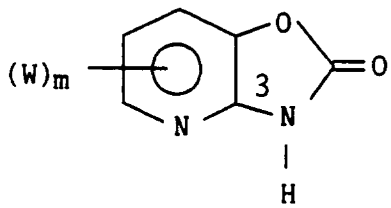

(III)

dans laquelle W et m ont la même signification que dans la formule (I), avec un hydroxyde de métal alcalin en milieu aqueux ou un alcoolate de métal alcalin en milieu organique, pour conduire à un dérivé de formule (IV) :

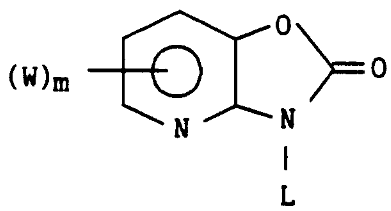

(IV)

dans laquelle W et m ont la même signification que dans la formule (I), et L représente un métal alcalin que l'on condense avec un dérivé de formule (VIII) :

**X - A - X (VIII)**

dans laquelle A a la même signification que dans la formule (I), X représente un atome d'halogène, préférentiellement sous atmosphère inerte, en milieu organique, préférentiellement à température du reflux du solvant choisi pour conduire, après éventuelle extraction et purification par chromatographie à un dérivé de formule (VI) :

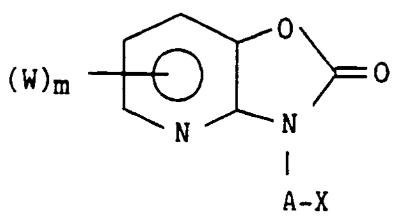

(VI)

dans laquelle W, X, m et A ont la même signification que précédemment, que l'on condense, préférentiellement sous atmosphère inerte avec un dérivé de formule (VII), préférentiellement en excès :

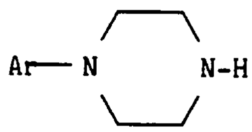

(VII)

dans laquelle Ar a la même définition que précédemment,
en milieu organique en présence d'un excès d'une amine tertiaire et à température du reflux du solvant choisi pour conduire après refroidissement, extraction et éventuelle purification par cristallisation à un dérivé de formule (I/A) pour lequel A représente un chaînon alkyl linéaire,
que l'on peut, si nécessaire, séparer en ses isomères et si on le désire, salifier par un acide pharmaceutiquement acceptable.

**17.** Procédé de préparation de composé de formule (I/B) selon la revendication III caractérisé en ce que l'on soumet un composé de formule (I/A) :

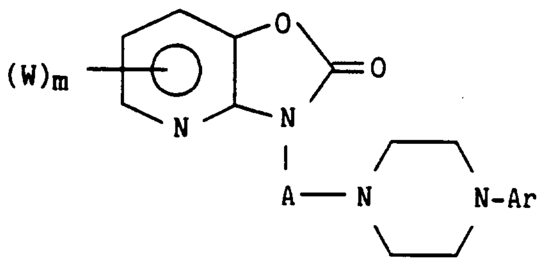

(I/A)

dans laquelle W, m, A, Ar ont la même signification que dans la formule (I), ou l'un de ses sels d'addition à un acide pharmaceutiquement acceptables à un agent alcalin en solution aqueuse à température comprise entre la température ambiante et la température d'ébullition du milieu réactionnel pour conduire, après neutralisation ou acidification du milieu réactionnel à un composé de formule (I/B) que l'on salifie, si on le souhaite, par un acide ou une base pharmaceutiquement acceptable après éventuelle purification.

**18.** Composition pharmaceutique contenant comme principe actif au moins un composé selon l'une des revendications 1 à 12 en combinaison avec un ou plusieurs excipients ou véhicules inertes, non-toxiques, pharmaceutiquement acceptables.

**19.** Composition pharmaceutique selon la revendication 18 contenant au moins un principe actif selon l'une des revendications 1 à 12 utilisables dans le traitement d'algies.

**Revendications pour l'Etat contractant suivant : GR**

**1.** Procédé de préparation de composés de formule générale (I) :

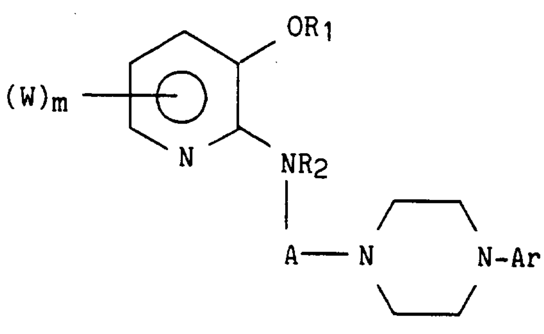

(I)

dans laquelle :

$R_1$ et $R_2$ représentent chacun un atome d'hydrogène ou forment avec l'oxygène et l'azote qui les portent un chaînon-O-CO-N,

W représente un atome d'halogène ou un groupement alkyle ou alkoxy inférieur éventuellement substitué par un ou plusieurs atomes d'halogène tel que trifluorométhyle, et m étant compris entre 0 et 3,

A représente un goupement alcoyle inférieur linéaire ou ramifié,

Ar représente un groupement aryle ou hétéroaryle éventuellement substitué par un ou plusieurs atomes d'halogène ou par un ou plusieurs groupements alkyle inférieur, hydroxy, hydroxysulfonyloxy, ou alkoxy inférieur ou aryloxy éventuellement substitués par un ou plusieurs atomes d'halogène tel que le groupement trifluorométhyle,

étant entendu que par radical alkyle inférieur ou alkyloxy inférieur, on entend un groupement alkyle linéaire ou ramifié comprenant de 1 à 6 atomes de carbone,

et que par groupements aryle ou hétéroaryle, on entend des groupements mono ou bicycliques insaturés comprenant de 5 à 12 atomes intégrant ou non dans leur squelette carbone, un, deux ou trois hétéroatomes choisis parmi azote, oxygène ou soufre,

leurs isomères,

ainsi que leurs sels d'addition à un acide pharmaceutiquement acceptable et lorsque $R_1$ et $R_2$ représentent un atome d'hydrogène, leurs sels d'addition à une base pharmaceutiquement acceptable,

caractérisé en ce que l'on fait réagir un dérivé de formule (II) :

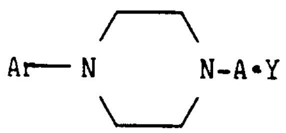

(II)

dans laquelle A et Ar ont la même signification que dans la formule (I), et Y représente un atome d'halogène,

après dissolution dans un solvant organique avec un dérivé de formule (III) :

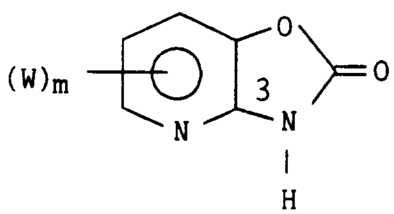

(III)

dans laquelle W et m ont la même signification que dans la formule (I),

préférentiellement sous forme de son dérivé sodé en 3 d'addition en 3 à un métal alcalin

pour conduire après chauffage du milieu réactionnel, refroidissement, filtration, évaporation du milieu réactionnel, reprise par l'eau, extraction par un solvant organique et éventuelle purification par chromatographie sur colonne de silice,

à un dérivé de formule (I/A) :

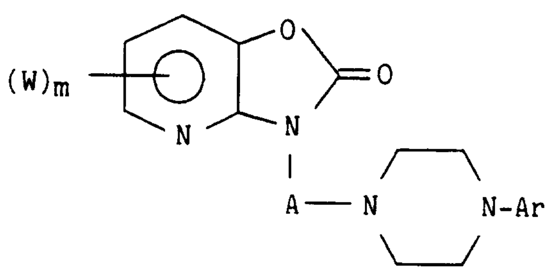

(I/A)

cas particulier des dérivés de formule (I) pour lesquels $R_1$ et $R_2$ forment avec l'azote et l'oxygène qui les portent un chaînon O-CO-A-,

que l'on peut si nécessaire, séparer en ses isomères et, si on le désire, salifier par un acide pharmaceutiquement acceptable,

dérivé de formule (I/A) que l'on peut traiter, si on le désire, par une solution aqueuse d'un agent alcalin, à une température comprise entre la température ambiante et la température d'ébullition du milieu

réactionnel pour conduire, après neutralisation ou acidification du milieu réactionnel et éventuelle purification, à un dérivé de formule (I/B) :

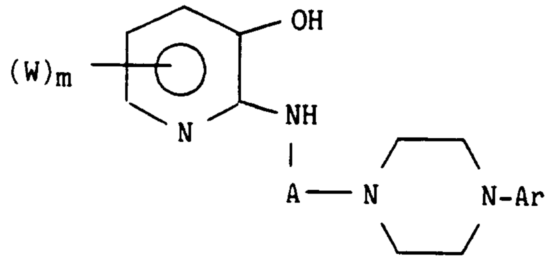

(I/B)

cas particulier des dérivés de formule (I) pour lesquels $R_1$ et $R_2$ représentent chacun un atome d'hydrogène,

que l'on peut si nécessaire séparer en ses isomères et salifier, si on le désire, par un acide ou une base pharmaceutiquement acceptable.

**2.** Procédé de préparation de composés de formule (I/A) selon la revendication 1 dans laquelle A représente un groupement $CH_2$, caractérisé en ce que l'on dissout en milieu d'alcool aliphatique inférieur,

d'une part un dérivé de formule (III) :

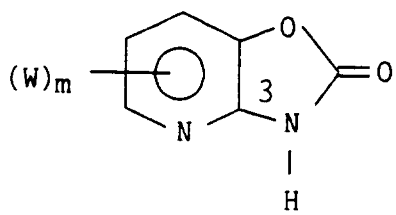

(III)

dans laquelle W et m ont la même signification que dans la formule (I),

d'autre part un léger excès d'une aryl pipérazine de formule (VII) :

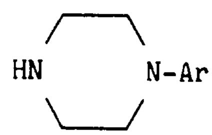

(VII)

dans laquelle Ar a la même signification que dans la formule (I),

et d'autre part un excès de formol,

pour conduire, après chauffage de la solution obtenue à une température comprise entre la température ambiante et la température d'ébullition, éventuel refroidissement, repos d'une à deux heures, filtration et éventuelle chromatographie sur colonne de silice à un dérivé de formule (I/A1) :

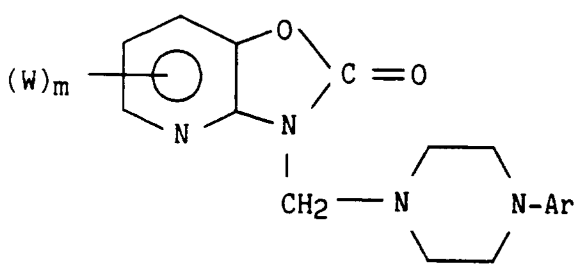

(I/A1)

dans laquelle W, m et Ar ont la même signification que dans la formule (I),

que l'on peut salifier si on le désire par un acide pharmaceutiquement acceptable.

3. Procédé de préparation des composés de formule (I/A) selon la revendication 1 caractérisé en ce que l'on soumet à réaction un dérivé de formule (III) :

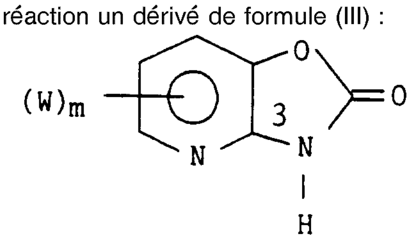

**(III)**

dans laquelle W et m ont la même signification que dans la formule (I),
avec l'hydroxyde de métal alcalin en milieu aqueux ou un alcoolate de métal alcalin en milieu organique, pour conduire à un dérivé de formule (IV) :

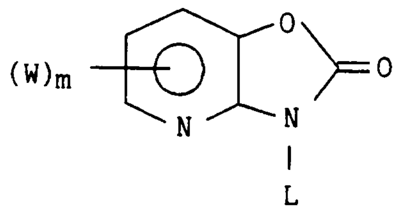

**(IV)**

dans laquelle W et m ont la même signification que précédemment, et L représente un métal alcalin que l'on condense avec un dérivé de formule (V) :

**X - A - X' (V)**

dans laquelle A a la même signification que dans la formule (I), X et X' identiques ou différents représentent un atome d'halogène,
préférentiellement sous atmosphère inerte, en milieu organique, préférentiellement à température du reflux du solvant choisi pour conduire, après éventuelle extraction et purification par chromatographie à un dérivé de formule (VI) :

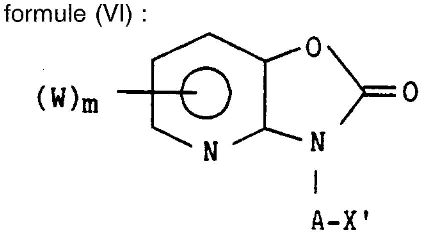

**(VI)**

dans laquelle W, X', m et A ont la même signification que précédemment,
que l'on condense, préférentiellement sous atmosphère inerte avec un dérivé de formule (VII), préférentiellement en excès :

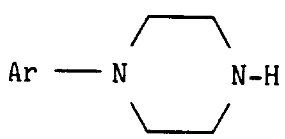

**(VII)**

dans laquelle Ar a la même définition que dans la formule (I),
en milieu organique en présence d'un excès d'une amine tertiaire et à température du reflux du solvant choisi pour conduire après refroidissement, extraction et éventuelle purification par cristallisation à un dérivé de formule (I/A),
que l'on peut, si nécessaire, séparer en ses isomères et si on le désire, salifier par un acide pharmaceutiquement acceptable.

4. Procédé selon la revendication 3 de préparation de composés de formule (I/A) dans laquelle A représente un chaînon alkyle inférieur linéaire caractérisé en ce que l'on soumet à réaction un dérivé de formule (III) :

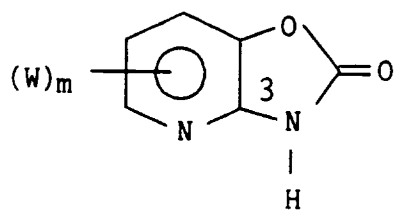

(III)

dans laquelle W et m ont la même signification que dans la formule (I),
avec un hydroxyde de métal alcalin en milieu aqueux ou un alcoolate de métal alcalin en milieu organique, pour conduire à un dérivé de formule (IV) :

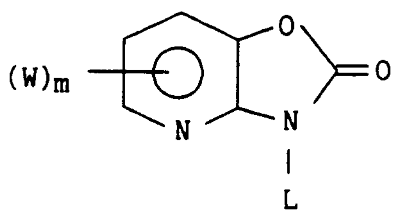

(IV)

dans laquelle W et m ont la même signification que dans la formule (I), et L représente un métal alcalin que l'on condense avec un dérivé de formule (VIII) :

**X - A - X (VIII)**

dans laquelle A a la même signification que dans la formule (I), X représente un atome d'halogène, préférentiellement sous atmosphère inerte, en milieu organique, préférentiellement à température du reflux du solvant choisi pour conduire, après éventuelle extraction et purification par chromatographie à un dérivé de formule (VI) :

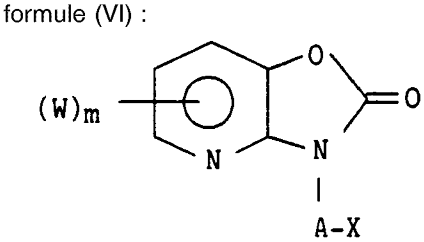

(VI)

dans laquelle W, X, m et A ont la même signification que précédemment,
que l'on condense, préférentiellement sous atmosphère inerte avec un dérivé de formule (VII), préférentiellement en excès :

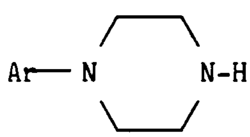

(VII)

dans laquelle Ar a la même définition que précédemment,
en milieu organique en présence d'un excès d'une amine tertiaire et à température du reflux du solvant choisi pour conduire après refroidissement, extraction et éventuelle purification par cristallisation à un dérivé de formule (I/A) pour lequel A représente un chaînon alkyl linéaire,
que l'on peut, si nécessaire, séparer en ses isomères et si on le désire, salifier par un acide pharmaceutiquement acceptable.

**5.** Procédé de préparation de composé de formule (I/B) selon la revendication 1 caractérisé en ce que l'on soumet un composé de formule (I/A) :

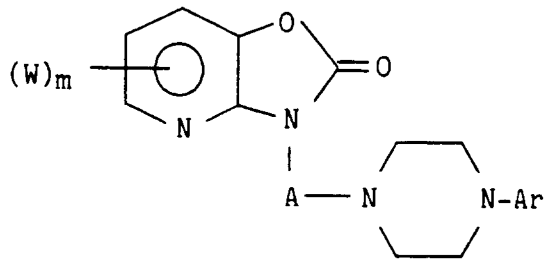

**(I/A)**

dans laquelle W, m, A, Ar ont la même signification que dans la formule (I), ou l'un de ses sels d'addition à un acide pharmaceutiquement acceptables à un agent alcalin en solution aqueuse à température comprise entre la température ambiante et la température d'ébullition du milieu réactionnel pour conduire, après neutralisation ou acidification du milieu réactionnel à un composé de formule (I/B) :

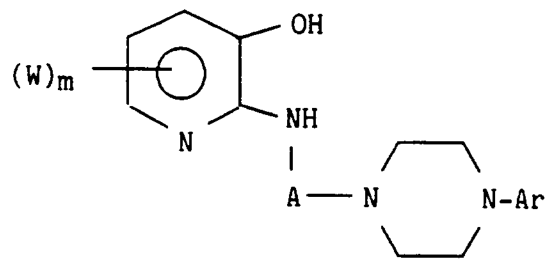

**(I/B)**

dans laquelle W, m, A, Ar ont la même signification que précédemment,
que l'on salifie, si on le souhaite, par un acide ou une base pharmaceutiquement acceptable après éventuelle purification.

**6.** Procédé de préparation selon la revendication 1 de composés pour lesquels $R_1$ et $R_2$ forment ensemble un groupement CO, de formule (I/A) :

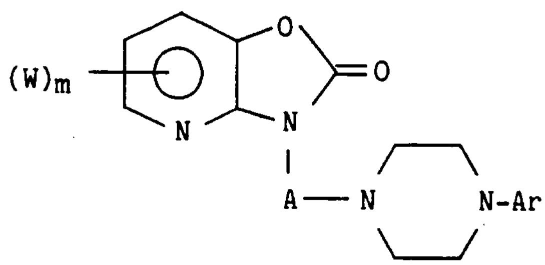

**(I/A)**

leurs isomères ainsi que leurs sels d'addition à un acide pharmaceutiquement acceptable.

**7.** Procédé de préparation selon la revendication 1 de composés pour lesquels $R_1$ et $R_2$ représentent chacun un atome d'hydrogène de formule (I/B) :

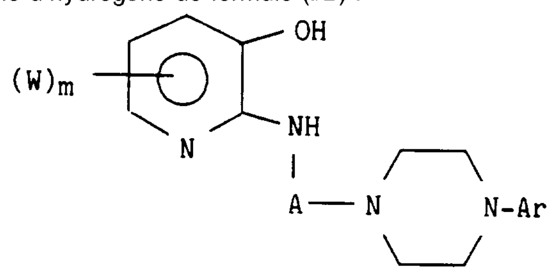

**(I/B)**

leurs isomères ainsi que leurs sels d'addition à un acide pharmaceutiquement acceptable ou à une

base pharmaceutiquement acceptable.

**8.** Procédé de préparation selon la revendication 1, de composés pour lesquels Ar représente un groupement phényle éventuellement substitué par un atome d'halogène ou par un groupement alkyle inférieur, hydroxy, hydroxysulfonyloxy, ou alkoxy inférieur, eux-mêmes éventuellement substitués par un ou plusieurs atomes d'halogène ainsi que leurs sels d'addition à un acide pharmaceutiquement acceptable ou lorsque $R_1$ et $R_2$ représentent un atome d'hydrogène, leurs isomères et leurs sels d'addition à une base pharmaceutiquement acceptable.

**9.** Procédé de préparation selon la revendication 1 du composé qui est la [(Trifluorométhyl - 3 phényl) - 4 pipérazinyl - 1] méthyl - 3 [3H] oxazolo [4,5b] pyridinone - 2 ainsi que ses sels d'addition à un acide pharmaceutiquement acceptable.

**10.** Procédé de préparation selon la revendication 1 du composé qui est la (Phényl - 4 pipérazinyl - 1) méthyl - 3 [3H] oxazolo [4,5b] pyridinone - 2 ainsi que ses sels d'addition à un acide pharmaceutiquement acceptable.

**11.** Procédé de préparation selon la revendication 1 du composé qui est la {[(Trifluorométhyl - 3 phényl) - 4 pipérazinyl - 1] - 2 éthyl} - 3 [3H] oxazolo [4,5b] pyridinone - 2 ainsi que ses sels d'addition à un acide pharmaceutiquement acceptable.

**12.** Procédé de préparation selon la revendication 1 du composé qui est la {[(Trifluorométhyl - 3 phényl) - 4 pipérazinyl - 1] - 3 propyl} - 3 [3H] oxazolo [4,5b] pyridinone - 2 ainsi que ses sels d'addition à un acide pharmaceutiquement acceptable.

**13.** Procédé de préparation selon la revendication 1 du composé qui est la [(Phényl - 4 pipérazinyl - 1) - 2 éthyl] - 3 [3H] oxazolo [4,5b] pyridinone - 2 ainsi que ses sels d'addition à un acide pharmaceutiquement acceptable.

**14.** Procédé de préparation selon la revendication 1 du composé qui est la {[(Pyridyl - 2) - 4 pipérazinyl - 1} - 2 éthyl] - 3 [3H] oxazolo [4,5b] pyridinone - 2 ainsi que ses sels d'addition à un acide pharmaceutiquement acceptable.

**15.** Procédé de préparation selon la revendications 1 du composé qui est la {[(Pyrimidinyl - 2) - 4 pipérazinyl - 1} - 2 éthyl] - 3 [3H] oxazolo [4,5b] pyridinone - 2 ainsi que ses sels d'addition à un acide pharmaceutiquement acceptable.

**16.** Procédé de préparation selon la des revendication 1 du composé qui est le [(Phényl - 4 pipérazinyl - 1) - 2 éthylamino] - 2 pyridinol - 3 ainsi que ses sels d'addition à un acide ou une base pharmaceutiquement acceptable.

**Revendications pour l'Etat contractant suivant : ES**

**1.** Procédé de préparation de composés de formule générale (I) :

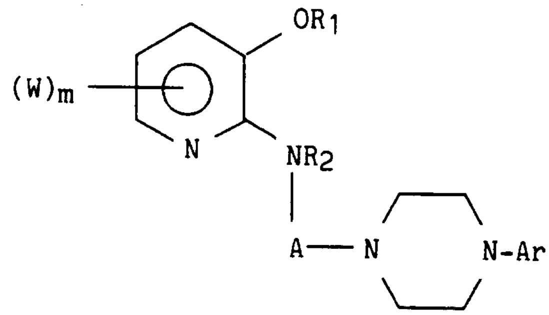

(I)

dans laquelle :

$R_1$ et $R_2$ représentent chacun un atome d'hydrogène ou forment avec l'oxygène et l'azote qui

les portent un chaînon-O-CO-N,

W représente un atome d'halogène ou un groupement alkyle ou alkoxy inférieur éventuellement substitué par un ou plusieurs atomes d'halogène tel que trifluorométhyle, et m étant compris entre 0 et 3,

A représente un goupement alcoyle inférieur linéaire ou ramifié,

Ar représente un groupement aryle ou hétéroaryle éventuellement substitué par un ou plusieurs atomes d'halogène ou par un ou plusieurs groupements alkyle inférieur, hydroxy, hydroxysulfonyloxy, ou alkoxy inférieur ou aryloxy éventuellement substitués par un ou plusieurs atomes d'halogène tel que le groupement trifluorométhyle,

étant entendu que par radical alkyle inférieur ou alkyloxy inférieur, on entend un groupement alkyle linéaire ou ramifié comprenant de 1 à 6 atomes de carbone,

et que par groupements aryle ou hétéroaryle, on entend des groupements mono ou bicycliques insaturés comprenant de 5 à 12 atomes intégrant ou non dans leur squelette carbone, un, deux ou trois hétéroatomes choisis parmi azote, oxygène ou soufre,

leurs isomères,

ainsi que leurs sels d'addition à un acide pharmaceutiquement acceptable et lorsque $R_1$ et $R_2$ représentent un atome d'hydrogène, leurs sels d'addition à une base pharmaceutiquement acceptable,

caractérisé en ce que l'on fait réagir un dérivé de formule (II) :

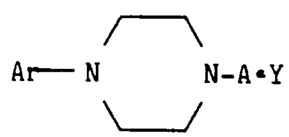

(II)

dans laquelle A et Ar ont la même signification que dans la formule (I), et Y représente un atome d'halogène,

après dissolution dans un solvant organique avec un dérivé de formule (III) :

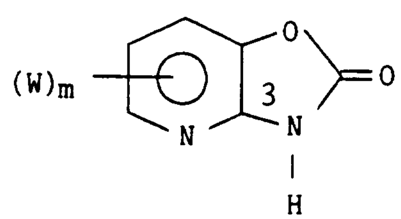

(III)

dans laquelle W et m ont la même signification que dans la formule (I),

préférentiellement sous forme de son dérivé sodé en 3 d'addition en 3 à un métal alcalin

pour conduire après chauffage du milieu réactionnel, refroidissement, filtration, évaporation du milieu réactionnel, reprise par l'eau, extraction par un solvant organique et éventuelle purification par chromatographie sur colonne de silice,

à un dérivé de formule (I/A) :

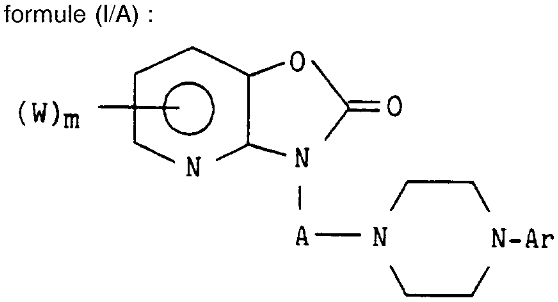

(I/A)

cas particulier des dérivés de formule (I) pour lesquels $R_1$ et $R_2$ forment avec l'azote et l'oxygène qui les portent un chaînon O-CO-A-,

que l'on peut si nécessaire, séparer en ses isomères et, si on le désire, salifier par un acide pharmaceutiquement acceptable,

dérivé de formule (I/A) que l'on peut traiter, si on le désire, par une solution aqueuse d'un agent alcalin,

à une température comprise entre la température ambiante et la température d'ébullition du milieu réactionnel pour conduire, après neutralisation ou acidification du milieu réactionnel et éventuelle purification, à un dérivé de formule (I/B) :

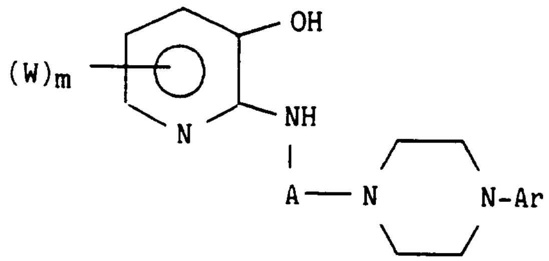

(I/B)

cas particulier des dérivés de formule (I) pour lesquels $R_1$ et $R_2$ représentent chacun un atome d'hydrogène,
que l'on peut si nécessaire séparer en ses isomères et salifier, si on le désire, par un acide ou une base pharmaceutiquement acceptable.

**2.** Procédé de préparation de composés de formule (I/A) selon la revendication 1 dans laquelle A représente un groupement $CH_2$, caractérisé en ce que l'on dissout en milieu d'alcool aliphatique inférieur,
d'une part un dérivé de formule (III) :

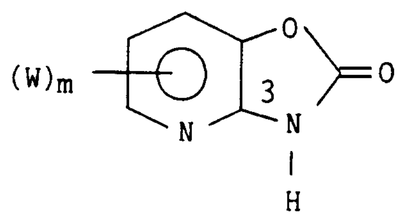

(III)

dans laquelle W et m ont la même signification que dans la formule (I),
d'autre part un léger excès d'une aryl pipérazine de formule (VII) :

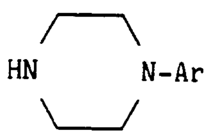

(VII)

dans laquelle Ar a la même signification que dans la formule (I),
et d'autre part un excès de formol,
pour conduire, après chauffage de la solution obtenue à une température comprise entre la température ambiante et la température d'ébullition, éventuel refroidissement, repos d'une à deux heures, filtration et éventuelle chromatographie sur colonne de silice à un dérivé de formule (I/A1) :

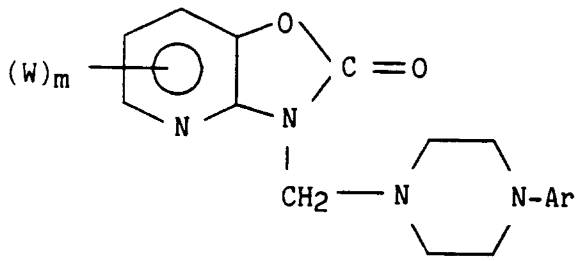

(I/A1)

dans laquelle W, m et Ar ont la même signification que dans la formule (I),
que l'on peut salifier si on le désire par un acide pharmaceutiquement acceptable.

3. Procédé de préparation des composés de formule (I/A) selon la revendication 1 caractérisé en ce que l'on soumet à réaction un dérivé de formule (III) :

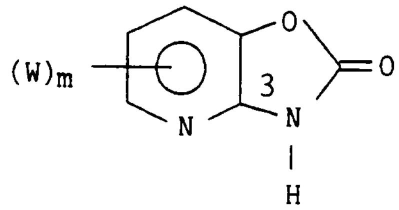

(III)

dans laquelle W et m ont la même signification que dans la formule (I),
avec l'hydroxyde de métal alcalin en milieu aqueux ou un alcoolate de métal alcalin en milieu organique, pour conduire à un dérivé de formule (IV) :

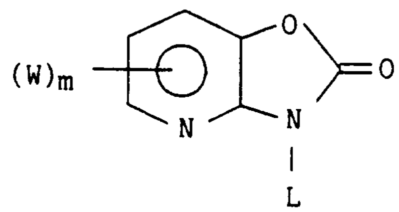

(IV)

dans laquelle W et m ont la même signification que précédemment, et L représente un métal alcalin
que l'on condense avec un dérivé de formule (V) :

**X - A - X' (V)**

dans laquelle A a la même signification que dans la formule (I), X et X' identiques ou différents représentent un atome d'halogène,
préférentiellement sous atmosphère inerte, en milieu organique, préférentiellement à température du reflux du solvant choisi pour conduire, après éventuelle extraction et purification par chromatographie à un dérivé de formule (VI) :

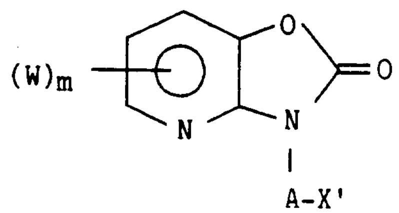

(VI)

dans laquelle W, X', m et A ont la même signification que précédemment,
que l'on condense, préférentiellement sous atmosphère inerte avec un dérivé de formule (VII), préférentiellement en excès :

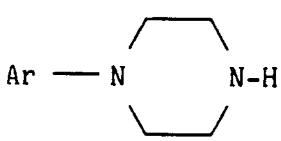

(VII)

dans laquelle Ar a la même définition que dans la formule (I),
en milieu organique en présence d'un excès d'une amine tertiaire et à température du reflux du solvant choisi pour conduire après refroidissement, extraction et éventuelle purification par cristallisation à un dérivé de formule (I/A),
que l'on peut, si nécessaire, séparer en ses isomères et si on le désire, salifier par un acide pharmaceutiquement acceptable.

4. Procédé selon la revendication 3 de préparation de composés de formule (I/A) dans laquelle A représente un chaînon alkyle inférieur linéaire caractérisé en ce que l'on soumet à réaction un dérivé de formule (III) :

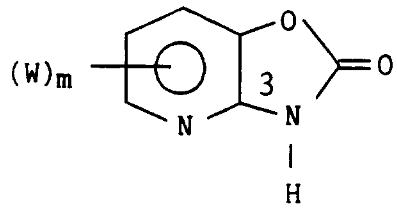

(III)

dans laquelle W et m ont la même signification que dans la formule (I),
avec un hydroxyde de métal alcalin en milieu aqueux ou un alcoolate de métal alcalin en milieu organique, pour conduire à un dérivé de formule (IV) :

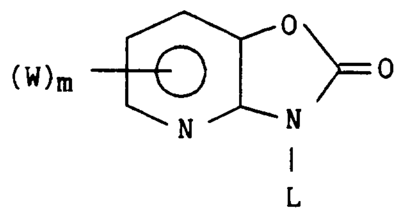

(IV)

dans laquelle W et m ont la même signification que dans la formule (I), et L représente un métal alcalin que l'on condense avec un dérivé de formule (VIII) :

**X - A - X (VIII)**

dans laquelle A a la même signification que dans la formule (I), X représente un atome d'halogène, préférentiellement sous atmosphère inerte, en milieu organique, préférentiellement à température du reflux du solvant choisi pour conduire, après éventuelle extraction et purification par chromatographie à un dérivé de formule (VI) :

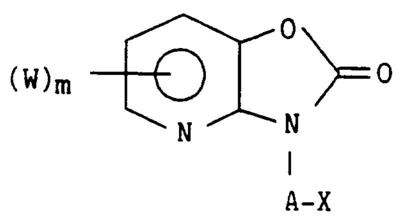

(VI)

dans laquelle W, X, m et A ont la même signification que précédemment,
que l'on condense, préférentiellement sous atmosphère inerte avec un dérivé de formule (VII), préférentiellement en excès :

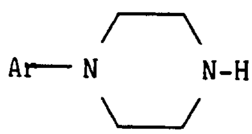

(VII)

dans laquelle Ar a la même définition que précédemment,
en milieu organique en présence d'un excès d'une amine tertiaire et à température du reflux du solvant choisi pour conduire après refroidissement, extraction et éventuelle purification par cristallisation à un dérivé de formule (I/A) pour lequel A représente un chaînon alkyl linéaire,
que l'on peut, si nécessaire, séparer en ses isomères et si on le désire, salifier par un acide pharmaceutiquement acceptable.

5. Procédé de préparation de composé de formule (I/B) selon la revendication 1 caractérisé en ce que l'on soumet un composé de formule (I/A) :

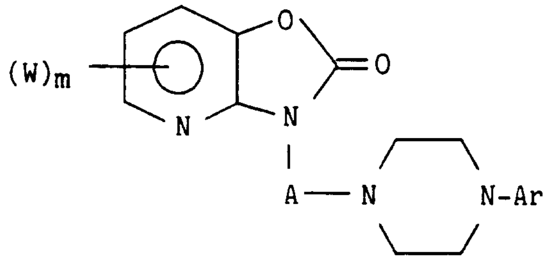

(I/A)

dans laquelle W, m, A, Ar ont la même signification que dans la formule (I), ou l'un de ses sels d'addition à un acide pharmaceutiquement acceptables à un agent alcalin en solution aqueuse à température comprise entre la température ambiante et la température d'ébullition du milieu réactionnel pour conduire, après neutralisation ou acidification du milieu réactionnel à un composé de formule (I/B) :

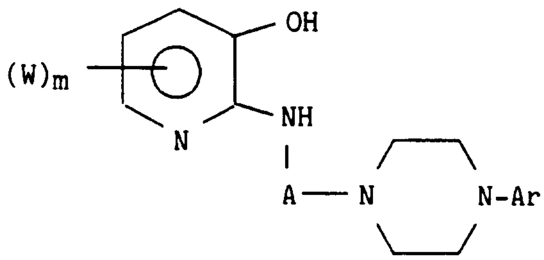

(I/B)

dans laquelle W, m, A, Ar ont la même signification que précédemment,
que l'on salifie, si on le souhaite, par un acide ou une base pharmaceutiquement acceptable après éventuelle purification.

6. Procédé de préparation selon la revendication 1 de composés pour lesquels $R_1$ et $R_2$ forment ensemble un groupement CO, de formule (I/A) :

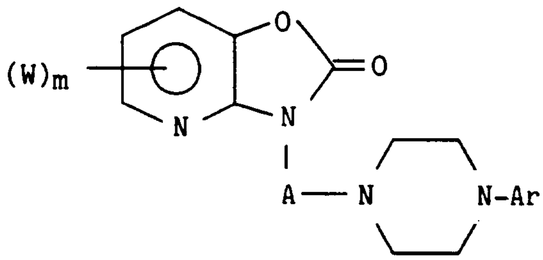

(I/A)

leurs isomères ainsi que leurs sels d'addition à un acide pharmaceutiquement acceptable.

7. Procédé de préparation selon la revendication 1 de composés pour lesquels $R_1$ et $R_2$ représentent chacun un atome d'hydrogène de formule (I/B) :

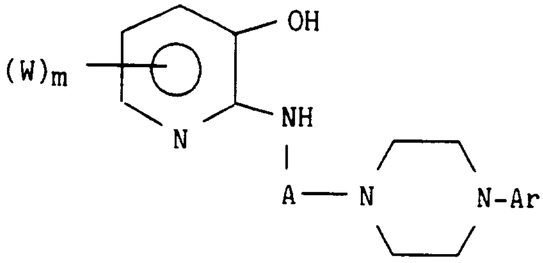

(I/B)

leurs isomères ainsi que leurs sels d'addition à un acide pharmaceutiquement acceptable ou à une

base pharmaceutiquement acceptable.

8. Procédé de préparation selon la revendication 1, de composés pour lesquels Ar représente un groupement phényle éventuellement substitué par un atome d'halogène ou par un groupement alkyle inférieur, hydroxy, hydroxysulfonyloxy, ou alkoxy inférieur, eux-mêmes éventuellement substitués par un ou plusieurs atomes d'halogène ainsi que leurs sels d'addition à un acide pharmaceutiquement acceptable ou lorsque $R_1$ et $R_2$ représentent un atome d'hydrogène, leurs isomères et leurs sels d'addition à une base pharmaceutiquement acceptable.

9. Procédé de préparation selon la revendication 1 du composé qui est la [(Trifluorométhyl - 3 phényl) - 4 pipérazinyl - 1] méthyl - 3 [3H] oxazolo [4,5b] pyridinone - 2 ainsi que ses sels d'addition à un acide pharmaceutiquement acceptable.

10. Procédé de préparation selon la revendication 1 du composé qui est la (Phényl - 4 pipérazinyl - 1) méthyl - 3 [3H] oxazolo [4,5b] pyridinone - 2 ainsi que ses sels d'addition à un acide pharmaceutiquement acceptable.

11. Procédé de préparation selon la revendication 1 du composé qui est la {[(Trifluorométhyl - 3 phényl) - 4 pipérazinyl - 1] - 2 éthyl} - 3 [3H] oxazolo [4,5b] pyridinone - 2 ainsi que ses sels d'addition à un acide pharmaceutiquement acceptable.

12. Procédé de préparation selon la revendication 1 du composé qui est la {[(Trifluorométhyl - 3 phényl) - 4 pipérazinyl - 1] - 3 propyl} - 3 [3H] oxazolo [4,5b] pyridinone - 2 ainsi que ses sels d'addition à un acide pharmaceutiquement acceptable.

13. Procédé de préparation selon la revendication 1 du composé qui est la [(Phényl - 4 pipérazinyl - 1) - 2 éthyl] - 3 [3H] oxazolo [4,5b] pyridinone - 2 ainsi que ses sels d'addition à un acide pharmaceutiquement acceptable.

14. Procédé de préparation selon la revendication 1 du composé qui est la {[(Pyridyl - 2) - 4 pipérazinyl - 1} - 2 éthyl] - 3 [3H] oxazolo [4,5b] pyridinone - 2 ainsi que ses sels d'addition à un acide pharmaceutiquement acceptable.

15. Procédé de préparation selon la revendications 1 du composé qui est la {[(Pyrimidinyl - 2) - 4 pipérazinyl - 1} - 2 éthyl] - 3 [3H] oxazolo [4,5b] pyridinone - 2 ainsi que ses sels d'addition à un acide pharmaceutiquement acceptable.

16. Procédé de préparation selon la des revendication 1 du composé qui est le [(Phényl - 4 pipérazinyl - 1) - 2 éthylamino] - 2 pyridinol - 3 ainsi que ses sels d'addition à un acide ou une base pharmaceutiquement acceptable.

**Claims**

**Claims for the following Contracting States : AT, BE, CH, DE, DK, FR, GB, IT, LI, LU, NL, SE**

1. Compounds of the general formula (I) :

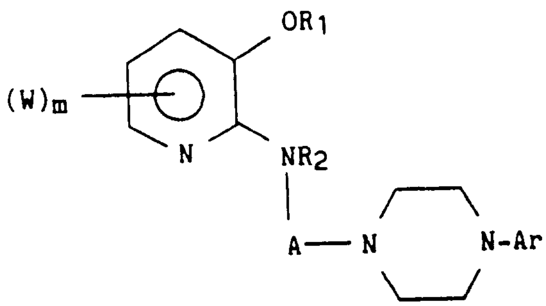

(I)

wherein :

| | |
|---|---|
| $R_1$ and $R_2$ | each represents a hydrogen atom or, together with the oxygen and nitrogen atom which carry them, form an -O-CO-N chain, |
| W | represents a halogen atom or a lower alkyl or lower alkoxy group optionally substituted by one or more halogen atoms, such as trifluoromethyl, m being from 0 to 3, |
| A | represents a straight-chain or branched lower alkyl group, |
| Ar | represents an aryl or heteroaryl group optionally substituted by one or more halogen atoms or by one or more lower alkyl, hydroxy, hydroxysulphonyloxy or lower alkoxy or aryloxy groups optionally substituted by one or more halogen atoms, such as the trifluoromethyl group, |

there being understood by lower alkyl or lower alkoxy radical a straight-chain or branched alkyl group containing from 1 to 6 carbon atoms and by aryl or heteroaryl groups unsaturated mono- or bi-cyclic groups containing from 5 to 12 atoms which may or may not include in their carbon skeleton one, two or three hetero atoms selected from nitrogen, oxygen and sulphur,
the isomers thereof,
and also the addition salts thereof with a pharmaceutically acceptable acid and, when $R_1$ and $R_2$ represent a hydrogen atom, the addition salts thereof with a pharmaceutically acceptable base.

**2.** Compounds according to claim 1, in which $R_1$ and $R_2$ together form a CO group, of formula (I/A) :

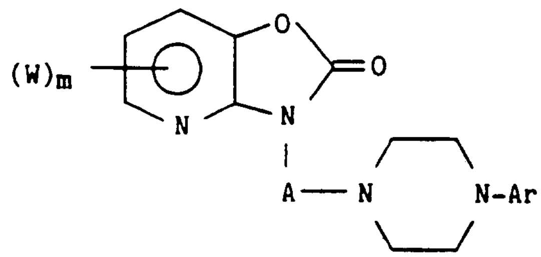

(I/A)

the isomers thereof, and also the addition salts thereof with a pharmaceutically acceptable acid.

**3.** Compounds according to claim 1, in which $R_1$ and $R_2$ each represents a hydrogen atom, of formula (I/B) :

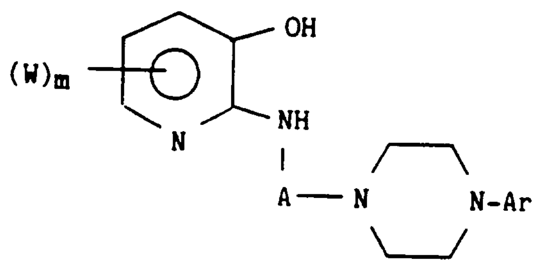

(I/B)

the isomers thereof, and also the addition salts thereof with a pharmaceutically acceptable acid or a pharmaceutically acceptable base.

**4.** Compounds according to claim 1, wherein Ar represents a phenyl group optionally substituted by a halogen atom or by a lower alkyl, hydroxy, hydroxysulphonyloxy or lower alkoxy group, themselves optionally substituted by one or more halogen atoms, and also the addition salts thereof with a pharmaceutically acceptable acid or, when $R_1$ and $R_2$ represent a hydrogen atom, the isomers thereof and the addition salts thereof with a pharmaceutically acceptable base.

**5.** Compound according to either claim 1 or claim 2, which is 3-[4-(3-trifluoromethylphenyl)-1-piperazinyl]-methyl[3H]oxazolo[4,5b]pyridin-2-one, and also the addition salts thereof with a pharmaceutically acceptable acid.

**6.** Compound according to either claim 1 or claim 2, which is 3-(4-phenyl-1-piperazinyl)methyl[3H]oxazolo-[4,5b]pyridin-2-one, and also the addition salts thereof with a pharmaceutically acceptable acid.

7. Compound according to either claim 1 or claim 2, which is 3-{2-[4-(3-trifluoromethylphenyl)-1-piperazinyl]ethyl)[3H]oxazolo[4,5b]pyridin-2-one, and also the addition salts thereof with a pharmaceutically acceptable acid.

8. Compound according to either claim 1 or claim 2, which is 3-{3-[4-(3-trifluoromethylphenyl)-1-piperazinyl]propyl}[3H]oxazolo[4,5b]pyridin-2-one, and also the addition salts thereof with a pharmaceutically acceptable acid.

9. Compound according to either claim 1 or claim 2, which is 3-[2-(4-phenyl-1-piperazinyl)ethyl][3H]-oxazolo[4,5b]pyridin-2-one, and also the addition salts thereof with a pharmaceutically acceptable acid.

10. Compound according to either claim 1 or claim 2, which is 3-{2-[4-(2-pyridyl)-1-piperazinyl]ethyl}[3H]-oxazolo[4,5b]pyridin-2-one, and also the addition salts thereof with a pharmaceutically acceptable acid.

11. Compound according to either claim 1 or claim 2, which is 3-{2-[4-(2-pyrimidinyl)-1-piperazinyl]ethyl}-[3H]oxazolo[4,5b]pyridin-2-one, and also the addition salts thereof with a pharmaceutically acceptable acid.

12. Compound according to any one of claims 1 to 3, which is 2-[2-(4-phenyl-1-piperazinyl)ethylamino]-pyridin-3-ol, and also the addition salts thereof with a pharmaceutically acceptable acid or base.

13. Process for the preparation of compounds of formula (I), characterised in that a compound of formula (II) :

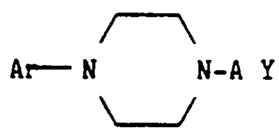

(II)

in which A and Ar are as defined for formula (I) and Y represents a halogen atom, is dissolved in an organic solvent and then reacted with a compound of formula (III) :

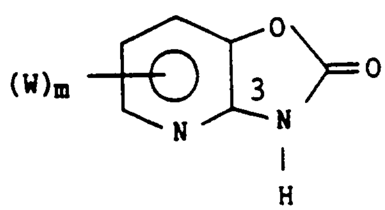

(III)

in which W and m are as defined for formula (I) preferably in the form of its 3-sodium derivative by 3-alkali metal addition
to yield, after the reaction mixture has been heated, cooled and filtered, and the reaction mixture has been evaporated, taken up in water and extracted with an organic solvent and optionally purified by chromatography on a silica column,
a compound of formula (I/A) :

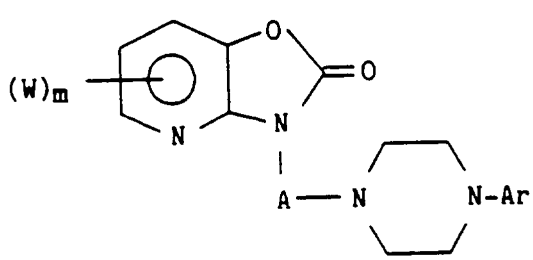

(I/A)

a particular instance of compounds of formula (I) in which $R_1$ and $R_2$, together with the nitrogen and oxygen carrying them, form an O-CO-N- chain,
which can, if necessary, be separated into its isomers and, if desired, converted into a salt with a pharmaceutically acceptable acid,
which compound of formula (I/A) may, if desired, be treated with an aqueous solution of an alkaline agent at a temperature of from room temperature to the boiling temperature of the reaction mixture to yield, after neutralisation or acidification of the reaction mixture and optional purification, a compound of formula (I/B) :

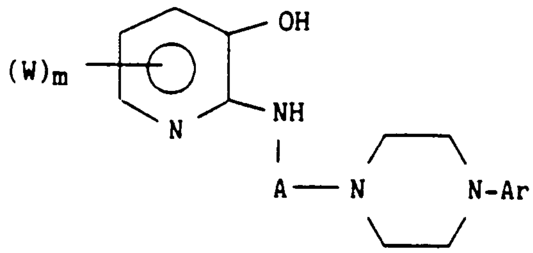

(I/B)

a particular instance of compounds of formula (I) in which $R_1$ and $R_2$ each represents a hydrogen atom, which can, if necessary, be separated into its isomers and, if desired, converted into a salt with a pharmaceutically acceptable acid or base.

**14.** Process for the preparation of compounds of formula (I/A) according to claim 2, in which A represents a $CH_2$ group, characterised in that there are dissolved in a lower aliphatic alcohol medium
a compound of formula (III) :

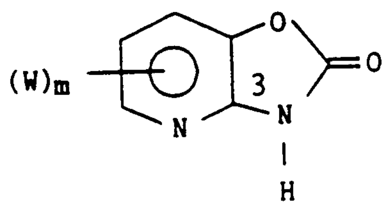

(III)

in which W and m are as defined for formula (I),
a slight excess of an arylpiperazine of formula (VII) :

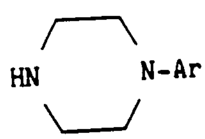

(VII)

in which Ar is as defined for formula (I),
and an excess of formaldehyde solution,
to yield, after heating the resulting solution to a temperature of from room temperature to the boiling temperature, optionally cooling, leaving to stand for from 1 to 2 hours, filtering and optionally chromatographing on a silica column, a compound of formula (I/A1) :

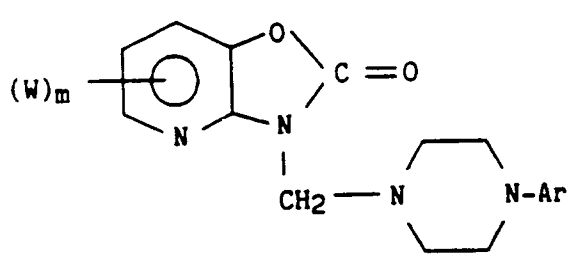

(IA/1)

in which W, m and Ar are as defined for formula (I),
which may, if desired, be converted into a salt with a pharmaceutically acceptable acid.

**15.** Process for the preparation of compounds of formula (I/A) according to claim 2, characterised in that a compound of formula (III) :

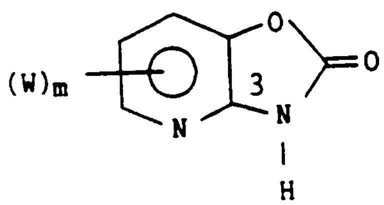

(III)

in which W and m are as defined for formula (I), is reacted with an alkali metal hydroxide in aqueous medium or an alkali metal alcoholate in organic medium, to yield a compound of formula (IV) :

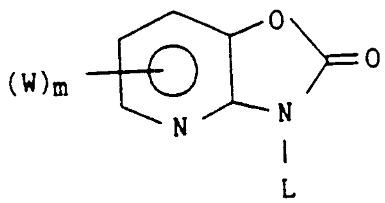

(IV)

in which W and m are as defined above and L represents an alkali metal
which is condensed with a compound of formula (V) :

X - A - X' (V)

in which A is as defined for formula (I) and X and X' are the same or different and each represents a halogen atom,
preferably under an inert atmosphere, in an organic medium, preferably at the reflux temperature of the chosen solvent, to yield, after optional extraction and purification by chromatography, a compound of formula (VI) :

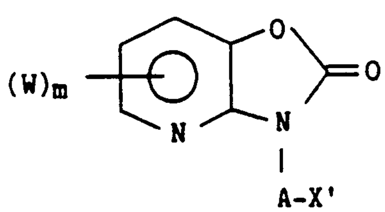

(VI)

in which W, X', m and A are as defined above,
which is condensed, preferably under an inert atmosphere, with a compound of formula (VII), preferably in excess :

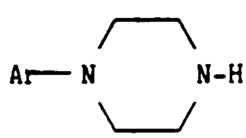

(VII)

in which Ar is as defined for formula (I),
in organic medium, in the presence of an excess of a tertiary amine and at the reflux temperature of the chosen solvent, to yield, after cooling, extraction and optional purification by crystallisation, a compound of formula (I/A),

which can, if necessary, be separated into its isomers and, if desired, be converted into a salt with a pharmaceutically acceptable acid.

**16.** Process according to claim 15 for the preparation of compounds of formula (I/a) in which A represents a straight lower alkyl chain, characterised in that a compound of formula (III) :

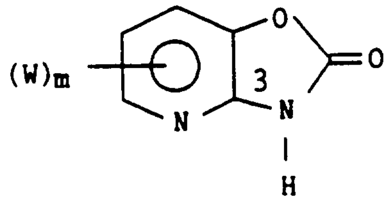

(III)

in which W and m are as defined for formula (I),
is reacted with an alkali metal hydroxide in aqueous medium or an alkali metal alcoholate in organic medium, to yield a compound of formula (IV) :

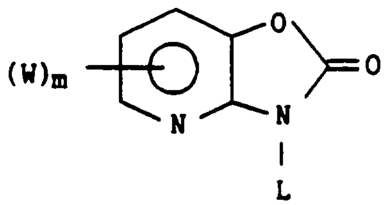

(IV)

in which W and m are as defined for formula (I) and L represents an alkali metal,
which is condensed with a compound of formula (VIII) :

X - A - X (VIII)

in which A is as defined for formula (I) and X represents a halogen atom,
preferably under an inert atmosphere, in organic medium, preferably at the reflux temperature of the chosen solvent to yield, after optional extraction and purification by chromatography, a compound of formula (VI) :

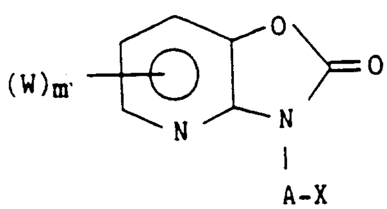

(VI)

in which W, X, m and A are as defined above,
which is condensed, preferably under an inert atmosphere, with a compound of formula (VII), preferably in excess :

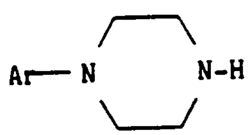

(VII)

in which Ar is as defined above,
in organic medium, in the presence of an excess of a tertiary amine and at the reflux temperature of the chosen solvent, to yield, after cooling, extraction and optional purification by crystallisation, a compound of formula (I/A) in which A represents a straight alkyl chain,
which can, if necessary, be separated into its isomers and, if desired, be converted into a salt with a

pharmaceutically acceptable acid.

**17.** Process for the preparation of a compound of formula (I/B) according to claim III, characterised in that a compound of formula (I/A) :

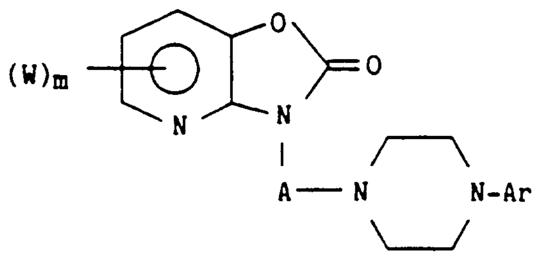

(I/A)

in which W, m, A and Ar are as defined for formula (I), or an addition salt thereof with a pharmaceutically acceptable acid, is subjected to the action of an alkaline agent in aqueous solution at a temperature of from room temperature to the boiling temperature of the reaction mixture to yield, after neutralisation or acidification of the reaction mixture, a compound of formula (I/B) which, if desired, is converted into a salt with a pharmaceutically acceptable acid or base after optional purification.

**18.** Pharmaceutical composition comprising as active ingredient at least one compound according to any one of claims 1 to 12 in combination with one or more pharmaceutically acceptable, inert, non-toxic excipients or carriers.

**19.** Pharmaceutical composition according to claim 18 comprising at least one active ingredient according to any one of claims 1 to 12, which can be used in the treatment of algias.

**Claims for the following Contracting State : GR**

**1.** Process for the preparation of compounds of the general formula (I) :

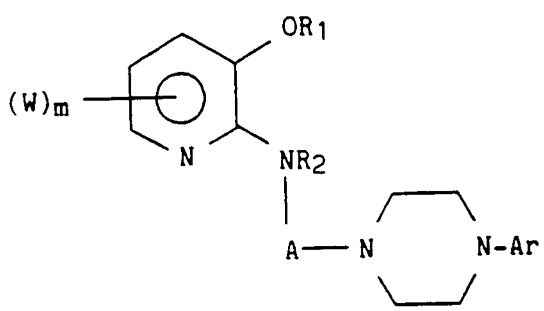

(I)

wherein :

| | |
|---|---|
| $R_1$ and $R_2$ | each represents a hydrogen atom or, together with the oxygen and nitrogen atom which carry them, form an -O-CO-N chain, |
| W | represents a halogen atom or a lower alkyl or lower alkoxy group optionally substituted by one or more halogen atoms, such as trifluoromethyl, m being from 0 to 3, |
| A | represents a straight-chain or branched lower alkyl group, |
| Ar | represents an aryl or heteroaryl group optionally substituted by one or more halogen atoms or by one or more lower alkyl, hydroxy, hydroxysulphonyloxy or lower alkoxy or aryloxy groups optionally substituted by one or more halogen atoms, such as the trifluoromethyl group, |

there being understood by lower alkyl or lower alkoxy radical a straight-chain or branched alkyl group containing from 1 to 6 carbon atoms and by aryl or heteroaryl groups unsaturated mono- or bi-cyclic groups containing from 5 to 12 atoms which may or may not include in their carbon skeleton one, two or three hetero atoms selected from nitrogen, oxygen and sulphur,

the isomers thereof,

and also the addition salts thereof with a pharmaceutically acceptable acid and, when $R_1$ and $R_2$

represent a hydrogen atom, the addition salts thereof with a pharmaceutically acceptable base, characterised in that a compound of formula (II) :

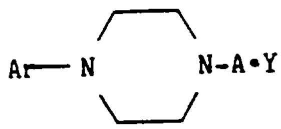

(II)

in which A and Ar are as defined for formula (I) and Y represents a halogen atom, is dissolved in an organic solvent and then reacted with a compound of formula (III) :

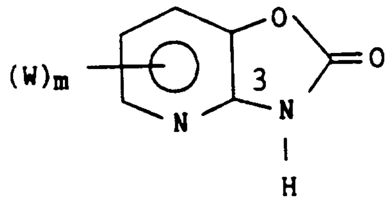

(III)

in which W and m are as defined for formula (I)
preferably in the form of its 3-sodium derivative by 3-alkali metal addition
to yield, after the reaction mixture has been heated, cooled and filtered, and the reaction mixture has been evaporated, taken up in water and extracted with an organic solvent and optionally purified by chromatography on a silica column,
a compound of formula (I/A) :

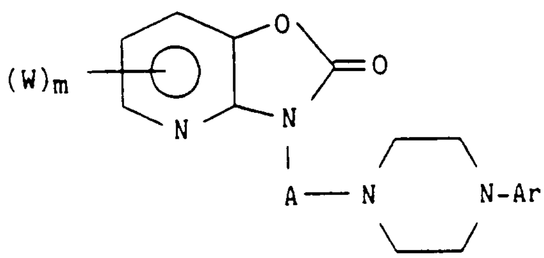

(I/A)

a particular instance of compounds of formula (I) in which $R_1$ and $R_2$, together with the nitrogen and oxygen carrying them, form an O-CO-N- chain,
which can, if necessary, be separated into its isomers and, if desired, converted into a salt with a pharmaceutically acceptable acid,
which compound of formula (I/A) may, if desired, be treated with an aqueous solution of an alkaline agent at a temperature of from room temperature to the boiling temperature of the reaction mixture to yield, after neutralisation or acidification of the reaction mixture and optional purification, a compound of formula (I/B) :

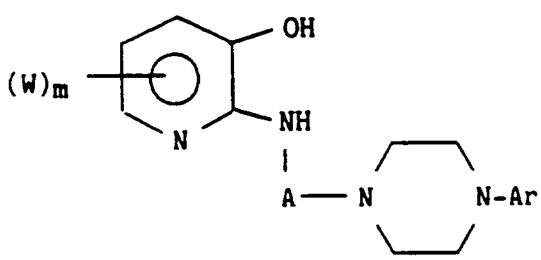

(I/B)

a particular instance of compounds of formula (I) in which $R_1$ and $R_2$ each represents a hydrogen atom, which can, if necessary, be separated into its isomers and, if desired, converted into a salt with a pharmaceutically acceptable acid or base.

2. Process for the preparation of compounds of formula (I/A) according to claim 1 in which A represents a $CH_2$ group, characterised in that there are dissolved in a lower aliphatic alcohol medium
a compound of formula (III) :

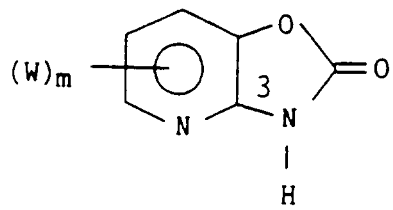

(III)

in which W and m are as defined for formula (I),
a slight excess of an arylpiperazine of formula (VII) :

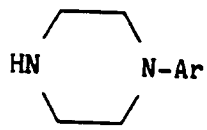

(VII)

in which Ar is as defined for formula (I),
and an excess of formaldehyde solution,
to yield, after heating the resulting solution to a temperature of from room temperature to the boiling temperature, optionally cooling, leaving to stand for from 1 to 2 hours, filtering and optionally chromatographing on a silica column, a compound of formula (I/A1) :

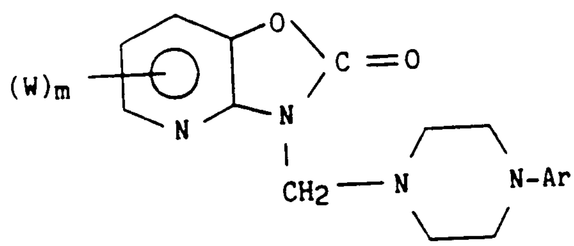

(IA/1)

in which W, m and Ar are as defined for formula (I),
which may, if desired, be converted into a salt with a pharmaceutically acceptable acid.

3. Process for the preparation of compounds of formula (I/A) according to claim 1, characterised in that a compound of formula (III) :

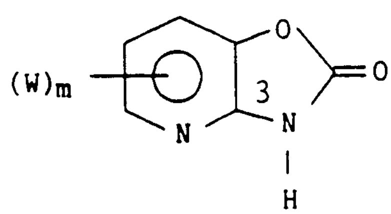

(III)

in which W and m are as defined for formula (I), is reacted with an alkali metal hydroxide in aqueous medium or an alkali metal alcoholate in organic medium, to yield a compound of formula (IV) :

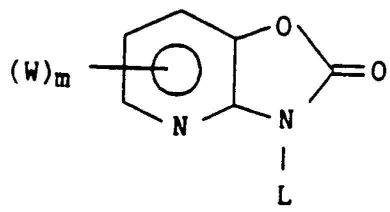

(IV)

in which W and m are as defined above and L represents an alkali metal, which is condensed with a compound of formula (V) :

X - A - X'      (V)

in which A is as defined for formula (I) and X and X' are the same or different and each represents a halogen atom,
preferably under an inert atmosphere, in an organic medium, preferably at the reflux temperature of the chosen solvent, to yield, after optional extraction and purification by chromatography, a compound of formula (VI) :

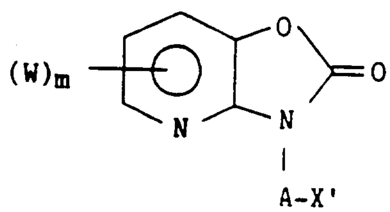

(VI)

in which W, X', m and A are as defined above,
which is condensed, preferably under an inert atmosphere, with a compound of formula (VII), preferably in excess :

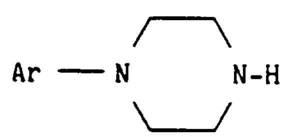

(VII)

in which Ar is as defined for formula (I),
in organic medium, in the presence of an excess of a tertiary amine and at the reflux temperature of the chosen solvent, to yield, after cooling, extraction and optional purification by crystallisation, a compound of formula (I/A),
which can, if necessary, be separated into its isomers and, if desired, be converted into a salt with a pharmaceutically acceptable acid.

**4.** Process according to claim 3 for the preparation of compounds of formula (I/a) in which A represents a straight lower alkyl chain, characterised in that a compound of formula (III) :

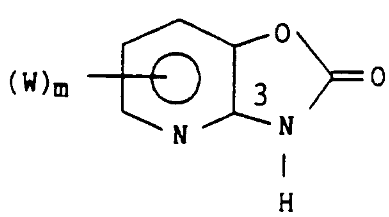

(III)

in which W and m are as defined for formula (I),

is reacted with an alkali metal hydroxide in aqueous medium or an alkali metal alcoholate in organic medium, to yield a compound of formula (IV) :

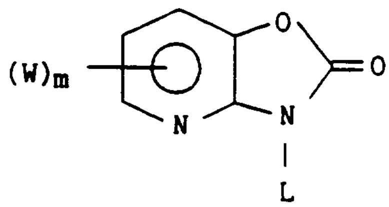

(IV)

in which W and m are as defined for formula (I) and L represents an alkali metal,
which is condensed with a compound of formula (VIII) :

X - A - X (VIII)

in which A is as defined for formula (I) and X represents a halogen atom,
preferably under an inert atmosphere, in organic medium, preferably at the reflux temperature of the chosen solvent to yield, after optional extraction and purification by chromatography, a compound of formula (VI) :

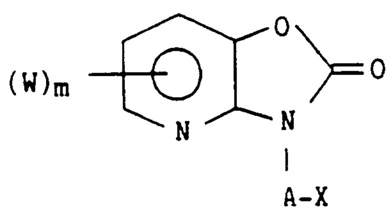

(VI)

in which W, X, m and A are as defined above,
which is condensed, preferably under an inert atmosphere, with a compound of formula (VII), preferably in excess :

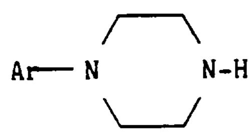

(VII)

in which Ar is as defined above,
in organic medium, in the presence of an excess of a tertiary amine and at the reflux temperature of the chosen solvent, to yield, after cooling, extraction and optional purification by crystallisation, a compound of formula (I/A) in which A represents a straight alkyl chain,
which can, if necessary, be separated into its isomers and, if desired, be converted into a salt with a pharmaceutically acceptable acid.

**5.** Process for the preparation of a compound of formula (I/B) according to claim 1, characterised in that a compound of formula (I/A) :

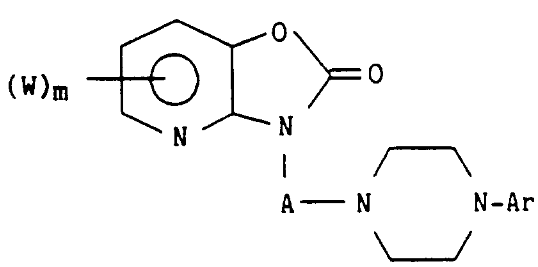

(I/A)

in which W, m, A and Ar are as defined for formula (I), or an addition salt thereof with a pharmaceutically acceptable acid, is subjected to the action of an alkaline agent in aqueous solution at a temperature of from room temperature to the boiling temperature of the reaction mixture to yield, after neutralisation or acidification of the reaction mixture, a compound of formula (I/B) :

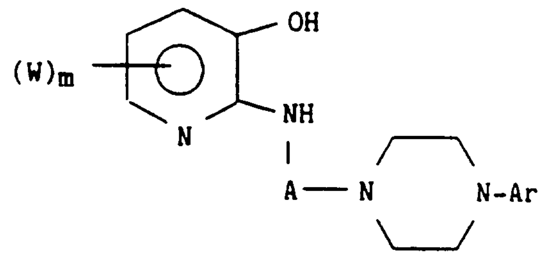

(I/B)

in which W, m, A and Ar are as defined above,
which, if desired, is converted into a salt with a pharmaceutically acceptable acid or base after optional purification.

**6.** Process according to claim 1 for the preparation of compounds in which $R_1$ and $R_2$ together form a CO group, of formula (I/A) :

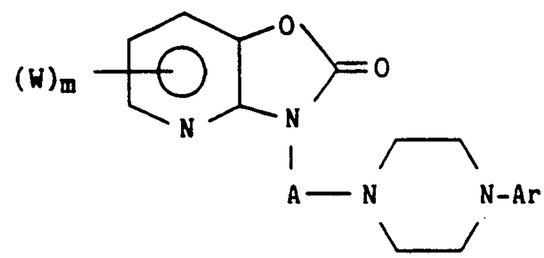

(I/A)

the isomers thereof, and also the addition salts thereof with a pharmaceutically acceptable acid.

**7.** Process according to claim 1 for the preparation of compounds in which $R_1$ and $R_2$ each represents a hydrogen atom, of formula (I/B) :

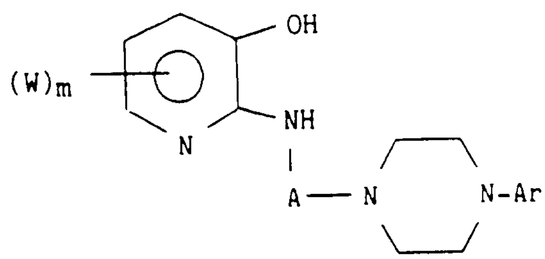

(I/B)

the isomers thereof, and also the addition salts thereof with a pharmaceutically acceptable acid or a pharmaceutically acceptable base.

**8.** Process according to claim 1 for the preparation of compounds wherein Ar represents a phenyl group optionally substituted by a halogen atom or by a lower alkyl, hydroxy, hydroxysulphonyloxy or lower alkoxy group, themselves optionally substituted by one or more halogen atoms, and also the addition salts thereof with a pharmaceutically acceptable acid or, when $R_1$ and $R_2$ represent a hydrogen atom, the isomers thereof and the addition salts thereof with a pharmaceutically acceptable base.

**9.** Process according to claim 1 for the preparation of the compound 3-[4-(3-trifluoromethylphenyl)-1-piperazinyl]methyl[3H]oxazolo[4,5b]pyridin-2-one, and also the addition salts thereof with a pharmaceutically acceptable acid.

**10.** Process according to claim 1 for the preparation of the compound 3-(4-phenyl-1-piperazinyl)methyl[3H]-oxazolo[4,5b]pyridin-2-one, and also the addition salts thereof with a pharmaceutically acceptable acid.

**11.** Process according to claim 1 for the preparation of the compound 3-{2-[4-(3-trifluoromethylphenyl)-1-piperazinyl]ethyl}[3H]oxazolo[4,5b]pyridin-2-one, and also the addition salts thereof with a pharmaceutically acceptable acid.

**12.** Process according to claim 1 for the preparation of the compound 3-{3-[4-(3-trifluoromethylphenyl)-1-piperazinyl]propyl}[3H]oxazolo[4,5b]pyridin-2-one, and also the addition salts thereof with a pharmaceutically acceptable acid.

**13.** Process according to claim 1 for the preparation of the compound 3-[2-(4-phenyl-1-piperazinyl)ethyl]-[3H]oxazolo[4,5b]pyridin-2-one, and also the addition salts thereof with a pharmaceutically acceptable acid.

**14.** Process according to claim 1 for the preparation of the compound 3-{2-[4-(2-pyridyl)-1-piperazinyl]-ethyl}[3H]oxazolo[4,5b]pyridin-2-one, and also the addition salts thereof with a pharmaceutically acceptable acid.

**15.** Process according to claim 1 for the preparation of the compound 3-{2-[4-(2-pyrimidinyl)-1-piperazinyl]-ethyl}[3H]oxazolo[4,5b]pyridin-2-one, and also the addition salts thereof with a pharmaceutically acceptable acid.

**16.** Process according to claim 1 for the preparation of the compound 2-[2-(4-phenyl-1-piperazinyl)-ethylamino]pyridin-3-ol, and also the addition salts thereof with a pharmaceutically acceptable acid or base.

**Claims for the following Contracting State : ES**

**1.** Process for the preparation of compounds of the general formula (I) :

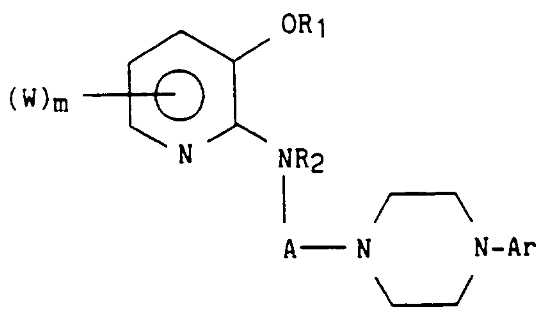

(I)

wherein :

| | |
|---|---|
| $R_1$ and $R_2$ | each represents a hydrogen atom or, together with the oxygen and nitrogen atom which carry them, form an -O-CO-N chain, |
| W | represents a halogen atom or a lower alkyl or lower alkoxy group optionally substituted by one or more halogen atoms, such as trifluoromethyl, m being from 0 to 3, |
| A | represents a straight-chain or branched lower alkyl group, |
| Ar | represents an aryl or heteroaryl group optionally substituted by one or more halogen atoms or by one or more lower alkyl, hydroxy, hydroxysulphonyloxy or lower alkoxy or aryloxy groups optionally substituted by one or more halogen atoms, such as the trifluoromethyl group, |

there being understood by lower alkyl or lower alkoxy radical a straight-chain or branched alkyl group containing from 1 to 6 carbon atoms and by aryl or heteroaryl groups unsaturated mono- or bi-cyclic groups containing from 5 to 12 atoms which may or may not include in their carbon skeleton one, two or three hetero atoms selected from nitrogen, oxygen and sulphur,
the isomers thereof,
and also the addition salts thereof with a pharmaceutically acceptable acid and, when $R_1$ and $R_2$ represent a hydrogen atom, the addition salts thereof with a pharmaceutically acceptable base, characterised in that a compound of formula (II) :

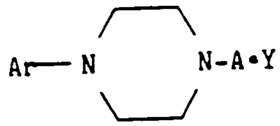

(II)

in which A and Ar are as defined for formula (I) and Y represents a halogen atom, is dissolved in an organic solvent and then reacted with a compound of formula (III) :

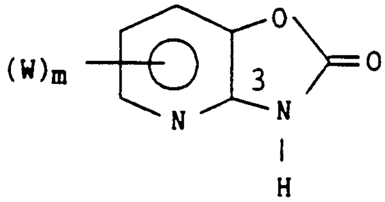

(III)

in which W and m are as defined for formula (I)
preferably in the form of its 3-sodium derivative by 3-alkali metal addition
to yield, after the reaction mixture has been heated, cooled and filtered, and the reaction mixture has been evaporated, taken up in water and extracted with an organic solvent and optionally purified by chromatography on a silica column,
a compound of formula (I/A) :

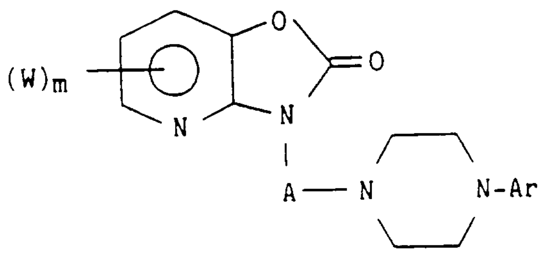

(I/A)

a particular instance of compounds of formula (I) in which $R_1$ and $R_2$, together with the nitrogen and oxygen carrying them, form an O-CO-N- chain,
which can, if necessary, be separated into its isomers and, if desired, converted into a salt with a pharmaceutically acceptable acid,
which compound of formula (I/A) may, if desired, be treated with an aqueous solution of an alkaline agent at a temperature of from room temperature to the boiling temperature of the reaction mixture to yield, after neutralisation or acidification of the reaction mixture and optional purification, a compound of formula (I/B) :

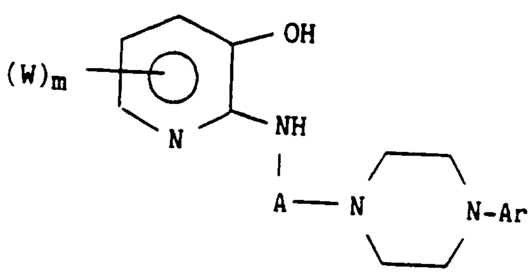

(I/B)

a particular instance of compounds of formula (I) in which $R_1$ and $R_2$ each represents a hydrogen atom, which can, if necessary, be separated into its isomers and, if desired, converted into a salt with a pharmaceutically acceptable acid or base.

2. Process for the preparation of compounds of formula (I/A) according to claim 1 in which A represents a $CH_2$ group, characterised in that there are dissolved in a lower aliphatic alcohol medium
a compound of formula (III) :

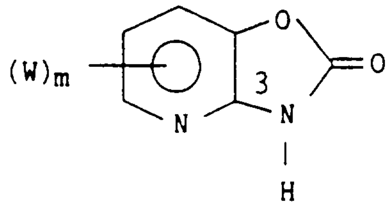

(III)

in which W and m are as defined for formula (I),
a slight excess of an arylpiperazine of formula (VII) :

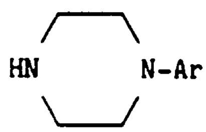

(VII)

in which Ar is as defined for formula (I),
and an excess of formaldehyde solution,
to yield, after heating the resulting solution to a temperature of from room temperature to the boiling temperature, optionally cooling, leaving to stand for from 1 to 2 hours, filtering and optionally chromatographing on a silica column, a compound of formula (I/A1) :

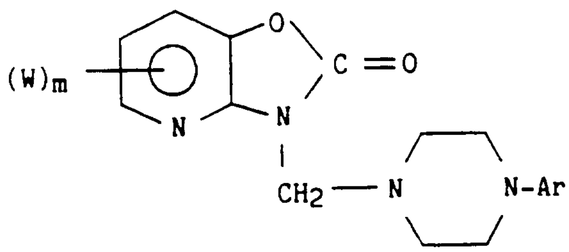

(IA/1)

in which W, m and Ar are as defined for formula (I),
which may, if desired, be converted into a salt with a pharmaceutically acceptable acid.

3. Process for the preparation of compounds of formula (I/A) according to claim 1, characterised in that a compound of formula (III) :

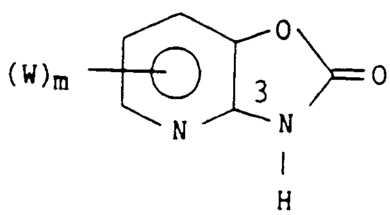

(III)

in which W and m are as defined for formula (I), is reacted with an alkali metal hydroxide in aqueous medium or an alkali metal alcoholate in organic medium, to yield a compound of formula (IV) :

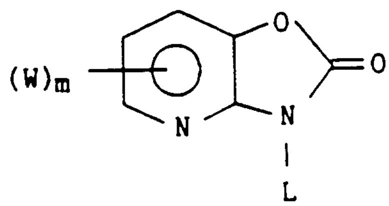

(IV)

in which W and m are as defined above and L represents an alkali metal, which is condensed with a compound of formula (V) :

X - A - X' (V)

in which A is as defined for formula (I) and X and X' are the same or different and each represents a halogen atom,
preferably under an inert atmosphere, in an organic medium, preferably at the reflux temperature of the chosen solvent, to yield, after optional extraction and purification by chromatography, a compound of formula (VI) :

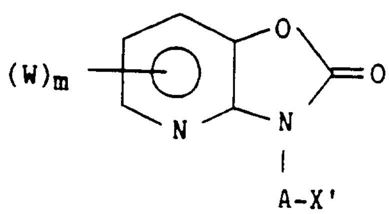

(VI)

in which W, X', m and A are as defined above,
which is condensed, preferably under an inert atmosphere, with a compound of formula (VII), preferably in excess :

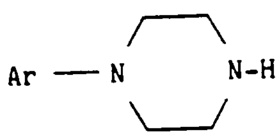

(VII)

in which Ar is as defined for formula (I),
in organic medium, in the presence of an excess of a tertiary amine and at the reflux temperature of the chosen solvent, to yield, after cooling, extraction and optional purification by crystallisation, a compound of formula (I/A),
which can, if necessary, be separated into its isomers and, if desired, be converted into a salt with a pharmaceutically acceptable acid.

4. Process according to claim 3 for the preparation of compounds of formula (I/a) in which A represents a straight lower alkyl chain, characterised in that a compound of formula (III) :

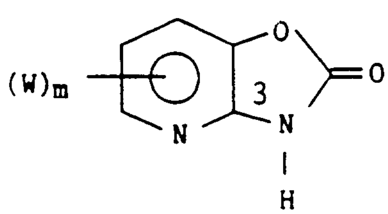

(III)

in which W and m are as defined for formula (I),
is reacted with an alkali metal hydroxide in aqueous medium or an alkali metal alcoholate in organic

medium, to yield a compound of formula (IV) :

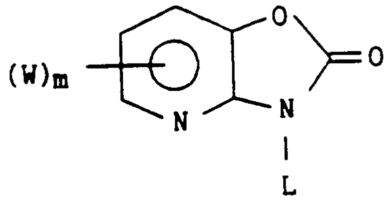

(IV)

in which W and m are as defined for formula (I) and L represents an alkali metal, which is condensed with a compound of formula (VIII) :

X - A - X (VIII)

in which A is as defined for formula (I) and X represents a halogen atom, preferably under an inert atmosphere, in organic medium, preferably at the reflux temperature of the chosen solvent to yield, after optional extraction and purification by chromatography, a compound of formula (VI) :

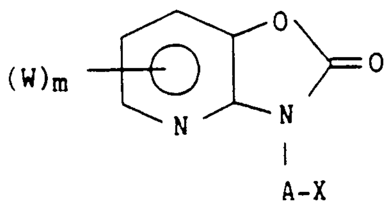

(VI)

in which W, X, m and A are as defined above, which is condensed, preferably under an inert atmosphere, with a compound of formula (VII), preferably in excess :

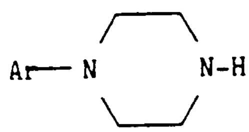

(VII)

in which Ar is as defined above, in organic medium, in the presence of an excess of a tertiary amine and at the reflux temperature of the chosen solvent, to yield, after cooling, extraction and optional purification by crystallisation, a compound of formula (I/A) in which A represents a straight alkyl chain, which can, if necessary, be separated into its isomers and, if desired, be converted into a salt with a pharmaceutically acceptable acid.

**5.** Process for the preparation of a compound of formula (I/B) according to claim 1, characterised in that a compound of formula (I/A) :

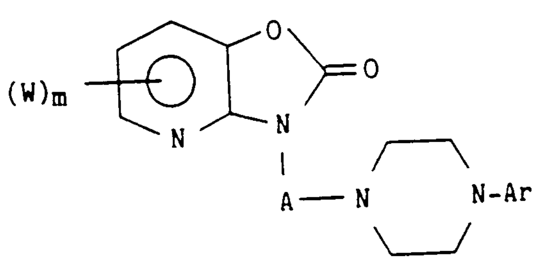

(I/A)

in which W, m, A and Ar are as defined for formula (I), or an addition salt thereof with a pharmaceutically acceptable acid, is subjected to the action of an alkaline agent in aqueous solution at a

temperature of from room temperature to the boiling temperature of the reaction mixture to yield, after neutralisation or acidification of the reaction mixture, a compound of formula (I/B) :

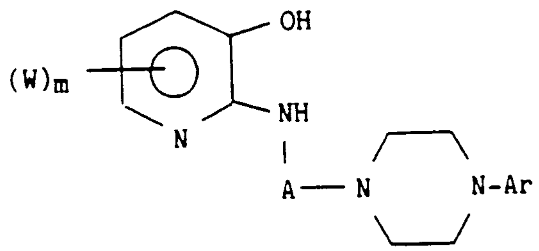

(I/B)

in which W, m, A and Ar are as defined above,
which, if desired, is converted into a salt with a pharmaceutically acceptable acid or base after optional purification.

**6.** Process according to claim 1 for the preparation of compounds in which $R_1$ and $R_2$ together form a CO group, of formula (I/A) :

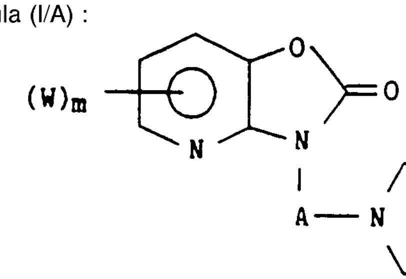

(I/A)

the isomers thereof, and also the addition salts thereof with a pharmaceutically acceptable acid.

**7.** Process according to claim 1 for the preparation of compounds in which $R_1$ and $R_2$ each represents a hydrogen atom, of formula (I/B) :

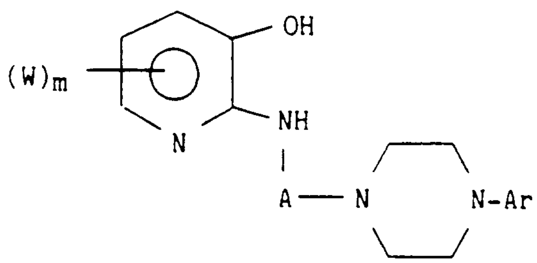

(I/B)

the isomers thereof, and also the addition salts thereof with a pharmaceutically acceptable acid or a pharmaceutically acceptable base.

**8.** Process according to claim 1 for the preparation of compounds wherein Ar represents a phenyl group optionally substituted by a halogen atom or by a lower alkyl, hydroxy, hydroxysulphonyloxy or lower alkoxy group, themselves optionally substituted by one or more halogen atoms, and also the addition salts thereof with a pharmaceutically acceptable acid or, when $R_1$ and $R_2$ represent a hydrogen atom, the isomers thereof and the addition salts thereof with a pharmaceutically acceptable base.

**9.** Process according to claim 1 for the preparation of the compound 3-[4-(3-trifluoromethylphenyl)-1-piperazinyl]methyl[3H]oxazolo[4,5b]pyridin-2-one, and also the addition salts thereof with a pharmaceutically acceptable acid.

**10.** Process according to claim 1 for the preparation of the compound 3-(4-phenyl-1-piperazinyl)methyl[3H]-oxazolo[4,5b]pyridin-2-one, and also the addition salts thereof with a pharmaceutically acceptable acid.

11. Process according to claim 1 for the preparation of the compound 3-{2-[4-(3-trifluoromethylphenyl)-1-piperazinyl]ethyl}[3H]oxazolo[4,5b]pyridin-2-one, and also the addition salts thereof with a pharmaceutically acceptable acid.

12. Process according to claim 1 for the preparation of the compound 3-{3-[4-(3-trifluoromethylphenyl)-1-piperazinyl]propyl}[3H]oxazolo[4,5b]pyridin-2-one, and also the addition salts thereof with a pharmaceutically acceptable acid.

13. Process according to claim 1 for the preparation of the compound 3-[2-(4-phenyl-1-piperazinyl)ethyl]-[3H]oxazolo[4,5b]pyridin-2-one, and also the addition salts thereof with a pharmaceutically acceptable acid.

14. Process according to claim 1 for the preparation of the compound 3-{2-[4-(2-pyridyl)-1-piperazinyl]-ethyl}[3H]oxazolo[4₁5b]pyridin-2-one, and also the addition salts thereof with a pharmaceutically acceptable acid.

15. Process according to claim 1 for the preparation of the compound 3-{2-[4-(2-pyrimidinyl)-1-piperazinyl]-ethyl}[3H]oxazolo[4,5b]pyridin-2-one, and also the addition salts thereof with a pharmaceutically acceptable acid.

16. Process according to claim 1 for the preparation of the compound 2-[2-(4-phenyl-1-piperazinyl)-ethylamino]pyridin-3-ol, and also the addition salts thereof with a pharmaceutically acceptable acid or base.

## Patentansprüche

## Patentansprüche für folgende Vertragsstaaten : AT, BE, CH, DE, DK, FR, GB, IT, LI, LU, NL, SE

1. Verbindung der allgemeinen Formel (I):

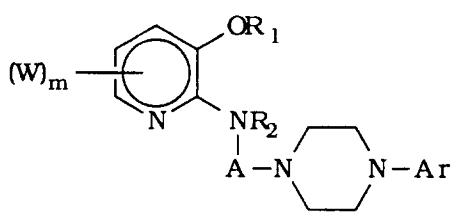

(I)

in der:

| | |
|---|---|
| $R_1$ und $R_2$ | jeweils ein Wasserstoffatom oder gemeinsam mit dem Sauerstoff und dem Stickstoff, die sie tragen, eine -O-CO-N-Kette, |
| W | ein Halogenatom oder eine Niedrigalkyl- oder Niedrigalkoxy-gruppe, die gegebenenfalls durch ein oder mehrere Halogenatome substituiert sein kann, wie eine Trifluormethylgruppe, und m zwischen 0 und 3 liegt, |
| A | eine geradkettige oder verzweigte Niedrigalkylgruppe und |
| Ar | eine Aryl- oder Heteroaryl-gruppe, die gegebenenfalls durch ein oder mehrere Halogenatome oder durch eine oder mehrere Niedrigalkylgruppen, Hydroxylgruppen, Hydroxysulfonyloxygruppen oder Niedrigalkoxy- oder Aryloxy-gruppen, die gegebenenfalls durch ein oder mehrere Halogenatome substituiert sein können, wie die Trifluormethylgruppe, substituiert sein kann, bedeuten, |

wobei unter einer Niedrigalkylgruppe oder Niedrigalkoxygruppe eine geradkettige oder verzweigte Alkylgruppe mit 1 bis 6 Kohlenstoffatomen zu verstehen ist und unter Aryl- oder Heteroaryl-gruppen mono- oder bicyclische ungesättigte Gruppen zu verstehen sind, die 5 bis 12 Atome aufweisen, die in ihrem Kohlenstoffskelett gegebenenfalls ein, zwei oder drei Heteroatome ausgewählt aus Stickstoff, Sauerstoff oder Schwefel aufweisen,

deren Isomeren,

sowie deren Additionssalze mit einer pharmazeutisch annehmbaren Säure und, wenn $R_1$ und $R_2$ ein Wasserstoffatom bedeuten, deren Additionssalze mit einer pharmazeutisch annehmbaren Base.

2. Verbindungen nach Anspruch 1, worin $R_1$ und $R_2$ gemeinsam eine Gruppe CO bilden, der Formel (I/A):

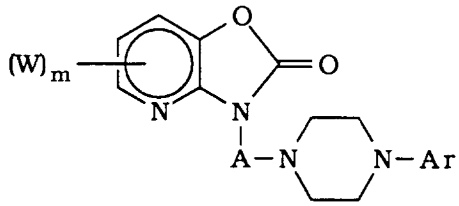

(I/A)

deren Isomere sowie deren Additionssalze mit einer pharmazeutisch annehmbaren Säure.

3. Verbindungen nach Anspruch 1, worin $R_1$ und $R_2$ jeweils ein Wasserstoffatom bedeuten, der Formel (I/B):

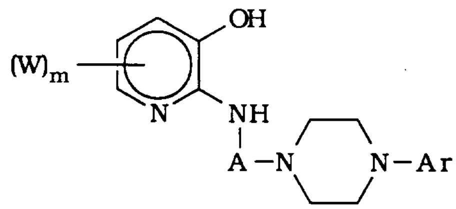

(I/B)

deren Isomere sowie deren Additionssalze mit einer pharmazeutisch annehmbaren Säure oder einer pharmazeutisch annehmbaren Base.

4. Verbindungen nach Anspruch 1, worin Ar eine Phenylgruppe bedeutet, die gegebenenfalls durch ein Halogenatom oder eine Niedrigalkylgruppe, Hydroxylgruppe, Hydroxysulfonyloxygruppe oder Niedrigalkoxygruppe substituiert ist, die ihrerseits gegebenenfalls durch ein oder mehrere Halogenatome substituiert sind, sowie deren Additionssalze mit einer pharmazeutisch annehmbaren Säure oder, wenn $R_1$ und $R_2$ ein Wasserstoffatom bedeuten, deren Isomeren und deren Additionssalze mit einer pharmazeutisch annehmbaren Base.

5. Verbindung nach einem der Ansprüche 1 und 2, nämlich [4-(3-Trifluormethyl-phenyl)-piperazin-1-yl]-3-methyl-[3H]-oxazolo[4,5b]pyridin-2-on sowie dessen Additionssalze mit einer pharmazeutisch annehmbaren Säure.

6. Verbindung nach einem der Ansprüche 1 und 2, nämlich (4-Phenyl)-piperazin-1-yl)-3-methyl-[3H]-oxazolo[4,5b]pyridin-2-on sowie dessen Additionssalze mit einer pharmazeutisch annehmbaren Säure.

7. Verbindung nach einem der Ansprüche 1 und 2, nämlich 3-{2-[4-(3-Trifluormethyl-phenyl)-piperazin-1-yl]-ethyl}-[3H]-oxazolo[4,5b]pyridin-2-on sowie dessen Additionssalze mit einer pharmazeutisch annehmbaren Säure.

8. Verbindung nach einem der Ansprüche 1 und 2, nämlich 3-{3-[4-(3-Trifluormethyl-phenyl)-piperazin-1-yl]-propyl}-[3H]-oxazolo[4,5b]pyridin-2-on sowie dessen Additionssalze mit einer pharmazeutisch annehmbaren Säure.

9. Verbindung nach einem der Ansprüche 1 und 2, nämlich 3-[2-(4-Phenyl-piperazin-1-yl)-ethyl]-[3H]-oxazolo[4,5b]pyridin-2-on sowie dessen Additionssalze mit einer pharmazeutisch annehmbaren Säure.

10. Verbindung nach einem der Ansprüche 1 und 2, nämlich 3-{2-[4-(Pyrid-2-yl)-piperazin-1-yl]-ethyl}-[3H]-oxazolo[4,5b]pyridin-2-on sowie dessen Additionssalze mit einer pharmazeutisch annehmbaren Säure.

11. Verbindung nach einem der Ansprüche 1 und 2, nämlich 3-{2-[4-(Pyrimidin-2-yl)-piperazin-1-yl]-ethyl}-[3H]-oxazolo[4,5b]pyridin-2-on sowie dessen Additionssalze mit einer pharmazeutisch annehmbaren Säure.

**12.** Verbindung nach einem der Ansprüche 1 und 3, nämlich 2-[2-(4-Phenyl-piperazin-1-yl]-ethylamino]-pyridin-3-ol sowie dessen Additionssalze mit einer pharmazeutisch annehmbaren Säure oder Base.

**13.** Verfahren zur Herstellung der Verbindungen der Formel (I), **dadurch gekennzeichnet**, daß man ein Derivat der Formel (II):

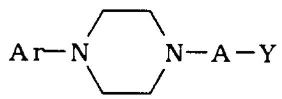

(II)

in der A und Ar die bezüglich der Formel (I) angegebenen Bedeutungen besitzen und Y ein Halogenatom darstellt,
nach dem Auflösen in einem organischen Lösungsmittel mit einem Derivat der Formel (III):

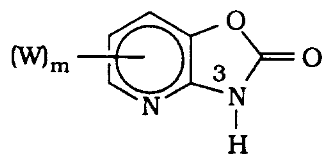

(III)

in der W und m die bezüglich der Formel (I) angegebenen Bedeutungen besitzen, vorzugsweise in Form seines Natriumderivats in der 3-Stellung durch Addition eines Alkalimetalls in der 3-Stellung, umsetzt,
so daß man nach dem Erhitzen des Reaktionsmediums, Abkühlen, Filtrieren, Verdampfen des Reaktionsmediums, Wiederaufnehmen mit Wasser, Extraktion mit einem organischen Lösungsmittel und eventuelle Reinigung durch Chromatographie über eine mit Siliciumdioxid beschickte Säule
ein Derivat der Formel (I/A) erhält:

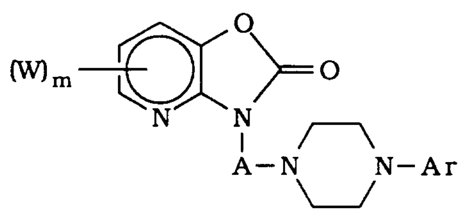

(I/A)

einem Sonderfall der Derivate der Formel (I), worin $R_1$ und $R_2$ mit dem Stickstoff und dem Sauerstoff, die sie tragen, eine O-CO-A-Kette bilden,
das man erforderlichenfalls in seine Isomeren auftrennen und gewünschtenfalls mit einer pharmazeutisch annehmbaren Säure in ein Salz überführen kann,
welches Derivat der Formel (I/A) man gewünschtenfalls mit einer wäßrigen Lösung eines alkalischen Mittels bei einer Temperatur zwischen Raumtemperatur und der Siedetemperatur des Reaktionsmediums behandeln kann, so daß man nach der Neutralisation oder dem Sauerstellen des Reaktionsmediums und der eventuellen Reinigung ein Derivat der Formel (I/B) erhält:

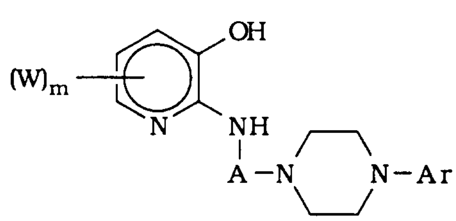

(I/B)

einem Sonderfall der Derivate der Formel (I), worin $R_1$ und $R_2$ jeweils ein Wasserstoffatom bedeuten,
das man gewünschtenfalls in seine Isomeren auftrennen und gewünschtenfalls mit einer pharmazeutisch annehmbaren Säure oder Base in ein Salz überführen kann.

**14.** Verfahren zur Herstellung der Verbindungen der Formel (I/A) nach Anspruch 2, worin A eine $CH_2$-Gruppe bedeutet, **dadurch gekennzeichnet**, daß man
einerseits ein Derivat der Formel (III):

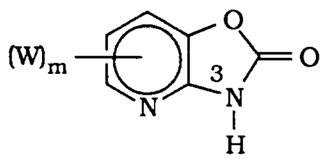 (III)

in der W und m die bezüglich der Formel (I) angegebenen Bedeutungen besitzen, andererseits einen schwachen Überschuß eines Arylpiperazins der Formel (VII):

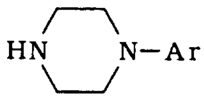 (VII)

in der Ar die bezüglich der Formel (I) angegebenen Bedeutungen besitzt,
und andererseits einen Überschuß Formaldehyd in einem niedrigaliphatischen alkoholischen Medium löst,
so daß man nach dem Erhitzen der erhaltenen Lösung auf eine Temperatur zwischen Raumtemperatur und der Siedetemperatur, dem eventuellen Abkühlen, dem Stehenlassen während einer bis zwei Stunden, dem Filtrieren und der eventuellen Säulenchromatographie über Siliciumdioxid ein Derivat der Formel (I/A1) erhält:

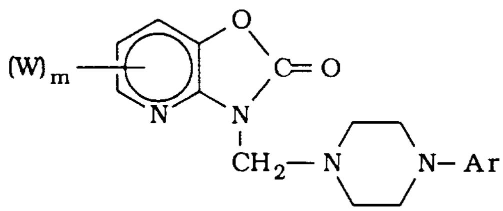 (I/A1)

in der W, m und Ar die bezüglich der Formel (I) angegebenen Bedeutungen besitzen,
das man gegebenenfalls mit einer pharmazeutisch annehmbaren Säure in ein Salz überführen kann.

**15.** Verfahren zur Herstellung von Verbindungen der Formel (I/A) nach Anspruch 2, **dadurch gekennzeichnet**, daß man ein Derivat der Formel (III):

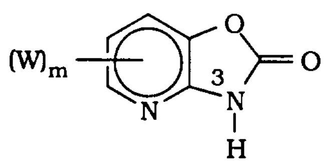 (III)

in der W und m die bezüglich der Formel (I) angegebenen Bedeutungen besitzen, der Reaktion mit einem Alkalimetallhydroxid in wäßrigem Medium oder einem Alkalimetallalkoholat in organischem Medium unterwirft zur Bildung eines Derivats der Formel (IV):

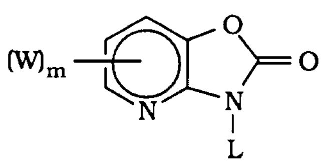 (IV)

in der W und m die oben angegebenen Bedeutungen besitzen und L ein Alkalimetall darstellt,
welches man mit einem Derivat der Formel (V):

X - A - X' (V)

in der A die bezüglich der Formel (I) angegebenen Bedeutungen besitzt und X und X', die gleichartig oder verschieden sind, ein Halogenatom darstellen,
vorzugsweise unter einer inerten Atmosphäre in organischem Medium, vorzugsweise bei der Rückflußtemperatur des ausgewählten Losungsmittels kondensiert, so daß man nach der eventuellen Extraktion und Reinigung durch Chromatographie ein Derivat der Formel (VI) erhält:

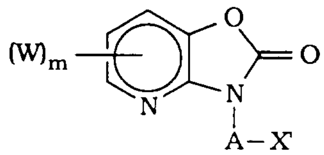 (VI)

in der W, X', m und A die oben angegebenen Bedeutungen besitzen,
welches man vorzugsweise unter einer inerten Atmosphäre mit einem Derivat der Formel (VII):

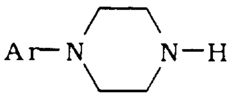 (VII)

in der Ar die bezüglich der Formel (I) angegebenen Bedeutungen besitzt,
das vorzugsweise im Überschuß eingesetzt wird, in organischem Medium und in Gegenwart eines Überschusses eines tertiären Amins und bei der Rückflußtemperatur des ausgewählten Lösungsmittels kondensiert, so daß man nach dem Abkühlen, der Extraktion und der eventuellen Reinigung durch Kristallisation ein Derivat der Formel (I/A) erhält,
welches man erforderlichenfalls in die Isomeren auftrennen und gewünschtenfalls mit einer pharmazeutisch annehmbaren Säure in ein Salz überführen kann.

**16.** Verfahren nach Anspruch 15 zur Herstellung von Verbindungen der Formel (I/A), in der A eine lineare Niedrigalkylkette bedeutet, **dadurch gekennzeichnet**, daß man ein Derivat der Formel (III):

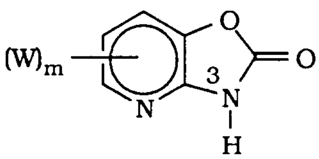 (III)

in der W und m die bezüglich der Formel (I) angegebenen Bedeutungen besitzen, einer Reaktion mit einem Alkalimetallhydroxid in wäßrigem Medium oder einem Alkalimetallalkoholat in organischem Medium unterwirft zur Bildung eines Derivats der Formel (IV):

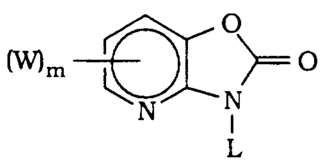 (IV)

in der W und m die bezüglich der Formel (I) angegebenen Bedeutungen besitzen und L ein Alkalimetall darstellt,
welches man mit einem Derivat der Formel (VIII):

X - A - X (VIII)

in der A die bezüglich der Formel (I) angegebenen Bedeutungen besitzt und X ein Halogenatom darstellt,

vorzugsweise unter einer inerten Atmosphäre in organischem Medium, vorzugsweise bei der Rückflußtemperatur des ausgewählten Lösungsmittels, kondensiert, so daß man nach der eventuellen Extraktion und Reinigung durch Chromatographie ein Derivat der Formel (VI) erhält:

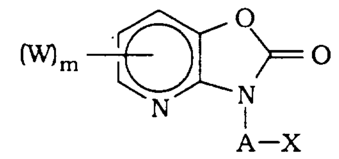

(VI)

in der W, X, m und A die oben angegebenen Bedeutungen besitzen,
welches man vorzugsweise unter inerter Atmosphäre mit einem Derivat der Formel (VII):

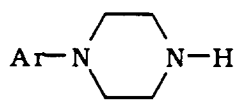

(VII)

in der Ar die oben angegebenen Bedeutungen besitzen, welches vorzugsweise im Überschuß verwendet wird, in organischem Medium und in Gegenwart eines Überschusses eines tertiären Amins und bei der Rückflußtemperatur des ausgewählten Lösungsmittels kondensiert, so daß man nach dem Abkühlen, der Extraktion und der eventuellen Reinigung durch Kristallisation ein Derivat der Formel (I/A) erhält, worin A eine lineare Alkylkette darstellt,
welches man erforderlichenfalls in die Isomeren auftrennen und gewünschtenfalls mit einer pharmazeutisch annehmbaren Säure in ein Salz überführen kann.

**17.** Verfahren zur Herstellung der Verbindung der Formel (I/B) nach Anspruch 3, **dadurch gekennzeichnet**, daß man eine Verbindung der Formel (I/A):

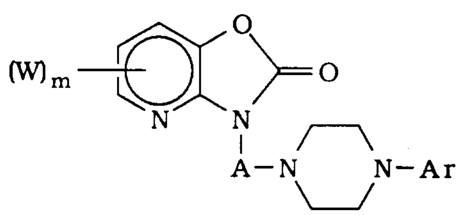

(I/A)

in der W, m, A und Ar die bezüglich der Formel (I) angegebenen Bedeutungen besitzen, oder eines seiner Additionssalze mit einer pharmazeutisch annehmbaren Säure mit einem alkalischen Mittel in wäßriger Lösung bei einer Temperatur zwischen Raumtemperatur und der Siedetemperatur des Reaktionsmediums behandelt, so daß man nach der Neutralisation oder dem Ansäuern des Reaktionsmediums eine Verbindung der Formel (I/B) erhält, die man nach der eventuellen Reinigung gewünschtenfalls mit einer pharmazeutisch annehmbaren Säure oder Base in ein Salz überführt.

**18.** Pharmazeutische Zubereitung enthaltend als Wirkstoff mindestens eine Verbindung nach einem der Ansprüche 1 bis 12 in Kombination mit einem oder mehreren inerten, nichttoxischen, pharmazeutisch annehmbaren Trägermaterialien oder Bindemitteln.

**19.** Pharmazeutische Zubereitung nach Anspruch 18 enthaltend mindestens einen Wirkstoff nach einem der Ansprüche 1 bis 12 für die Behandlung von Schmerzen.

**Patentansprüche für folgenden Vertragsstaat : GR**

**1.** Verfahren zur Herstellung von Verbindungen der allgemeinen Formel (I):

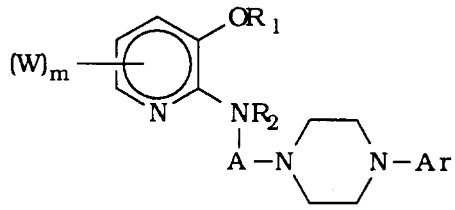

(I)

in der:

- $R_1$ und $R_2$ jeweils ein Wasserstoffatom oder gemeinsam mit dem Sauerstoff und dem Stickstoff, die sie tragen, eine -O-CO-N-Kette,
- W ein Halogenatom oder eine Niedrigalkyl- oder Niedrigalkoxy-gruppe, die gegebenenfalls durch ein oder mehrere Halogenatome substituiert sein kann, wie eine Trifluormethylgruppe, und m zwischen 0 und 3 liegt,
- A eine geradkettige oder verzweigte Niedrigalkylgruppe und
- Ar eine Aryl- oder Heteroaryl-gruppe, die gegebenenfalls durch ein oder mehrere Halogenatome oder durch eine oder mehrere Niedrigalkylgruppen, Hydroxylgruppen, Hydroxysulfonyloxygruppen oder Niedrigalkoxy- oder Aryloxy-gruppen, die gegebenenfalls durch ein oder mehrere Halogenatome substituiert sein können, wie die Trifluormethylgruppe, substituiert sein kann, bedeuten,

wobei unter einer Niedrigalkylgruppe oder Niedrigalkoxygruppe eine geradkettige oder verzweigte Alkylgruppe mit 1 bis 6 Kohlenstoffatomen zu verstehen ist und unter Aryl- oder Heteroaryl-gruppen mono- oder bicyclische ungesättigte Gruppen zu verstehen sind, die 5 bis 12 Atome aufweisen, die in ihrem Kohlenstoffskelett gegebenenfalls ein, zwei oder drei Heteroatome ausgewählt aus Stickstoff, Sauerstoff oder Schwefel aufweisen,

von deren Isomeren,

sowie von deren Additionssalzen mit einer pharmazeutisch annehmbaren Säure und, wenn $R_1$ und $R_2$ ein Wasserstoffatom darstellen, von deren Additionssalzen mit einer pharmazeutisch annehmbaren Base, **dadurch gekennzeichnet**, daß man ein Derivat der Formel (II):

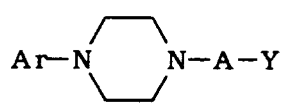

**(II)**

in der A und Ar die bezüglich der Formel (I) angegebenen Bedeutungen besitzen und Y ein Halogenatom darstellt,

nach dem Auflösen in einem organischen Lösungsmittel mit einem Derivat der Formel (III):

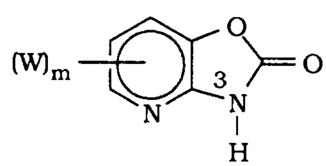

(III)

in der W und m die bezüglich der Formel (I) angegebenen Bedeutungen besitzen, vorzugsweise in Form seines Natriumderivats in der 3-Stellung durch Addition eines Alkalimetalls in der 3-Stellung, umsetzt,

so daß man nach dem Erhitzen des Reaktionsmediums, Abkühlen, Filtrieren, Verdampfen des Reaktionsmediums, Wiederaufnehmen mit Wasser, Extraktion mit einem organischen Lösungsmittel und eventuelle Reinigung durch Chromatographie über eine mit Siliciumdioxid beschickte Säule

ein Derivat der Formel (I/A) erhält:

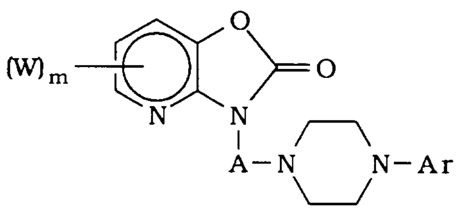

(I/A)

einem Sonderfall der Derivate der Formel (I), worin $R_1$ und $R_2$ mit dem Stickstoff und dem Sauerstoff, die sie tragen, eine O-CO-A-Kette bilden,
das man erforderlichenfalls in seine Isomeren auftrennen und gewünschtenfalls mit einer pharmazeutisch annehmbaren Säure in ein Salz überführen kann, welches Derivat der Formel (I/A) man gewünschtenfalls mit einer wäßrigen Lösung eines alkalischen Mittels bei einer Temperatur zwischen Raumtemperatur und der Siedetemperatur des Reaktionsmediums behandeln kann, so daß man nach der Neutralisation oder dem Sauerstellen des Reaktionsmediums und der eventuellen Reinigung ein Derivat der Formel (I/B) erhält:

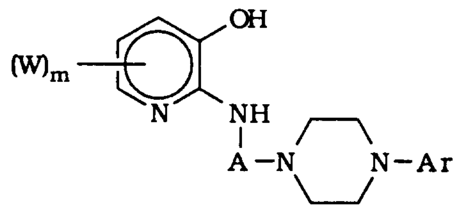

(I/B)

einem Sonderfall der Derivate der Formel (I), worin $R_1$ und $R_2$ jeweils ein Wasserstoffatom bedeuten,
das man gewünschtenfalls in seine Isomeren auftrennen und gewünschtenfalls mit einer pharmazeutisch annehmbaren Säure oder Base in ein Salz überführen kann.

**2.** Verfahren zur Herstellung von Verbindungen der Formel (I/A) nach Anspruch 1, worin A eine $CH_2$-Gruppe bedeutet, **dadurch gekennzeichnet**, daß man
einerseits ein Derivat der Formel (III):

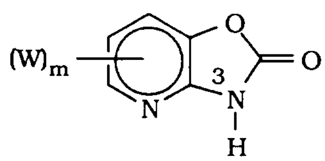

(III)

in der W und m die bezüglich der Formel (I) angegebenen Bedeutungen besitzen, andererseits einen schwachen Überschuß eines Arylpiperazins der Formel (VII):

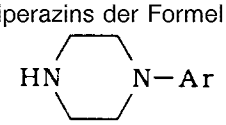

(VII)

in der Ar die bezüglich der Formel (I) angegebenen Bedeutungen besitzt,
und andererseits einen Überschuß Formaldehyd in einem niedrigaliphatischen alkoholischen Medium löst,
so daß man nach dem Erhitzen der erhaltenen Lösung auf eine Temperatur zwischen Raumtemperatur und der Siedetemperatur, dem eventuellen Abkühlen, dem Stehenlassen während einer bis zwei Stunden, dem Filtrieren und der eventuellen Säulenchromatographie über Siliciumdioxid ein Derivat der Formel (I/A1) erhält:

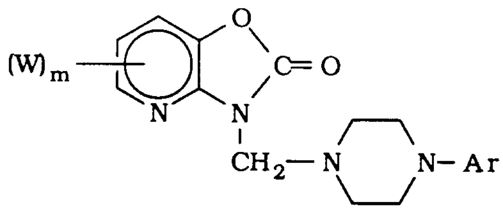

(I/A1)

in der W, m und Ar die bezüglich der Formel (I) angegebenen Bedeutungen besitzen, das man gegebenenfalls mit einer pharmazeutisch annehmbaren Säure in ein Salz überführen kann.

3. Verfahren zur Herstellung von Verbindungen der Formel (I/A) nach Anspruch 1, **dadurch gekennzeichnet**, daß man ein Derivat der Formel (III):

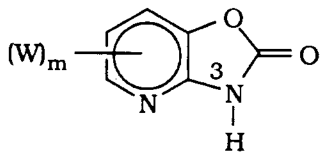

(III)

in der W und m die bezüglich der Formel (I) angegebenen Bedeutungen besitzen, der Reaktion mit einem Alkalimetallhydroxid in wäßrigem Medium oder einem Alkalimetallalkoholat in organischem Medium unterwirft zur Bildung eines Derivats der Formel (IV):

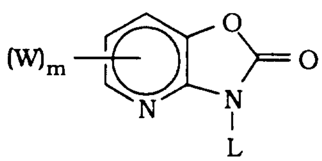

(IV)

in der W und m die oben angegebenen Bedeutungen besitzen und L ein Alkalimetall darstellt, welches man mit einem Derivat der Formel (V):

X - A - X'     (V)

in der A die bezüglich der Formel (I) angegebenen Bedeutungen besitzt und X und X', die gleichartig oder verschieden sind, ein Halogenatom darstellen, vorzugsweise unter einer inerten Atmosphäre in organischem Medium, vorzugsweise bei der Rückflußtemperatur des ausgewählten Lösungsmittels kondensiert, so daß man nach der eventuellen Extraktion und Reinigung durch Chromatographie ein Derivat der Formel (VI) erhält:

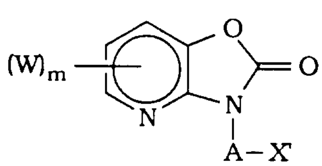

(VI)

in der W, X', m und A die oben angegebenen Bedeutungen besitzen, welches man vorzugsweise unter einer inerten Atmosphäre mit einem Derivat der Formel (VII):

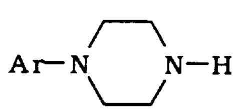

(VII)

in der Ar die bezüglich der Formel (I) angegebenen Bedeutungen besitzt,
das vorzugsweise im Überschuß eingesetzt wird, in organischem Medium und in Gegenwart eines Überschusses eines tertiären Amins und bei der Rückflußtemperatur des ausgewählten Lösungsmittels kondensiert, so daß man nach dem Abkühlen, der Extraktion und der eventuellen Reinigung durch Kristallisation ein Derivat der Formel (I/A) erhält,
welches man erforderlichenfalls in die Isomeren auftrennen und gewünschtenfalls mit einer pharmazeutisch annehmbaren Säure in ein Salz überführen kann.

**4.** Verfahren nach Anspruch 3 zur Herstellung von Verbindungen der Formel (I/A), worin A eine lineare Niedrigalkylkette darstellt, **dadurch gekennzeichnet**, daß man ein Derivat der Formel (III):

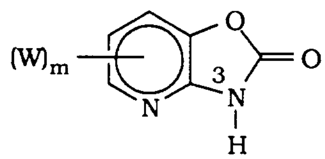

(III)

in der W und m die bezüglich der Formel (I) angegebenen Bedeutungen besitzen, einer Reaktion mit einem Alkalimetallhydroxid in wäßrigem Medium oder einem Alkalimetallalkoholat in organischem Medium unterwirft zur Bildung eines Derivats der Formel (IV):

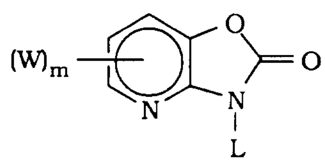

(IV)

in der W und m die bezüglich der Formel (I) angegebenen Bedeutungen besitzen und L ein Alkalimetall darstellt,
welches man mit einem Derivat der Formel (VIII):

X - A - X (VIII)

in der A die bezüglich der Formel (I) angegebenen Bedeutungen besitzt und X ein Halogenatom darstellt,
vorzugsweise unter einer inerten Atmosphäre in organischem Medium, vorzugsweise bei der Rückflußtemperatur des ausgewählten Lösungsmittels, kondensiert, so daß man nach der eventuellen Extraktion und Reinigung durch Chromatographie ein Derivat der Formel (VI) erhält:

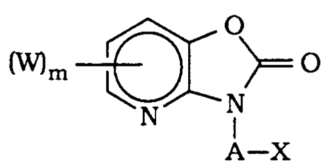

(VI)

in der W, X, m und A die oben angegebenen Bedeutungen besitzen,
welches man vorzugsweise unter inerter Atmosphäre mit einem Derivat der Formel (VII):

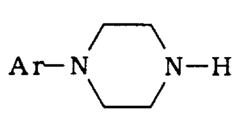

(VII)

in der Ar die oben angegebenen Bedeutungen besitzen, welches vorzugsweise im Überschuß verwendet wird, in organischem Medium und in Gegenwart eines Überschusses eines tertiären Amins und bei der Rückflußtemperatur des ausgewählten Lösungsmittels kondensiert, so daß man nach dem Abkühlen, der Extraktion und der eventuellen Reinigung durch Kristallisation ein Derivat der Formel (I/A) erhält, worin A eine lineare Alkylkette darstellt,

welches man erforderlichenfalls in die Isomeren auftrennen und gewünschtenfalls mit einer pharmazeutisch annehmbaren Säure in ein Salz überführen kann.

5. Verfahren zur Herstellung der Verbindung der Formel (I/B) nach Anspruch 1, **dadurch gekennzeichnet**, daß man eine Verbindung der Formel (I/A):

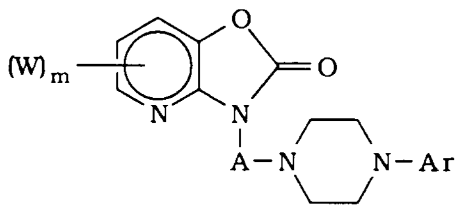

(I/A)

in der W, m, A und Ar die bezüglich der Formel (I) angegebenen Bedeutungen besitzen, oder eines seiner Additionssalze mit einer pharmazeutisch annehmbaren Säure mit einem alkalischen Mittel in wäßriger Lösung bei einer Temperatur zwischen Raumtemperatur und der Siedetemperatur des Reaktionsmediums behandelt, so daß man nach der Neutralisation oder dem Ansäuern des Reaktionsmediums eine Verbindung der Formel (I/B) erhält:

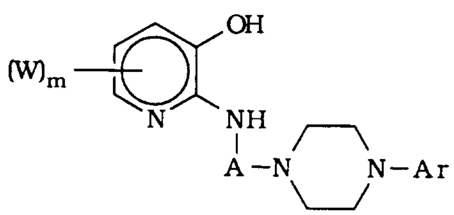

(I/B)

in der W, m, A und Ar die oben angegebenen Bedeutungen besitzen,
die man nach der eventuellen Reinigung gewünschtenfalls mit einer pharmazeutisch annehmbaren Säure oder Base in ein Salz überführt.

6. Verfahren nach Anspruch 1 zur Herstellung von Verbindungen, worin $R_1$ und $R_2$ gemeinsam eine CO-Gruppe darstellen, der Formel (I/A):

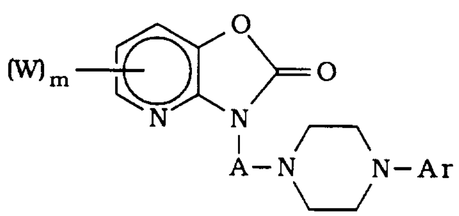

(I/A)

von deren Isomeren sowie von deren Additionssalzen mit einer pharmazeutisch annehmbaren Säure.

7. Verfahren nach Anspruch 1 zur Herstellung von Verbindungen, worin $R_1$ und $R_2$ jeweils ein Wasserstoffatom bedeuten, der Formel (I/B):

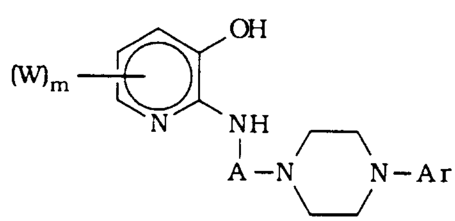

(I/B)

von deren Isomeren sowie von deren Additionssalzen mit einer pharmazeutisch annehmbaren Säure

oder einer pharmazeutisch annehmbaren Base.

8. Verfahren nach Anspruch 1 zur Herstellung von Verbindungen, worin Ar eine Phenylgruppe bedeutet, die gegebenenfalls durch ein Halogenatom oder eine Niedrigalkylgruppe, Hydroxylgruppe, Hydroxysulfonyloxygruppe oder Niedrigalkoxygruppe substituiert ist, die ihrerseits gegebenenfalls durch ein oder mehrere Halogenatome substituiert sind, sowie von deren Additionssalzen mit einer pharmazeutisch annehmbaren Säure oder, wenn $R_1$ und $R_2$ ein Wasserstoffatom bedeuten, von deren Isomeren und von deren Additionssalzen mit einer pharmazeutisch annehmbaren Base.

9. Verfahren nach Anspruch 1 zur Herstellung der Verbindung [4-(3-Trifluormethyl-phenyl)-piperazin-1-yl]-3-methyl-[3H]-oxazolo[4,5b]pyridin-2-on sowie von dessen Additionssalzen mit einer pharmazeutisch annehmbaren Säure.

10. Verfahren nach Anspruch 1 zur Herstellung der Verbindung (4-Phenyl)-piperazin-1-yl)-3-methyl-[3H]-oxazolo[4,5b]pyridin-2-on sowie von dessen Additionssalzen mit einer pharmazeutisch annehmbaren Säure.

11. Verfahren nach Anspruch 1 zur Herstellung der Verbindung 3-{2-[4-(3-Trifluormethyl-phenyl)-piperazin-1-yl]-ethyl}-[3H]-oxazolo[4,5b]pyridin-2-on sowie von dessen Additionssalzen mit einer pharmazeutisch annehmbaren Säure.

12. Verfahren nach Anspruch 1 zur Herstellung der Verbindung 3-{3-[4-(3-Trifluormethyl-phenyl)-piperazin-1-yl]-propyl}-[3H]-oxazolo[4,5b]pyridin-2-on sowie von dessen Additionssalzen mit einer pharmazeutisch annehmbaren Säure.

13. Verfahren nach Anspruch 1 zur Herstellung der Verbindung 3-[2-(4-Phenyl-piperazin-1-yl)-ethyl]-[3H]-oxazolo[4,5b]pyridin-2-on sowie von dessen Additionssalzen mit einer pharmazeutisch annehmbaren Säure.

14. Verfahren nach Anspruch 1 zur Herstellung der Verbindung 3-{2-[4-(Pyrid-2-yl)-piperazin-1-yl]-ethyl}-[3H]-oxazolo[4,5b]pyridin-2-on sowie von dessen Additionssalzen mit einer pharmazeutisch annehmbaren Säure.

15. Verfahren nach Anspruch 1 zur Herstellung der Verbindung 3-{2-[4-(Pyrimidin-2-yl)-piperazin-1-yl]-ethyl}-[3H]-oxazolo[4,5b]pyridin-2-on sowie von dessen Additionssalzen mit einer pharmazeutisch annehmbaren Säure.

16. Verfahren nach Anspruch 1 zur Herstellung der Verbindung 2-[2-(4-Phenyl-piperazin-1-yl]-ethylamino]-pyridin-3-ol sowie von dessen Additionssalzen mit einer pharmazeutisch annehmbaren Säure oder Base.

**Patentansprüche für folgenden Vertragsstaat : ES**

1. Verfahren zur Herstellung von Verbindungen der allgemeinen Formel (I):

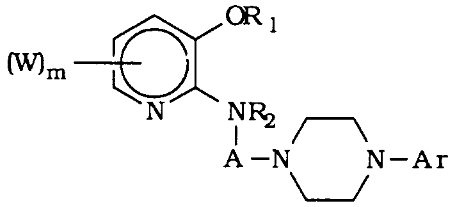

(I)

in der:

| | |
|---|---|
| $R_1$ und $R_2$ | jeweils ein Wasserstoffatom oder gemeinsam mit dem Sauerstoff und dem Stickstoff, die sie tragen, eine -O-CO-N-Kette, |
| W | ein Halogenatom oder eine Niedrigalkyl- oder Niedrigalkoxy-gruppe, die gegebenenfalls durch ein oder mehrere Halogenatome substituiert sein kann, wie eine Trifluor- |

methylgruppe, und m zwischen 0 und 3 liegt,

A eine geradkettige oder verzweigte Niedrigalkylgruppe und

Ar eine Aryl- oder Heteroaryl-gruppe, die gegebenenfalls durch ein oder mehrere Halogenatome oder durch eine oder mehrere Niedrigalkylgruppen, Hydroxylgruppen, Hydroxysulfonyloxygruppen oder Niedrigalkoxy- oder Aryloxy-gruppen, die gegebenenfalls durch ein oder mehrere Halogenatome substituiert sein können, wie die Trifluormethylgruppe, substituiert sein kann, bedeuten,

wobei unter einer Niedrigalkylgruppe oder Niedrigalkoxygruppe eine geradkettige oder verzweigte Alkylgruppe mit 1 bis 6 Kohlenstoffatomen zu verstehen ist und unter Aryl- oder Heteroaryl-gruppen mono- oder bicyclische ungesättigte Gruppen zu verstehen sind, die 5 bis 12 Atome aufweisen, die in ihrem Kohlenstoffskelett gegebenenfalls ein, zwei oder drei Heteroatome ausgewählt aus Stickstoff, Sauerstoff oder Schwefel aufweisen,

von deren Isomeren,

sowie von deren Additionssalzen mit einer pharmazeutisch annehmbaren Säure und, wenn $R_1$ und $R_2$ ein Wasserstoffatom darstellen, von deren Additionssalzen mit einer pharmazeutisch annehmbaren Base, **dadurch gekennzeichnet**, daß man ein Derivat der Formel (II):

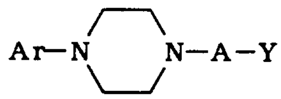

(II)

in der A und Ar die bezüglich der Formel (I) angegebenen Bedeutungen besitzen und Y ein Halogenatom darstellt,

nach dem Auflösen in einem organischen Lösungsmittel mit einem Derivat der Formel (III):

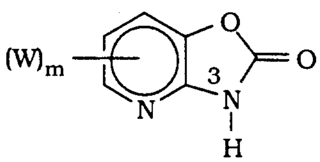

(III)

in der W und m die bezüglich der Formel (I) angegebenen Bedeutungen besitzen, vorzugsweise in Form seines Natriumderivats in der 3-Stellung durch Addition eines Alkalimetalls in der 3-Stellung, umsetzt,

so daß man nach dem Erhitzen des Reaktionsmediums, Abkühlen, Filtrieren, Verdampfen des Reaktionsmediums, Wiederaufnehmen mit Wasser, Extraktion mit einem organischen Lösungsmittel und eventuelle Reinigung durch Chromatographie über eine mit Siliciumdioxid beschickte Säule

ein Derivat der Formel (I/A) erhält:

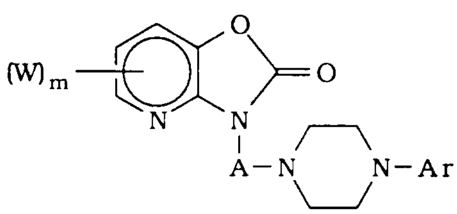

(I/A)

einem Sonderfall der Derivate der Formel (I), worin $R_1$ und $R_2$ mit dem Stickstoff und dem Sauerstoff, die sie tragen, eine O-CO-A-Kette bilden,

das man erforderlichenfalls in seine Isomeren auftrennen und gewünschtenfalls mit einer pharmazeutisch annehmbaren Säure in ein Salz überführen kann,

welches Derivat der Formel (I/A) man gewünschtenfalls mit einer wäßrigen Lösung eines alkalischen Mittels bei einer Temperatur zwischen Raumtemperatur und der Siedetemperatur des Reaktionsmediums behandeln kann, so daß man nach der Neutralisation oder dem Sauerstellen des Reaktionsmediums und der eventuellen Reinigung ein Derivat der Formel (I/B) erhält:

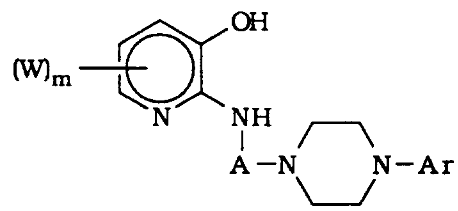

(I/B)

einem Sonderfall der Derivate der Formel (I), worin $R_1$ und $R_2$ jeweils ein Wasserstoffatom bedeuten, das man gewünschtenfalls in seine Isomeren auftrennen und gewünschtenfalls mit einer pharmazeutisch annehmbaren Säure oder Base in ein Salz überführen kann.

**2.** Verfahren zur Herstellung von Verbindungen der Formel (I/A) nach Anspruch 1, worin A eine $CH_2$-Gruppe bedeutet, **dadurch gekennzeichnet**, daß man
einerseits ein Derivat der Formel (III):

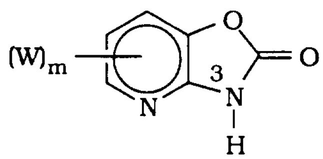

(III)

in der W und m die bezüglich der Formel (I) angegebenen Bedeutungen besitzen, andererseits einen schwachen Überschuß eines Arylpiperazins der Formel (VII):

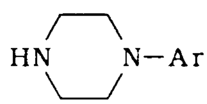

(VII)

in der Ar die bezüglich der Formel (I) angegebenen Bedeutungen besitzt,
und andererseits einen Überschuß Formaldehyd in einem niedrigaliphatischen alkoholischen Medium löst,
so daß man nach dem Erhitzen der erhaltenen Lösung auf eine Temperatur zwischen Raumtemperatur und der Siedetemperatur, dem eventuellen Abkühlen, dem Stehenlassen während einer bis zwei Stunden, dem Filtrieren und der eventuellen Säulenchromatographie über Siliciumdioxid ein Derivat der Formel (I/A1) erhält:

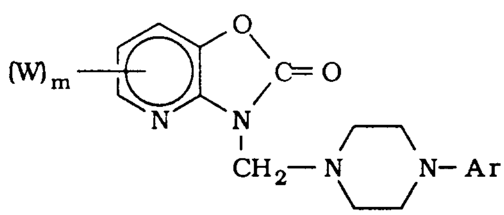

(I/A1)

in der W, m und Ar die bezüglich der Formel (I) angegebenen Bedeutungen besitzen,
das man gegebenenfalls mit einer pharmazeutisch annehmbaren Säure in ein Salz überführen kann.

**3.** Verfahren zur Herstellung von Verbindungen der Formel (I/A) nach Anspruch 1, **dadurch gekennzeichnet**, daß man ein Derivat der Formel (III):

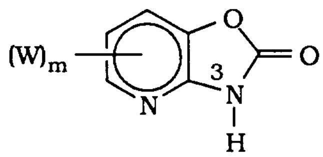

(III)

in der W und m die bezüglich der Formel (I) angegebenen Bedeutungen besitzen, der Reaktion mit einem Alkalimetallhydroxid in wäßrigem Medium oder einem Alkalimetallalkoholat in organischem Medium unterwirft zur Bildung eines Derivats der Formel (IV):

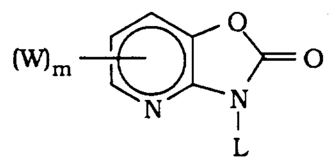

(IV)

in der W und m die oben angegebenen Bedeutungen besitzen und L ein Alkalimetall darstellt, welches man mit einem Derivat der Formel (V):

X - A - X' (V)

in der A die bezüglich der Formel (I) angegebenen Bedeutungen besitzt und X und X', die gleichartig oder verschieden sind, ein Halogenatom darstellen,
vorzugsweise unter einer inerten Atmosphäre in organischem Medium, vorzugsweise bei der Rückflußtemperatur des ausgewählten Lösungsmittels kondensiert, so daß man nach der eventuellen Extraktion und Reinigung durch Chromatographie ein Derivat der Formel (VI) erhält:

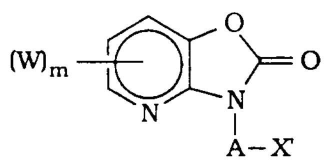

(VI)

in der W, X', m und A die oben angegebenen Bedeutungen besitzen,
welches man vorzugsweise unter einer inerten Atmosphäre mit einem Derivat der Formel (VII):

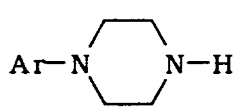

(VII)

in der Ar die bezüglich der Formel (I) angegebenen Bedeutungen besitzt,
das vorzugsweise im Überschuß eingesetzt wird, in organischem Medium und in Gegenwart eines Überschusses eines tertiären Amins und bei der Rückflußtemperatur des ausgewählten Lösungsmittels kondensiert, so daß man nach dem Abkühlen, der Extraktion und der eventuellen Reinigung durch Kristallisation ein Derivat der Formel (I/A) erhält,
welches man erforderlichenfalls in die Isomeren auftrennen und gewünschtenfalls mit einer pharmazeutisch annehmbaren Säure in ein Salz überführen kann.

**4.** Verfahren nach Anspruch 3 zur Herstellung von Verbindungen der Formel (I/A), worin A eine lineare Niedrigalkylkette darstellt, **dadurch gekennzeichnet**, daß man ein Derivat der Formel (III):

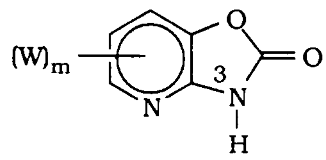

(III)

in der W und m die bezüglich der Formel (I) angegebenen Bedeutungen besitzen, einer Reaktion mit einem Alkalimetallhydroxid in wäßrigem Medium oder einem Alkalimetallalkoholat in organischem Medium unterwirft zur Bildung eines Derivats der Formel (IV):

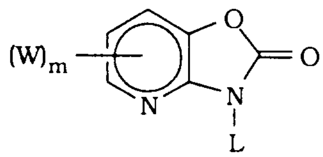

(IV)

in der W und m die bezüglich der Formel (I) angegebenen Bedeutungen besitzen und L ein Alkalimetall darstellt,
welches man mit einem Derivat der Formel (VIII):

X - A - X (VIII)

in der A die bezüglich der Formel (I) angegebenen Bedeutungen besitzt und X ein Halogenatom darstellt,
vorzugsweise unter einer inerten Atmosphäre In organischem Medium, vorzugsweise bei der Rückflußtemperatur des ausgewählten Lösungsmittels, kondensiert, so daß man nach der eventuellen Extraktion und Reinigung durch Chromatographie ein Derivat der Formel (VI) erhält:

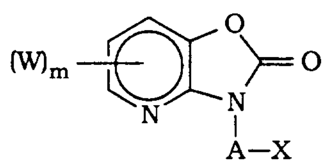

(VI)

in der W, X, m und A die oben angegebenen Bedeutungen besitzen,
welches man vorzugsweise unter inerter Atmosphäre mit einem Derivat der Formel (VII):

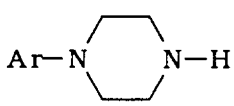

(VII)

in der Ar die oben angegebenen Bedeutungen besitzen, welches vorzugsweise im Überschuß verwendet wird, in organischem Medium und in Gegenwart eines Überschusses eines tertiären Amins und bei der Rückflußtemperatur des ausgewählten Lösungsmittels kondensiert, so daß man nach dem Abkühlen, der Extraktion und der eventuellen Reinigung durch Kristallisation ein Derivat der Formel (I/A) erhält, worin A eine lineare Alkylkette darstellt,
welches man erforderlichenfalls in die Isomeren auftrennen und gewünschtenfalls mit einer pharmazeutisch annehmbaren Säure in ein Salz überführen kann.

**5.** Verfahren zur Herstellung der Verbindung der Formel (I/B) nach Anspruch 1, **dadurch gekennzeichnet**, daß man eine Verbindung der Formel (I/A):

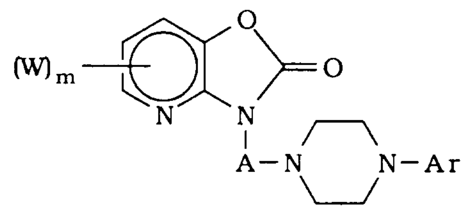

(I/A)

in der W, m, A und Ar die bezüglich der Formel (I) angegebenen Bedeutungen besitzen, oder eines seiner Additionssalze mit einer pharmazeutisch annehmbaren Säure mit einem alkalischen Mittel in wäßriger Lösung bei einer Temperatur zwischen Raumtemperatur und der Siedetemperatur des Reaktionsmediums behandelt, so daß man nach der Neutralisation oder dem Ansäuern des Reaktionsmediums eine Verbindung der Formel (I/B) erhält:

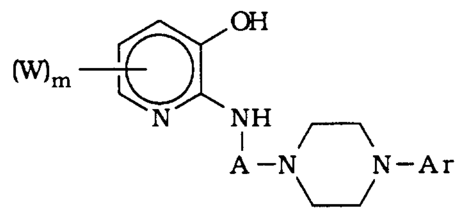

(I/B)

in der W, m, A und Ar die oben angegebenen Bedeutungen besitzen,
die man nach der eventuellen Reinigung gewünschtenfalls mit einer pharmazeutisch annehmbaren Säure oder Base in ein Salz überführt.

**6.** Verfahren nach Anspruch 1 zur Herstellung von Verbindungen, worin $R_1$ und $R_2$ gemeinsam eine CO-Gruppe darstellen, der Formel (I/A):

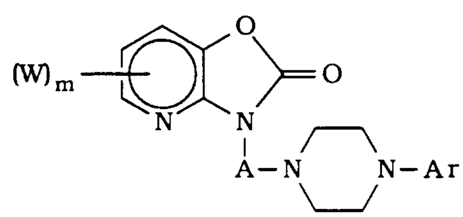

(I/A)

von deren Isomeren sowie von deren Additionssalzen mit einer pharmazeutisch annehmbaren Säure.

**7.** Verfahren nach Anspruch 1 zur Herstellung von Verbindungen, worin $R_1$ und $R_2$ jeweils ein Wasserstoffatom bedeuten, der Formel (I/B):

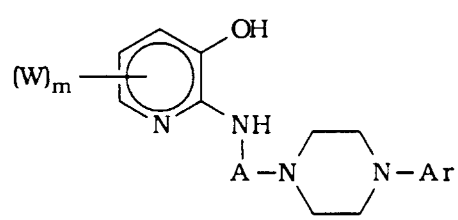

(I/B)

von deren Isomeren sowie von deren Additionssalzen mit einer pharmazeutisch annehmbaren Säure oder einer pharmazeutisch annehmbaren Base.

**8.** Verfahren nach Anspruch 1 zur Herstellung von Verbindungen, worin Ar eine Phenylgruppe bedeutet, die gegebenenfalls durch ein Halogenatom oder eine Niedrigalkylgruppe, Hydroxylgruppe, Hydroxysulfonyloxygruppe oder Niedrigalkoxygruppe substituiert ist, die ihrerseits gegebenenfalls durch ein oder mehrere Halogenatome substituiert sind, sowie von deren Additionssalzen mit einer pharmazeutisch

annehmbaren Säure oder, wenn $R_1$ und $R_2$ ein Wasserstoffatom bedeuten, von deren Isomeren und von deren Additionssalzen mit einer pharmazeutisch annehmbaren Base.

**9.** Verfahren nach Anspruch 1 zur Herstellung der Verbindung [4-(3-Trifluormethyl-phenyl)-piperazin-1-yl]-3-methyl-[3H]-oxazolo[4,5b]pyridin-2-on sowie von dessen Additionssalzen mit einer pharmazeutisch annehmbaren Säure.

**10.** Verfahren nach Anspruch 1 zur Herstellung der Verbindung (4-Phenyl)-piperazin-1-yl)-3-methyl-[3H]-oxazolo[4,5b]pyridin-2-on sowie von dessen Additionssalzen mit einer pharmazeutisch annehmbaren Säure.

**11.** Verfahren nach Anspruch 1 zur Herstellung der Verbindung 3-{2-[4-(3-Trifluormethyl-phenyl)-piperazin-1-yl]-ethyl}-[3H]-oxazolo[4,5b]pyridin-2-on sowie von dessen Additionssalzen mit einer pharmazeutisch annehmbaren Säure.

**12.** Verfahren nach Anspruch 1 zur Herstellung der Verbindung 3-{3-[4-(3-Trifluormethyl-phenyl)-piperazin-1-yl]-propyl}-[3H]-oxazolo[4,5b]pyridin-2-on sowie von dessen Additionssalzen mit einer pharmazeutisch annehmbaren Säure.

**13.** Verfahren nach Anspruch 1 zur Herstellung der Verbindung 3-[2-(4-Phenyl-piperazin-1-yl)-ethyl]-[3H]-oxazolo[4,5b]pyridin-2-on sowie von dessen Additionssalzen mit einer pharmazeutisch annehmbaren Säure.

**14.** Verfahren nach Anspruch 1 zur Herstellung der Verbindung 3-{2-[4-(Pyrid-2-yl)-piperazin-1-yl]-ethyl}-[3H]-oxazolo[4,5b]pyridin-2-on sowie von dessen Additionssalzen mit einer pharmazeutisch annehmbaren Säure.

**15.** Verfahren nach Anspruch 1 zur Herstellung der Verbindung 3-{2-[4-(Pyrimidin-2-yl)-piperazin-1-yl]-ethyl}-[3H]-oxazolo[4,5b]pyridin-2-on sowie von dessen Additionssalzen mit einer pharmazeutisch annehmbaren Säure.

**16.** Verfahren nach Anspruch 1 zur Herstellung der Verbindung 2-[2-(4-Phenyl-piperazin-1-yl]-ethylamino]-pyridin-3-ol sowie von dessen Additionssalzen mit einer pharmazeutisch annehmbaren Säure oder Base.